# FASCICULE DE BREVET EUROPEEN

(11) **EP 1 572 246 B1**
(45) Date de publication et mention de la délivrance du brevet: **25.02.2009**
(21) Numéro de dépôt: 03799680.8
(22) Date de dépôt: 19.12.2003
(51) Int. Cl.: A61K 49/18, C07F 9/38

(54) **NOUVELLES COMPOSITIONS DE PARTICULES MAGNETIQUES RECOUVERTES DE DERIVES GEM-BISPHOSPHONATES**
ZUSAMMENSETZUNGEN VON MAGNETISCHEN PARTIKELN MIT GEM-BIPHOSPHONATE DERIVATEN BESCHICHTET
NOVEL COMPOSITIONS OF MAGNETIC PARTICLES COVERED WITH GEM-BISPHOSPHONATE DERIVATIVES

(30) Priorité: 20.12.2002 FR 0216410
(43) Date de publication de la demande: 14.09.2005
(73) Titulaire: GUERBET, 93420 Villepinte (FR)
(72) Inventeur: PORT, Marc, F-95170 Deuil la Barre (FR); COROT, Claire, F-69006 Lyon (FR); RAYNAL, Isabelle, F-94100 Saint-Maur des Fosses (FR); ROUSSEAUX, Olivier, F-60300 Senlis (FR)
(74) Mandataire: Colombet, Alain André
(86) Numéro de dépôt international: PCT/FR2003/003855
(87) Numéro de publication internationale: WO 2004/058275

(56) Documents cités:
- WO-A-97/01760
- FR-A- 2 461 521
- GB-A- 1 435 295
- US-A- 4 329 241
- US-A- 4 440 646
- US-A1- 2002 098 149
- US-A1- 2002 122 806
- PORTET D ET AL: "NONPOLYMERIC COATINGS OF IRON OXIDE COLLOIDS FOR BIOLOGICAL USE AS MAGNETIC RESONANCE IMAGING CONTRAST AGENTS" JOURNAL OF COLLOID AND INTERFACE SCIENCE, ACADEMIC PRESS, NEW YORK, NY, US, vol. 238, no. 1, 1 juillet 2001 (2001-07-01), pages 37-42, XP001162799 ISSN: 0021-9797 cité dans la demande
- MASSART R ET AL: "Synthese en milieu alcalin de magnetite colloidale: controle du rendement et de la taille des particules" JOURNAL DE CHIMIE PHYSIQUE, vol. 84, no. 7-8, 1987, pages 967-973, XP008021240
- SAHOO Y ET AL: "Alkyl phosphonate/phosphate coating on magnetite nanoparticles: a comparison with fatty acids" LANGMUIR, vol. 17, 2001, pages 7907-7911, XP002253807
- FAUCONNIER ET AL: "Synthesis of aqueous magnetic liquids by surface complexation of maghemite nanoparticles" JOURNAL OF MOLECULAR LIQUIDS, vol. 83, 1999, pages 233-242, XP001165785
- TRONC ET AL.: "Magnetic behaviour of gamma-Fe2O3 nanoparticles by Mössbauer spectroscopy and magnetic measurements" HYPERFINE INTERACTIONS, vol. 95, 1995, pages 129-148, XP008021192
- VAYSSIERES L ET AL.: "Size tailoring of magnetite particles formed by aqueous precipitation: an example of thermodynamic stability of nanometric oxide particles" JOURNAL OF COLLOID AND INTERFACE SCIENCE, vol. 205, 1998, pages 205-212, XP001165781
- MASSART R: "PREPARATION OF AQUEOUS MAGNETIC LIQUIDS IN ALKALINE AND ACIDIC MEDIA" IEEE TRANSACTIONS ON MAGNETICS, IEEE INC. NEW YORK, US, vol. 17, no. 2, mars 1981 (1981-03), pages 1247-1248, XP001165602 ISSN: 0018-9464 cité dans la demande
- FAUCONNIER ET AL: "Adsorption of gluconic and citric acids on maghemite particles in aqueous medium" PROGR. COLLOID POLYM SCI, vol. 100, 1996, pages 212-216, XP008021219
- TRONC ET AL: "Surface effects on magnetically coupled "gamma-Fe2O3" colloids" HYPERFINE INTERACTIONS, vol. 28, 1986, pages 525-528, XP008021196
- ROGER J ET AL: "SOME BIOMEDICAL APPLICATIONS OF FERROFLUIDS" EUROPEAN PHYSICAL JOURNAL APPLIED PHYSICS, EDP SCIENCES, LES ULIS, FR, vol. 5, no. 3, mars 1999 (1999-03), pages 321-325, XP000896706 ISSN: 1286-0042

## Description

L'invention concerne des compositions de particules magnétiques recouvertes de molécules comprenant d'une part, une fonction *gem-*bisphosphonate liée à la surface de la particule et, d'autre part, une fonction réactive susceptible de lier covalemment une entité de ciblage appelée biovecteur. L'invention vise également leur procédé de préparation et leur utilisation notamment comme produit de contraste pour l'Imagerie par Résonance Magnétique nucléaire (IRM).

L'IRM permet la visualisation d'organes internes chez des êtres vivants et constitue donc un auxiliaire et un guide puissant dans le diagnostic, le traitement médical et la chirurgie.

L'administration de produits de contraste contribue à améliorer la résolution des images obtenues par cette technique et la précision du diagnostic.

Ainsi, l'effet de contraste peut être accentué par la présence, dans l'environnement des organes soumis à examen, de diverses espèces magnétiques, par exemple paramagnétiques, ferromagnétiques ou superparamagnétiques.

Les substances paramagnétiques comprennent certains métaux comme le fer, le manganèse, le gadolinium à l'état ionique ou organométallique. Les substances de contraste ferromagnétiques comprennent généralement des particules d'agrégat magnétiques de taille micrométrique ou sous-micrométrique, c'est-à-dire non inférieure à 100-200 nm, par exemple des particules de ferrites, dont notamment de magnétite (Fe₃O₄), de maghémite (γ-Fe₂O₃) et autres composés minéraux magnétiques d'éléments de transition qui se comportent comme des aimants permanents. Les particules superparamagnétiques sont habituellement de très petites particules de ferrite, dont notamment de magnétite (Fe₃O₄), de maghémite (γ-Fe₂O₃) et autres composés minéraux magnétiques d'éléments de transition, de taille inférieure à environ 100-150 nm. Contrairement aux particules ferromagnétiques, les particules superparamagnétiques ne se comportent plus comme de petits aimants autonomes du fait que leur taille est inférieure à une valeur critique, c'est-à-dire qu'elles s'alignent dans une direction préférentielle uniquement lorsqu'elles sont soumises à un champ magnétique externe. Les particules superparamagnétiques présentent avantageusement une densité d'efficacité plus élevée par rapport aux particules ferromagnétiques.

Pour obtenir des solutions colloïdales de particules magnétiques, encore dénommées ferrofluides dans ce qui suit, stables en milieu physiologique, il est nécessaire de conditionner la surface des particules magnétiques.

Le plus souvent, les particules magnétiques utilisées en Imagerie médicale sont recouvertes de macromolécules telles que des carbohydrates comme le dextrane, des protéines comme l'albumine ou des polymères de synthèse comme les méthacrylates et les organosilanes. Les synthèses permettant d'aboutir à ce type de particules sont généralement effectuées selon le procédé dit « de Molday » (Référence : Robert S. Molday and D. Mackenzie ; J. of Immunological Methods (1982), 52, p 353-367) et nécessitent des purifications laborieuses et coûteuses.

Dans beaucoup d'applications biomédicales des particules magnétiques, il est nécessaire de coupler ces dernières à une molécule d'affinité spécifique, de manière à ce que les particules se lient spécifiquement aux cellules cibles et tissus. Cette reconnaissance est souvent assurée par la formation d'un complexe d'affinité entre un ligand (biovecteur) fixé à la particule et un récepteur à la surface de la cellule cible ou d'un tissu. Cette molécule d'affinité spécifique peut être soit adsorbée directement à la surface des particules, soit liée par covalence.

De façon générale, le greffage covalent de la molécule d'affinité spécifique sur une macromolécule requiert préalablement une activation chimique, par exemple par oxydation, de manière à générer des sites de fixation sur la macromolécule.

Cependant, le nombre de sites de fixation généré sur la macromolécule est généralement difficile à maîtriser et, par voie de conséquence, le greffage ultérieur d'une molécule d'affinité spécifique ne peut être contrôlé.

Or, le contrôle de la quantité de molécule d'affinité spécifique greffée est important pour moduler l'affinité de la solution finale de particules vis-à-vis de la cible, mais aussi pour moduler la pharmacocinétique, la biodistribution et éventuellement le métabolisme, l'excrétion et l'innocuité de ces particules. En outre, d'un point de vue industriel, il est intéressant de pouvoir minimiser la quantité de molécule d'affinité spécifique, dont le coût est en général très élevé.

De plus, les fonctions réactives à la surface de la macromolécule n'ayant pas réagi avec ladite molécule d'affinité spécifique, sont susceptibles d'induire des réactions toxiques *in vivo.*

De même, de façon générale, le greffage d'une molécule d'affinité spécifique par chimisorption est difficile à contrôler d'un point de vue physico-chimique et, de plus, beaucoup moins stable en milieu biologique. En outre, la chimisorption induit un équilibre avec une fraction libre de la molécule d'affinité spécifique. Cette fraction libre est préjudiciable au contraste en IRM.

Des particules magnétiques recouvertes de molécules organiques de faible poids moléculaire ont également été décrites à ce jour.

Le document J. of Coll. And Interf. Science 2001, 238, p 37-42 enseigne vue le recouvrement de particules magnétiques par des molécules organiques comportant une partie bisphosphonate permet d'obtenir des objets de petite taille (inférieure à 15 nm) et stables dans un large domaine de pH.

Cependant, la mesure de la taille hydrodynamique des particules recouvertes de composés bisphosphonates par PCS (Photon Correlation Spectroscopy) décrite dans ce document requiert une étape de filtration préalable sur des filtres de 100 nm, pour éliminer les agrégats et n'isoler que les particules de faible taille hydrodynamique. Cette mesure ne reflète donc pas la taille hydrodynamique réelle des particules obtenues qui est, en fait, beaucoup plus élevée.

En outre, la purification des particules magnétiques recouvertes par les composés *gem*-bisphosphonates via le procédé décrit dans ce document nécessite la mise en oeuvre d'une étape de dialyse ou de traitement par des résines, ce qui s'avère laborieux, peu reproductible et très difficilement exploitable industriellement. On rappelle que les gem-bisphosphonates sont les groupements -CH(PO₃H₂)₂.

Ce procédé ne permet donc de contrôler ni le greffage des composés *gem*-bisphosphonates à la surface des particules magnétiques, ni la taille hydrodynamique de la particule résultante, sans avoir recours à des purifications laborieuses. En outre, ce document ne décrit ni ne suggère de coupler des entités de ciblage spécifique à des particules recouvertes de composés bisphosphonates, et en particulier des gem-bisphonates, pour des applications biomédicales spécifiques, ce qui rend leur utilisation fort inadaptée pour ce ciblage.

Le diamètre hydrodynamique, au sens de la présente invention, fait référence au diamètre de la particule en solution entourée par sa couche d'hydratation, tel que déterminé par diffusion de lumière selon la technique PCS (Spectroscopie d'AutoCorrélation de Photon, Photon Corrélation Spectroscopy : Référence : R. Pecora - J. of Nanoparticle Research (2000), Vol. 2, pp. 123-131).

La publication WO 97/01760 décrit des particules magnétiques recouvertes d'acide dimercaptosuccinique (ADMS), couplées à une entité de ciblage spécifique, l'annexine, via les fonctions thiols de l'ADMS.

Cependant, dans les conditions de pH et de force ionique habituelles, les fonctions thiols de l'ADMS présentes à la surface de la particule ont tendance à s'oxyder et à former des ponts dissulfures, ce qui conduit à la formation d'agrégats. Il s'ensuit une taille hydrodynamique élevée ainsi qu'une forte polydispersion.

De plus, le greffage de l'annexine nécessite, au préalable, de régénérer les fonctions thiols par réduction des ponts dissulfures.

Enfin, le greffage de l'annexine est mal contrôlé. Il s'ensuit qu'après couplage, il s'avère nécessaire de désactiver les fonctions thiols résiduelles par réaction avec la BSA (Bovin Serum Albumin) de manière à éviter des réactions toxiques in vivo ou l'agrégation ultérieure des particules obtenues.

Le besoin demeure donc d'obtenir des particules à la fois efficaces pour un diagnostic spécifique, peu polydisperses et stables.

Il a maintenant été découvert que l'on pouvait contrôler à la fois le greffage de composés *gem*-bisphosphonates fonctionnalisés sur des particules magnétiques et le couplage des particules résultantes à des biovecteurs, grâce à l'utilisation combinée desdits composés *gem*-bisphosphonates et de particules magnétiques acides. Ce double contrôle présente l'avantage de permettre notamment la maîtrise de la taille hydrodynamique des particules résultantes, à savoir des particules magnétiques acides (p) greffées par des composés *gem*-biphosphonates, qu'elles soient couplées ou non à des biovecteurs. L'invention concerne ainsi notamment :
- les particules acides sur lesquelles sont greffées les gem-bisphosphonates, ces particules une fois greffées étant capables de se lier à au moins un biovecteur ;
- ces particules greffées par des gem-bisphosphonates et couplées à au moins un biovecteur.

Dans ce cadre, il s'est avéré que le procédé d'obtention de telles particules magnétiques comprend un nombre limité d'étapes et est à la fois simple à mettre en oeuvre et reproductible. De plus, il ne requiert pas de purifications laborieuses et se caractérise par de très bons rendements.

Au sens de la présente invention, on entend par "particule magnétique acide" les particules magnétiques du type de celles présentes au sein des ferrofluides dits "acides", connus de l'état de la technique, mais dont l'association à des *gem*-bisphonates n'était ni décrite ni suggérée.

Ces ferrofluides "acides" de même que les ferrofluides "alcalins" ont largement été décrits dans la littérature, notamment dans la publication Massart et al., C. R. Acad. Sc. Paris, t. 291, 7/07/1980, Série C et IEE, Transactions on Magnetics, 1981, vol. MAG-17, n°2, p. 1247.

On décrit généralement les particules magnétiques acides des ferrofluides acides comme présentant des sites hydroxyles protonés en surface, en milieu acide.

Par opposition, les particules magnétiques des ferrofluides alcalins sont décrites comme présentant généralement des sites complexés par des ions OH- en milieu basique.

Cette représentation en termes de sites correspond en fait à un modèle qui a d'ailleurs été conforté par différents travaux montrant que les particules magnétiques acides et basiques se comportent comme des polyacides et/ou polybases faibles.

De façon avantageuse, les inventeurs ont montré que, contrairement aux ferrofluides "alcalins", les ferrofluides "acides" sont très stables et ne présentent pas de phénomène d'agrégation de particules, d'où un meilleur contrôle de la taille hydrodynamique du précurseur (c'est-à-dire de la particule non greffée) et ultérieurement des particules greffées par les composés *gem-*bisphosphonates, puis, le cas échéant, desdites particules couplées à des biovecteurs, sans étape de purification laborieuse.

De façon surprenante, les inventeurs ont montré par ailleurs qu'on peut obtenir un très bon recouvrement des particules magnétiques par les composés *gem*-bisphosphonates, chaque site hydroxyle protoné à la surface de la particule magnétique acide pouvant être potentiellement greffé par une molécule d'agent complexant *gem*-bisphosphonate. Cette interaction permet, avantageusement, de masquer la surface de la particule de façon homogène, ce qui peut permettre, en particulier, d'empêcher la reconnaissance ultérieure de la particule par le système immunitaire.

De façon particulière, les inventeurs ont découvert en outre un procédé permettant de réduire la taille hydrodynamique des particules magnétiques acides et d'obtenir en conséquence des particules magnétiques de très faible taille. Avantageusement, le greffage contrôlé de ces particules par les agents *gem*-bisphosphonates suivi éventuellement du couplage, également contrôlé, avec des biovecteurs permettent d'obtenir des particules magnétiques finales de faible taille hydrodynamique sans aucune étape de purification laborieuse, et donc d'avoir au final, un procédé de préparation robuste, reproductible et donc, industrialisable.

Les inventeurs ont par ailleurs étudié la complexation des particules magnétiques acides (p) par des composés comportant, d'une part, une fonction X susceptible de lier covalemment un biovecteur et d'autre part une fonction diphosphonate en lieu et place de la fonction *gem*-bisphosphonate.

Ils ont ainsi obtenu des particules stables, ce qui est déjà une amélioration distinctive par rapport à l'art antérieur, mais le couplage à un biovecteur est significativement moins efficace qu'avec un *gem*-bisphosphonate. En particulier, lorsqu'un composé diphosphonate HOOC-CH₂-N(CH₂(PO₃H₂))₂ est utilisé, pour des raisons non totalement élucidées, les particules présentent un taux de couplage avec le biovecteur deux fois plus faible qu'avec un *gem-*bisphosphonate. Il en résulte donc que le coût de production des particules magnétiques greffées par des diphosphonates et couplées à des biovecteurs serait beaucoup plus élevé.

La présente invention vise donc à proposer des compositions de particules magnétiques greffées de façon contrôlée par des dérivés *gem*-bisphosphonates et éventuellement couplées de façon contrôlée, via ces dérivés, à des espèces de type entité de ciblage spécifique (biovecteur).

De façon plus spécifique, l'invention vise également à fournir des compositions de ce type, stables en suspension en particulier en milieu biologique, et avantageusement de taille hydrodynamique contrôlée, notamment de faible taille hydrodynamique.

La présente invention vise également à proposer un procédé de préparation desdites compositions.

Selon un premier aspect, l'invention concerne une composition comprenant des particules magnétiques acides (p) à base d'un composé du fer, lesdites particules magnétiques acides (p) étant chacune complexée par un ou plusieurs composés *gem*-bisphosphonates, de formule (I) :

X-L-CH(PO₃H₂)₂ (I)

dans laquelle :
- L représente un groupement organique reliant la fonction X à la fonction *gem*-bisphosphonate -CH(PO₃H₂)₂ ;
- X représente une fonction chimique susceptible de réagir avec un biovecteur ; lesdites fonctions X desdites particules (p) étant éventuellement couplées en tout ou partie à un biovecteur.

Sous l'expression "étant chacune complexée", l'homme du métier comprendra que la composition peut comprendre également des particules magnétiques (p) non complexées par des composés de formule (I), ceci principalement pour des raisons de distribution statistiques et/ou en raison du procédé de préparation de ladite composition décrit ci-après.

La composition se présente sous forme d'une solution aqueuse stable de nanoparticules.

Dans ces compositions, le taux de complexation du composé (I) sur les particules est supérieur à 70%, et de préférence supérieur à 80, 90, 95%, étant entendu que l'expression "taux de complexation" dans la présente description se réfère de préférence à la quantité de composés de formule (I), par rapport à la quantité de sites protonés présents sur les particules magnétiques acides (p).

On préfère particulièrement que les particules magnétiques acides (p) soient complexées sur au moins 90% de leurs sites protonés par des composés de formule (I), ce par quoi on obtient avantageusement un bon recouvrement de la particule, apte en particulier à assurer une bonne stabilité de la particule, à empêcher la reconnaissance de la particule par le système immunitaire et à rendre l'éventuel couplage ultérieur avec un biovecteur plus efficace.

### PARTICULE MAGNÉTIQUE (P)

Par "particules à base d'un composé du fer", on entend, au sens de la présente description, des particules constituées en tout ou partie par un dérivé du fer, comprenant généralement du fer (III), généralement un oxyde ou un hydroxyde de fer.

En règle générale, les particules magnétiques acides (p) sont composées en tout ou partie d'hydroxyde de fer ; d'oxyde de fer hydraté ; de ferrites ; d'oxydes de fer mixtes tels que des oxydes de fer mixtes de cobalt, de nickel, de manganèse, de béryllium, de magnésium, de calcium, de baryum, de strontium, de cuivre, de zinc ou de platine ; ou d'un mélange de ceux-ci.

Au sens de la présente invention; le terme de "ferrite" désigne les oxydes de fer de formule générale [x Fe₂O₃, y MO_{z}], où M désigne un métal magnétisable sous l'effet d'un champ magnétique tel que Fe, Co, Ru, Mg, Mn, le métal magnétisable pouvant être éventuellement radioactif.

De façon préférentielle, les particules magnétiques acides (p) des compositions de l'invention comprennent une ferrite, notamment la maghémite (γ Fe₂O₃) et la magnétite (Fe₃O₄), ou encore les ferrites mixtes de cobalt (Fe₂CoO₄) ou de manganèse (Fe₂MnO₄).

Dans ce cadre, on préfère tout particulièrement les particules magnétiques acides (p) composées en tout ou partie, et de préférence essentiellement, de maghémite ou de magnétite ou d'un mélange de ceux-ci.

Les particules magnétiques acides (p) des compositions selon l'invention présentent en surface des sites hydroxyles protonés en milieu acide, qui correspondent plus précisément à l'espèce Fe-OH₂⁺ résultant d'une interaction très forte entre un ion Fe³⁺ à la surface de la particule et une molécule d'eau acide (H₃O⁺). Ces protons peuvent être considérés comme constitutifs de la particule d'après les travaux de J. Lyklema et al., Materials Science Research, 1984, 17, p. 1-24. Le pourcentage de sites protonnés sur une particule acide est compris typiquement entre 1.3 et 2.5% (mole de sites / mole de fer).

Selon une variante particulièrement préférée, les particules acides (p) sont superparamagnétiques.

Les particules magnétiques (p) possèdent alors, un diamètre hydrodynamique compris entre 5 et 300 nm, de préférence entre 5 et 60 nm, encore de préférence entre 5 et 30 nm.

De préférence, la composition de l'invention est une suspension de particules magnétiques acides (p) qui présente avantageusement, le cas échéant, une concentration adaptée à la formation d'une solution colloïdale stable (suspension aqueuse de nanoparticules stable), à savoir, en général, une concentration en particules magnétiques (p) allant de 2 µmoles L⁻¹ à 4 moles L⁻¹, exprimée en nombre de moles d'ions métalliques total.

### COMPOSÉS DE FORMULE (I)

Les composés de formule (I) selon l'invention comportent une fonction *gem*-bisphosphonate qui assure la fixation du composé de formule (I) à la surface de la particule magnétique acide.

Plus précisément, les inventeurs ont mis en évidence que chaque fonction *gem*-bisphosphonate interagit avec un seul site hydroxyle protoné à la surface de la particule acide.

Les composés de formule (I) de l'invention comprennent un linker (L) permettant de relier et/ou d'espacer la fonction *gem*-bisphosphonate et l'entité réactive X capable d'assurer le greffage covalent d'un biovecteur.

A titre préféré, le linker L représente un groupement divalent.

De préférence, le linker L est choisi parmi :
- un groupe aliphatique , alicyclique ; alicyclique-aliphatique ; aromatique ; aromatique-aliphatique, lesdits groupes aliphatiques, alicycliques et aromatiques pouvant être éventuellement substitués par un groupe méthyle, hydroxy, méthoxy, acétoxy, amido, ou un atome de chlore, d'iode ou de brome ;
- un groupement -L₁-NHCO-L₂ où L₁ et L₂ sont soit identiques, soit différents et représentent un groupe aliphatique ; alicyclique ; aromatique ; alicyclique-aliphatique ou aromatique-aliphatique, lesdits groupes pouvant être éventuellement substitués par un groupe méthyle, hydroxy, méthoxy, acétoxy, amido ou un atome de chlore, d'iode ou de brome.

Un groupe aliphatique, au sens de la présente invention, désigne une chaîne hydrocarbonée linéaire ou ramifiée comportant de préférence de 1 à 16 atomes de carbone, mieux encore de 1 à 6 atomes de carbone. Des exemples en sont notamment les radicaux méthyle, éthyle, propyle, isopropyle, butyle, tert-butyle, isobutyle, pentyle et hexyle.

Le terme "alicyclique" désigne une chaîne hydrocarbonée cyclique comportant de préférence de 3 à 8 atomes de carbone. A titre d'exemple, on citera notamment le cyclopropyle et le cyclohexyle.

Le terme "aromatique" représente un groupement hydrocarboné mono- ou polycyclique aromatique comprenant préférentiellement de 5 à 20 atomes de carbone, mieux encore de 6 à 18. Des exemples en sont notamment les radicaux phényles et 1- ou 2-naphtyles. Selon une variante particulière, un groupe "aromatique" au sens de l'invention peut intégrer un ou plusieurs hétéroatomes tels que le soufre, l'oxygène ou l'azote. Dans ce cas particulier, le groupe "aromatique" désigne un groupement hétéroaromatique mono- ou polycyclique.

Les groupes "aliphatique-alicyclique" et "aliphatique-aromatique" représentent des chaînes aliphatiques répondant à la définition susmentionnée, substituées par respectivement un groupement alicyclique ou aromatique tels que définis ci-dessus. A titre d'exemple de groupe aliphatique-aromatique, on peut notamment citer le benzyle.

Selon une variante préférée, L représente un groupe phénylène éventuellement substitué, les groupements X et *gem*-bisphosphonates pouvant être en position ortho, méta ou para.

Selon un mode de réalisation particulièrement préféré, L représente un groupe aliphatique substitué ou non, et plus préférentiellement un groupe -(CH₂)ₚ-, où p est un entier de 1 à 5.

Selon un autre mode préférentiel, L représente un groupe L₁-CONH-L₂ et plus préférentiellement un groupe -(CH₂)ₙ-NHCO-(CH₂)ₘ- où n et m représentent un nombre entier de 0 à 5.

L'extrémité X du composé *gem*-bisphosphonate de formule (I) est choisie de telle sorte qu'elle soit susceptible de réagir et de former une liaison covalente avec une fonction présente sur le biovecteur. Pour plus d'informations concernant ces couplages, on pourra se référer notamment à l'ouvrage *Bioconjugate techniques,* Greg T. Hermanson, 1995, Publisher : Academic, San Diego, Calif.

A titre de groupes X préférés, on peut citer notamment :
- -COOH,
- -NH₂, -NCS, -NH-NH₂, -CHO, alkylpyrocarbonyle (-CO-O-CO-alk), acylazidyle (-CO-N₃), iminocarbonate (-O-C(NH)-NH₂), vinylsulfuryle (-S-CH=CH₂), pyridyldisulfuryle (-S-S-Py), haloacétyle, maléimidyle, dichlorotriazinyle, halogène,
- les groupes -COOH et -NH₂ étant particulièrement préférés.

Le terme « alk » désigne au sens de la présente description un radical alkyle en C₁ - C₆, le terme « Py » désignant quant à lui un radical pyridyle.

Le radical maléimidyle désigne un radical cyclique de formule :

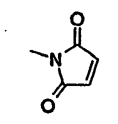

Le radical dichlorotriazinyle désigne un radical de formule :

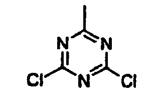

Parmi les groupes halogènes, on peut citer notamment le chlore, le brome, le fluor et l'iode, le chlore et le brome étant particulièrement préférés.

Par « haloacétyl », au sens de la présente description, on entend un radical acétyl CH₃-CO-, dont l'un des atomes d'hydrogène est substitué par un atome d'halogène, celui-ci étant tel que défini précédemment.

De préférence, X représente un groupe -COOH ou -NH₂ et L un groupe aliphatique substitué ou non, mieux encore un groupe -(CH₂)ₚ -, où p est un entier de 1 à 5.

On préfère tout particulièrement le composé de formule (Ia)

HOOC-(CH₂)₂-CH(PO₃H₂)₂ (Ia)

Selon un autre mode préférentiel, L représente un groupe L₁-CONH-L₂ et plus préférentiellement un groupe -(CH₂)ₙ-NHCO-(CH₂)ₘ- où n et m représentent un nombre entier de 0 à 5 et X représente -COOH ou -NH₂.

Bien entendu, entre également dans le cadre de l'invention le couplage de la fonction X et du biovecteur de façon indirecte, c'est-à-dire via un réactif homobifonctionnel ou hétérobifonctionnel. A titre illustratif de réactif homobifonctionnel, le glutaraldéhyde peut convenir pour réaliser le couplage, par exemple, d'une fonction X = -NH₂ avec une fonction -NH₂ du biovecteur.

Selon une variante préférée de l'invention, les fonctions X forment une liaison covalente L₃ avec le biovecteur de type
-CONH-, -COO-, -NHCO-, -OCO-, -NH-CS-NH-, -C-S-, -N-NH-CO-, -CO-NH-N-, -CH₂-NH-, -N-CH₂-, -N-CS-N-, -CO-CH₂-S-, -N-CO-CH₂-S-, -N-CO-CH₂--CH₂-S-, -CH=NH-NH-, -NH-NH=CH-, -CH=N-O-, -O-N=CH- ou répondant aux formules suivantes :

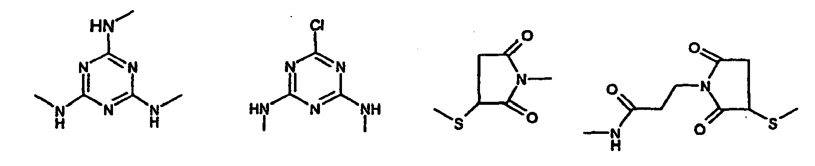

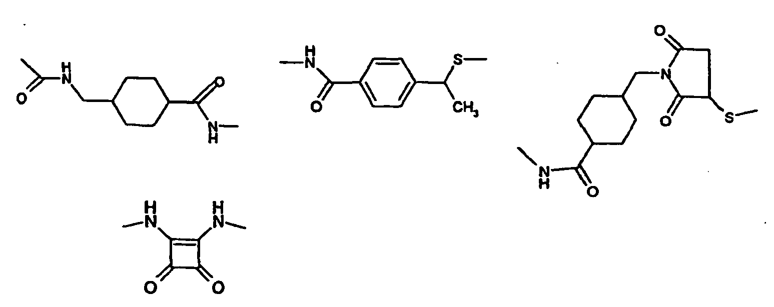

Selon un mode de réalisation particulier, on peut envisager de coupler deux biovecteurs sur une même fonction X via une liaison de type :

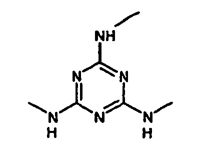

Les sites protonés des particules magnétiques acides (p) selon l'invention peuvent être complexés par au moins deux composés de formule (I) de structures identiques ou différentes.

Selon un mode de réalisation préféré, les particules magnétiques (p) sont complexées par une ou plusieurs molécules de deux composés de formule (I) de structures différentes, notamment de fonctions X différentes. Ceci permet avantageusement le greffage ultérieur de biovecteurs possédant des fonctions susceptibles de réagir avec des fonctions X différentes.

Selon un mode préférentiel, tout ou partie des fonctions X du composé *gem*-bisphosphonate sont couplées à un biovecteur

L'invention concerne ainsi également une composition (utilisable comme produit de contraste pour IRM) comprenant une suspension aqueuse stable de nanoparticules acides (p) dont au moins 90% des sites protonés sont complexés par un ou plusieurs composés *gem*-bisphosphonates identiques ou différents de formule (I) :

X-L-CH(PO₃H₂)₂ (I)

dans laquelle les fonctions X sont couplées en tout ou partie à au moins un biovecteur.

Par l'expression "en tout ou partie", on entend que toutes les fonctions X du composé *gem*-bisphosphonate ne sont pas nécessairement couplées à un biovecteur, mais que le taux de couplage est suffisant pour que la spécificité diagnostique recherchée soit obtenue avec le ou les biovecteurs utilisés. On précise aussi que si plusieurs X et/ou plusieurs biovecteurs sont utilisés, les X et/ou les biovecteurs peuvent être identiques ou différents.

La particule ainsi vectorisée s'écrit :

(BRANCHE)r- PARTICULE ACIDE (III)

dans laquelle r représente le nombre de branches greffées par particule et BRANCHE défini par:
[((BIOVECTEUR) - L₃)ₛ - L -CH (PO₃H₂)₂], L₃ étant une liaison covalente telle que définie ci-dessus résultant du couplage de la fonction X du composé de formule (I) et du biovecteur, éventuellement via un réactif homobifonctionnel ou hétérobifonctionnel ; s représente le nombre de biovecteurs couplés par fonction X, et peut être notamment égal à 1, 2 ou 3.

### BIOVECTEUR

Par "biovecteur", au sens de la présente invention, on entend de préférence une entité de ciblage spécifique (généralement un ligand), c'est-à-dire possédant une affinité spécifique pour un tissu et/ou pour un récepteur biologique et/ou un transporteur, et/ou une matrice biologique cibles donnés.

Les couples d'affinités récepteurs/ligands sont multiples et l'invention telle que définie pourra être aisément appliquée par l'homme du métier à différents ligands susceptibles de former une paire d'affinité avec un récepteur cible tels que les couples anticorps/antigènes, lectines/polysaccharides, biotine/avidine, acides nucléiques (brin + et -), étant entendu que le ligand puisse être couplé par liaison covalente soit directement à l'extrémité X du composé de formule (I), soit indirectement par l'intermédiaire d'un réactif bifonctionnel.

Par extension, au sens de la présente description, le terme "biovecteur" peut également couvrir des molécules organiques ayant une activité pharmacologique et/ou cytotoxique, ou plus généralement des pharmacophores utiles dans les méthodes de traitement thérapeutique ou prophylactique.

Le terme "pharmacophore" couvre au sens de la présente invention des molécules organiques à activité pharmacologique et/ou cytotoxique, ainsi que leurs analogues structuraux, ces derniers pouvant posséder ou non une activité pharmacologique et/ou cytotoxique.

Au sens de la présente description, un "biovecteur" désigne par extension des molécules biocompatibles possédant un caractère hydrophile marqué, telles que les aminoalcools.

A titre de biovecteurs préférés, on peut notamment citer les protéines éventuellement recombinantes ou mutées, les glycoprotéines, les lectines, la biotine, les vitamines, les dérivés ptéroïques ou aminoptéroïques, les dérivés de l'acide folique et antifolique, les anticorps ou fragments d'anticorps, les peptides et leurs dérivés, les mono- ou polysaccharides, l'avidine, les inhibiteurs ou substrats de récepteurs (membranaires ou nucléaires), les phospholipide, les stéroïdes et leurs analogues, les oligonucléotides, les séquences d'acide ribonucléique, les séquences d'acide désoxyribonucléique, les hormones ou substances analogues aux hormones, les acides aminés, les molécules organiques à activité pharmacologique, les pharmacophores, les protéines éventuellement recombinantes ou mutées, les anticorps ou fragments d'anticorps, les aminoalcools, les dérivés de phospholipide, les pharmacophores ; les dérivés de l'acide folique et antifolique, les agents de ciblage d'intégrines ou de MMP étant particulièrement préférés.

Les biovecteurs, dont notamment les anticorps et les pharmacophores, peuvent être éventuellement fonctionnalisés de manière à pouvoir réagir avec les fonctions X et former une liaison covalente de préférence de type
-CONH-, -COO-, -NHCO-, -OCO-, -NH-CS-NH-, -C-S-, -N-NH-CO-, -CO-NH-N-, -CH₂-NH-, -N-CH₂-, -N-CS-N-, -CO-CH₂-S-, -N-CO-CH₂-S-, -N-CO-CH₂-CH₂-S-, -CH=NH-NH-, -NH-NH=CH-, -CH=N-O-, -O-N=CH- ou répondant aux formules suivantes :

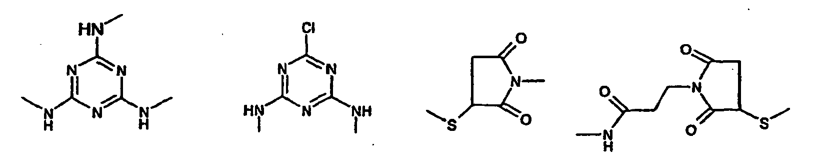

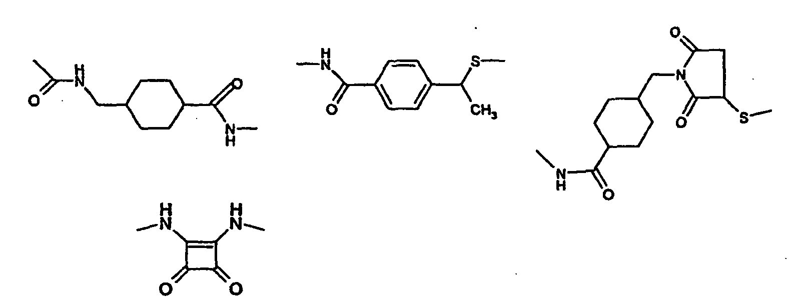

Le terme "dérivés de l'acide folique et dérivés antifoliques" désigne des molécules obtenues par fonctionnalisation d'au moins un groupe chimique du folate ou de l'acide ptéroïque de manière à ce qu'elles puissent former une liaison covalente avec les fonctions X, en particulier du type de celles mentionnées ci-dessus. A titre d'exemple de fonctionnalisation, on peut citer notamment l'éthérification d'une fonction hydroxy, estérification ou amidification d'une fonction carboxylate, ou la modification et/ou l'introduction de substituants sur le motif structural (2-amino-pyridiminique) et plus particulièrement sur le motif structural (2-amino-pyrimidinique) présent dans les dérivés aminoptérinique et ptéroïque. A titre d'exemples préférés des dérivés du folate, on peut citer plus particulièrement ceux décrits dans l'article de M.P Costi - Current Drug Targets (2001), vol 2, p. 135-166*.*

A titre d'exemples préférés de dérivés de « phospholipides », on peut citer les dérivés de phosphatidylsérine, de phosphatidyléthanolamine, de phosphatidylcholine, de phosphatidylinositol, de sphingomyéline ou les dérivés de céramides, le terme "dérivé" signifiant ici que les molécules sont fonctionnalisées de manière à ce qu'elles soient capables de réagir covalemment avec les fonctions X décrites précédemment, notamment avec X = -COOH ou -NH₂.

Par "dérivé de peptide", on entend, au sens de la présente description, un peptide issu d'acides aminés naturels ou non, dont la structure native a été modifiée chimiquement par fonctionnalisation de certaines fonctions chimiques, de manière à ce que le peptide résultant soit capable de réagir covalemment avec les fonctions X, par exemple par modification d'une liaison peptidique. A titre indicatif, un dérivé de peptide peut désigner un polypeptide, un pseudo-peptide, un rétropeptide, un peptidomimétique, un peptide cyclique, des peptides inhibiteurs d'enzymes, des peptides agonistes ou peptides antagonistes. A titre d'exemples préférés, on peut citer les peptides fonctionnalisés, utilisés en Médecine nucléaire (Référence : S. Liu and D. Scott Edwards ; Topics in Current Chemistry (2002), 222, p 259-278) et Okarvi, Nuclear Medicine Communication, 1999, 20 : 1093-1112) et les inhibiteurs ou substrats de protéases , en particulier de métalloprotéases.

Une classe particulière de biovecteurs sont les molécules à forte hydrophilie telles que les aminoalcools ou les aminopolyéthylèneglycols. Ces aminoalcools sont particulièrement utiles pour obtenir des particules magnétiques finales suffisamment hydrophiles en termes de stabilité aqueuse et de biocompatibilité, ainsi que pour moduler la pharmacocinétique et la biodistribution.

Parmi les aminoalcools, on préfère particulièrement ceux répondant à la formule (II) suivante : 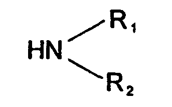 dans laquelle R₁ et R₂ sont identiques ou différents et représentent une chaîne hydrocarbonée aliphatique comportant de 2 à 6 atomes de carbone, substituée de préférence par 6 à 10 groupements hydroxyles, ou bien par 4 à 8 groupements hydroxyles dans le cas où R₁ et/ou R₂ est interrompu par un atome d'oxygène.

Dans ce cadre, les aminoalcools pour lesquels R₁ représente un groupe -(CH₂)-(CHOH)₄-CH₂OH ou -(CH₂)-CHOH-CH₂OH et R₂ un groupe -CH₂-(CHOH)₄-CH₂OH sont généralement préférés.

Parmi les aminopolyéthylèneglycols, on préfère particulièrement les composés de formule (II) dans laquelle R₁ et R₂, identiques ou différents, représentent H, un groupe alkyle ou une chaîne polyéthylèneglycol de formule - CH₂-(CH₂-O-CH₂)ₖ-CH₂OR₃ dans laquelle k varie de 2 à 100, et R₃ est choisi parmi H, alkyle ou -(CO)Alk, le terme "alkyle" ou "alk" désignant un groupe aliphatique hydrocarboné, linéaire ou ramifié, ayant environ de 1 à 6 atomes de carbone dans la chaîne.

Des exemples d'aminopolyéthylèneglycols sont notamment les composés O-(2-aminoéthyl)-O'-méthylpolyéthylèneglycol 1100, O-(2-aminoéthyl)-O'-méthylpolyéthylèneglycol 2000, O-(2-aminoéthyl)-O'-méthylpolyéthylèneglycol 750.

Selon une variante particulière de l'invention, le cas échéant, des biovecteurs de natures différentes sont couplés via les fonctions X à la surface de particules magnétiques (p). Dans ce cadre, on préfère tout particulièrement coupler sur chaque particule magnétique acide (p) deux biovecteurs différents.

Selon un mode particulier, on réalise un couplage "mixte" entre un aminoalcool et un biovecteur de nature différente, i. e. présentant soit une affinité spécifique pour une cible donnée, soit une activité pharmacologique et/ou cytotoxique, de manière à modifier la biodistribution du produit final.

On préfère que 1 à 100 % des fonctions X des composés *gem-*bisphosphonates soient couplées à au moins deux biovecteurs identiques ou différents. A titre d'exemple, 30 % des fonctions X peuvent être couplées à un biovecteur B1, 30 % à un biovecteur B2 et 30 % couplées à un biovecteur B3, B1, B2 et B3 étant différents.

S'il est envisageable de coupler 100% des fonctions X présentes à la surface des particules magnétiques acides (p) à des biovecteurs, on préfère généralement un rendement de couplage limitant l'augmentation de la taille hydrodynamique de la particule finale, tout en permettant d'assurer un ciblage spécifique efficace.

Aussi, selon un mode de réalisation préféré de l'invention, 1 à 70%, notamment 1 à 50% des fonctions X des composés de formule (I) sont couplées, et notamment lorsque les biovecteurs sont identiques.

Selon une variante également préférée de l'invention, dans le cas où les biovecteurs sont différents, en particulier lorsqu'il s'agit d'un aminoalcool et d'un biovecteur de nature différente, 1 à 100% des fonctions X des composés de formule (I) sont alors couplées.

De manière plus générale, un couplage mixte peut par exemple être utilisé avec des biovecteurs différents mais capables de cibler spécifiquement une même pathologie, tels que des peptides de séquences différentes capables de cibler différentes métalloprotéases (MMP) surexprimées dans une zone pathologique cancéreuse ou cardiovasculaire.

On décrit maintenant plus précisément différents biovecteurs, particulièrement utilisables dans le cadre de l'invention dans trois domaines :
- A) cardiovasculaire
- B) oncologie
- C) maladies inflammatoires et dégénératives.

### A) Domaine cardiovasculaire et la plaque d'athérome à risque

Différents systèmes/mécanismes biologiques présents dans les maladies cardiovasculaires notamment celles liées à la plaque d'athérome sont des cibles privilégiées pour les agents de contraste ou thérapeutiques selon l'invention notamment le système faisant intervenir des métalloprotéases (MMP), le système du thrombus, le système de l'annexine V.

On sait que les Matrix Metalloprotéinases (MMP) ou Matrixines, sont des enzymes qui possèdent la propriété de dégrader les composants protéiques de la matrice extracellulaire. Cette matrice extracellulaire qui entoure cellules et tissus est constituée de protéines comme le collagène. Les MMP ont été classées en 3 groupes : les gélatinases (Collagénases de type IV), les stromélisines, les collagènases interstitielles.

Les MMP sont surexprimées dans les plaques d'athérome. Dans le domaine cardiovasculaire, de nombreuses études indiquent que les MMPs sont impliquées dans le remodelage de la matrice extra-cellulaire au niveau de la plaque. Pour ce qui est des plaques d'athéromes, de manière non exhaustive au moins huit MMPs y sont surexprimées:

| Enzyme | Référence MMP |
|---|---|
| Collagénase interstitielle | MMP-1 |
| Gélatinase A | MMP-2 |
| Gélatinase B | MMP-9 |
| Stromélysine-1 | MMP-3 |
| Matrilysine | MMP-7 |
| Elastase macrophagique | MMP-12 |
| Collagénase-3 | MMP-13 |
| MMP-membranaire MT-MMP-1 | MMP-14 |

Les MMP1 et 3 sont notamment décrites dans Johnson J et al., Activation of Matrix-degrading Metalloproteinases by Mast Cell Proteases in Atherosclerotic Plaques, Arterioscl. Thromb. Vasc. Biol. 1998; 18: 1707-1715.

L'invention concerne ainsi des nanoparticules vectorisées de formule (III) dont le biovecteur est un agent capable d'inhiber au moins une MMP, notamment un inhibiteur de MMP, pour des applications dans le domaine cardiovasculaire. Selon une réalisation préférée, l'inhibiteur est un dérivé de l'illomastat ou un peptide tel qu'exemplifié plus loin. On préfèrera aussi des inhibiteurs de MMP choisis parmi ceux décrits dans Current Medicinal Chemistry, 2001, 8, 425-474 ; Chem.Rev, 1999, 99, 2735-2776. On pourra notamment utiliser des inhibiteurs de MMP désignés TIMP rappelés dans DDT vol 1, N°1, January 1996, Elsevier Science, 16-17 ; Bioconjugate Chem, 2001, 12, 964-971 .

Pour le thrombus, il est connu que :
- le récepteur GpIIb/IIIa est exprimé sur les plaquettes activées (il est déjà utilisé en thérapeutique comme cible d'agents antiplaquettaires) ;
- la fibrine est associée à la thrombose.

Plus précisément, en ce qui concerne le thrombus, l'intervention de glycoprotéines GpIIb/IIIa sur les plaquettes activées a été démontrée. Les plaquettes sont des fragments anucléés des mégacaryocytes de la moelle osseuse qui jouent un rôle pivot dans les processus d'athérosclérose et de thrombose. Les médicaments antiplaquettaires classiques les plus utilisés sont l'aspirine, la ticlopidine et le clopidogrel. La connaissance des mécanismes moléculaires aboutissant à l'agrégation plaquettaire a permis le développement d'une nouvelle famille de molécules dirigées contre le récepteur plaquettaire du fibrinogène, l'intégrine GPIIb/IIIa. L'avantage majeur par rapport aux antagonistes cités précédemment est de bloquer l'étape finale de l'activation des plaquettes, indépendamment de la voie d'activation des plaquettes. L'interaction plaquette-plaquette étant critique pour la formation du thrombus, la fixation du fibrinogène (qui forme des ponts entre les plaquettes) sur le complexe GP IIb/IIIa est un événement clé dans l'hémostase et la thrombose. Quand les plaquettes sont activées, les récepteurs glycoprotéiques GP IIb/IIIa qui se trouvent à la surface des membranes des plaquettes, subissent une modification de leur conformation spatiale et peuvent alors fixer des molécules de fibrinogène soluble dans le plasma et du calcium. Le fibrinogène établit les liens entre les plaquettes par l'intermédiaire de ponts Ca²⁺-fibrinogène formant un réseau dans lequel vont être emprisonnées les hématies. La thrombine en transformant le fibrinogène en fibrine resserre les mailles de ce filet. Cette agrégation va conduire à la formation d'un thrombus au niveau de la zone lésée. De nombreux efforts ont donc été réalisés afin d'identifier sur le récepteur GP IIb/IIIa, les sites d'interaction des ligands. Le complexe GP IIb/IIIa est un complexe hétérodimérique glycoprotéique membranaire important des plaquettes (environ 50000 copies par plaquette).

Au moins deux séries de peptides, correspondant à des séquences d'acides aminées présentes naturellement dans le fibrinogène humain, sont connues pour inhiber la fixation des macromolécules adhésives sur le récepteur GPIIb/IIIa : la séquence Arg-Gly-Asp (RGD) et la chaîne gamma Lys-Gln-Ala-Gly-Asp-Val.

La séquence Arg-Gly-Asp (RGD) a initialement été identifiée comme la séquence adhésive de la fibronectine, intégrine qui joue un rôle important dans les interactions plaquettes-plaquettes et plaquettes-vaisseaux après sa libération par les plaquettes. Cette séquence est également présente dans le fibrinogène, le facteur von Willebrand et la vitronectine (rôle dans la fibrinolyse et la liaison aux vaisseaux).

Le complexe GPIIb/IIIa reconnaît cette séquence qui inhibe la fixation de la fibronectine, du fibrinogène, du facteur de von Willebrand et la vitronectine sur les plaquettes. Tous ces ligands contiennent au moins une séquence RGD; alors que le fibrinogène en contient deux par demi-molécule. In vivo, le fibrinogène est le principal ligand du fait de sa forte concentration dans le plasma.

L'invention concerne ainsi des particules vectorisées (III) :
- dont le biovecteur est capable de cibler le récepteur GpIIb/IIIa ;
- dont le biovecteur est capable de cibler la fibrine, notamment par des peptides sélectifs des monomères de fibrine afin de différencier la fibrine de la molécule soluble de fibrinogène.

L'invention concerne en particulier des produits (III) dont le biovecteur comprend un motif RGD, pour des applications cardiovasculaires.

On pourra par exemple utiliser des peptides décrits dans WO 2001/9188, US 6 521 211, US 6 403 056, Seminars in nuclear medicine, 1990, 52-67, Nucear Medicine and Radiology, 28,2001,515-526, le bibapcitide Acutect de la société Diatide.

### B) Domaine oncologique

L'imagerie spécifique obtenue par la présente invention vise à obtenir selon une réalisation un marquage de la néo-angiogénèse, un ciblage spécifiquement des cellules tumorales ou des altérations de la matrice extracellulaire, non obtenues par les techniques connues. Différents systèmes (ou mécanismes) biologiques associés au développement tumoral sont des cibles privilégiées pour les agents de contraste ou thérapeutiques selon l'invention : le système faisant intervenir des composés capable de se lier à des récepteurs aux folates, le système faisant intervenir les MMP, le système faisant intervenir des facteurs de croissance impliqués dans l'angiogenèse.

L'invention concerne selon un mode de réalisation des produits (III) dont le biovecteur est un dérivé capable de cibler un récepteur au folate, ce biovecteur étant capable de donner lieu à une reconnaissance spécifique de cellules tumorales. On utilisera notamment comme BIOVECTEUR des dérivés du folate de formule (E) s'écrivant : 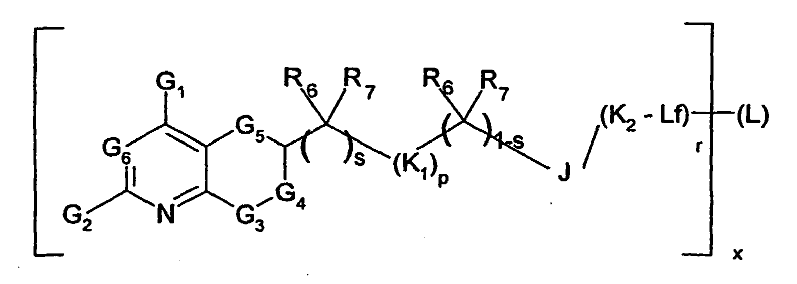 dans laquelle :
a) G1 est choisi indépendamment dans le groupe constitué par : halo, R_{f}2, O R_{f} 2, S R_{f} 3, N R_{f} 4 R_{f} 5 ; de préférence G1 est NH₂ ou OH ;
b) G2 est choisi indépendamment dans le groupe constitué par : halo, R_{f} 2, O R_{f} 2, S R_{f} 3, et N R_{f} 4 R_{f} 5 ;
c) G3, G4 représentent des groupes divalents choisis indépendamment dans le groupe constitué par -(R_{f} 6') C=, -N=, -( R_{f} 6') C (R_{f} 7')-, -N (R_{f} 4')- ;
   de préférence, G3 est -N= (acide folique) ou -CH- (composés décrits plus loin : CB3717, raltitrexed, MAI) lorsque le cycle comprenant G3 est aromatique, et G3 est -NH- ou -CH2- (composés décrits plus loin : AG-2034, lometrexol) lorsque le cycle comprenant G3 est non aromatique ;
   de préférence, G4 est -CH- ou -C(CH3)- lorsque le cycle comprenant G3 est aromatique, et -CH2- ou -CH(CH3)- lorsque le cycle comprenant G3 est non aromatique ;
e) G5 est absent (composé pemetrexed) ou choisi parmi -( R_{f} 6') C=, -N=, -(R_{f} 6') C (R_{f} 7')-, -N (R_{f} 4')- ;
f) Le cycle J est un cycle aromatique hétérocyclique ou non à 5 ou 6 sommets, les atomes du cycle pouvant être C, N, O, S ;
vii) G6 est N ou C (composé décrit plus loin : 3-deaza-ICI-198,583) ;
h) K1 et K2 sont choisis indépendamment dans le groupe constitué par - C (Z_{f})-,-C (Z_{f}) O-,-OC (Z_{f})-,-N (R_{f} 4")-,-C (Z_{f})-N (R_{f} 4), -N (R_{f} 4")-C (Z_{f}),-OC(Z)-N(R_{f} 4")-, -N(R_{f} 4")-C(Z_{f})-O-, N(R_{f} 4")-C(Z_{f})-N(R_{f} 5")-, -O-, -S-, -S(O)-, -S(O)2-, -N(R_{f} 4")S(O)2-, -C(R_{f} 6")( R_{f} 7")-, -N(C ≡ CH)-, -N(CH2-C ≡ CH)-, C1-C12 alkyle et C1-C12 alkoxy; dans lequel Zf est O ou S ; de préférence K1 est -N(R_{f}4 ")- ou -C(R_{f} 6")( R_{f} 7")- avec R_{f}"4, R_{f} 6", R_{f} 7" étant H ; A2 étant ou non lié de manière covalente à un acide aminé ;
i) R_{f} 1 est choisi dans le groupe constitué par : H, halo, C1-C12 alkyl, et C1-C12 alkoxy ; R_{f} 2, R_{f} 3, R_{f} 4, Rf4', R_{f} 4", R_{f} 5, R_{f} 5"', R_{f} 6" et R_{f} 7" sont choisis indépendamment dans le groupe constitué par : H, halo, C1-C12 alkyle, C1-C12 alkoxy, C,-C, 2 alkanoyle, C,-C, 2 alcényle, C1-C12 alcynyle, (C1-C12 alkoxy)carbonyle, et (C,-C, 2 alkylamino) carbonyle ;
j) R_{f} 6 et R_{f} 7 sont choisis indépendamment dans le groupe constitué par : H, halo, C1-C12 alkyle, C1-C12 alkoxy ; ou R_{f} 6 et R_{f} 7 forment ensemble O= ;
k) R_{f} 6' and R_{f} 7' sont choisis indépendamment dans le groupe constitué par : H, halo, C1-C12 alkyle, C1-C12 alkoxy ; ou R_{f} 6' et R_{f} 7' forment ensemble O= ;
l) Lf est un lien divalent incluant le cas échéant un acide aminé naturel ou un poly acide aminé naturel, lié à K2 ou à K1 par son groupement alpha-amino par une liaison amide ;
m) n, p, r et s sont indépendamment 0 ou 1.

La formule (E) inclut les formes tautomériques, par exemple des composés pour lesquels G1 est OH, SH ou NH.

Pour les composés de l'invention dans lesquels au moins un des groupes K1, K2, R_{f} 1, R_{f} 2, R_{f} 3, R_{f} 4, R_{f} 4', R_{f} 4", R_{f} 5, R_{f} 5", R_{f} 6, R_{f} 7", R_{f} 6, R_{f} 7, R_{f} 6' et R_{f} 7' comprend un atome d'hydrogène, un groupe alkyle, alkoxy, alkylamino, alkanoyle, alcényle, alcynyle, alkoxy carbonyle, alkylamino carbonyle, le groupe comprend de préférence 1 à 6 atomes de carbone (C1-C6), encore de préférence 1 à 4 atomes de carbone (C1-C4).

Concernant les MMP dans le domaine oncologique, il est connu que les MMP ont deux fonctions distinctes :
a) elles participent à la dissémination tumorale en détruisant la matrice extra-cellulaire;
b) elles créent un environnement qui favorise la croissance et l'angiogénèse des tumeurs primaires et métastasées.

Les MMP exprimées dans les principales tumeurs humaines sont notamment les suivantes :
- cancer du sein : MMP-1,2,3,7,9,11,13,14 ;
- cancer colo-rectal : MMP-1,2,3,7,9,11 ;
- cancer du poumon : MMP- 2,3,7,9,11,14 ;
- cancer de la prostate : MMP-1,2,3,7,9.

Il y a une corrélation fréquente entre le stade de progression tumorale et le niveau d'expression des MMP. En général, les tumeurs malignes expriment une plus grande quantité de MMP que les tumeurs bénignes.

L'invention concerne ainsi des produits (III) dont le biovecteur est un inhibiteur de MMP, pour des applications dans le domaine oncologie. Selon des réalisations préférées, on utilisera des inhibiteurs choisis parmi ceux décrits dans Current Medicinal Chemistry, 2001, 8, 425-474 ; Chem. Rev, 1999, 99, 2735-2776. On pourra notamment utiliser des inhibiteurs de MMP désignés TIMP rappelés dans DDT vol 1, N°1, January 1996, Elsevier Science, 16-17 ; Bioconjugate Chem, 2001, 12, 964-971 .

En ce qui concerne l'angiogenèse, des études ont montré que l'angiogénèse est un facteur pronostique dans diverses tumeurs, notamment le cancer du sein, du rein, de la prostate, du côlon et du cerveau, ainsi que dans le mélanome.

L'invention concerne ainsi des produits (III) dont le biovecteur est capable de cibler un marqueur d'angiogenèse.

Il est montré que certains facteurs de croissance endothéliaux sont spécifiques des tumeurs. Les cellules endothéliales, qui constituent la paroi interne des vaisseaux, ne manifestent aucune tendance naturelle à la prolifération chez l'adulte sain. En revanche, lors de situations pathologiques, par exemple lors du développement de tumeurs ou de l'éclosion de métastases, les besoins accrus en oxygène et en apports nutritifs sont véhiculés par un accroissement de l'irrigation locale. Les tumeurs dérivent ainsi à leur profit un nouveau réseau vasculaire, processus connu sous le nom d'angiogénèse.

Le facteur de croissance des cellules endothéliales vasculaires (VEGF) est un facteur de croissance angiogénique puissant et sélectif. Il agit en stimulant au moins trois récepteurs sur la membrane extracellulaire : VEGFR-1 (Flt-1), VEGFR-2 (Flk-1 / -KDR) et NP-1. Les récepteurs aux VEGFs appartiennent à la grande famille des RTK (Tyrosine-Kinase Receptor). Ces protéines de la famille des intégrines possèdent une région extracellulaire capable de lier les ligands, un domaine transmembranaire et une région cytoplasmique portant l'activité tyrosine kinase. Dans le cas des VEGFR, le domaine kinase est interrompu par une courte séquence spécifique à chaque récepteur.

L'invention concerne ainsi des produits (III) dont le biovecteur est un agent capable de se lier à des récepteurs angiogéniques présents à la surface des cellules endothéliales.

On pourra utiliser notamment des biovecteurs (notamment des peptides obtenus par phage display) décrits dans les documents WO 01/012809, WO 01/83693, WO 02/057299 (peptides de 8 acides aminés ciblant VEGFR3) ; Nuclear Medicine Communications (1999), 20, Pharmacological Review, 179, 206, 2000 ; Journal of Biological Chemistry,2000,275,13588-13596 ; Journal of Biological Chemistry, 2002,277,45,43137-43142 ; J. Mol. Biol, 2001, 316, 769-787 ; Biochemisty, 1998, 37, 17754-17772 ; Chem. J. Biochem. Mol. Biol, 2000, 16, 803-806. On pourra notamment utiliser (i) des inhibiteurs de l'activité enzymatique liée aux VEGF tels que des composés quinazolines, aminothiazoles, anthranilamide, (ii) des composés antagonistes de VEGF, notamment des petites molécules telles que des dibenzothiophènes et des molécules de documents JP2001-353075, JP2000-395413, des anticorps. On pourra aussi utiliser des biovecteurs du document US 6,610,269 qui décrit de nombreux agents de ciblage de récepteurs surexprimés en cas d'angiogenèse.

On sait aussi que l'intégrine αvβ3 est peu exprimée dans le système vasculaire mais est surexprimée dans les cellules tumorales (glioblastome dernier stade, carcinome ovarien, mélanome). L'αvβ3 prend part à l'angiogénèse à différents stades: l'αvβ3 régule l'adhésion des cellules endothéliales à la matrice, il transmet des signaux aux noyaux des cellules et est un récepteur pro-angiogénique en coopérant avec le récepteur du facteur de croissance des cellules endothéliales (VEGFR-2, flk). L'αvβ3, en action avec la metalloprotéinase-1 de la membrane (MT1-MMP) est responsable de l'activation de la metalloprotéinase-2 de la matrice à la surface des cellules. Le blocage de ce récepteur mais aussi de l'αvβ5 par des peptides RGD ou par des anticorps induit l'apoptose des cellules. L'αvβ5 comme l'αvβ5 interviennent surtout dans la phase de prolifération de l'angiogénèse. De nombreuses protéines de la matrice possèdent une séquence commune: Arg-Gly-Asp (notée RGD) qui a été identifiée comme le site d'interaction avec certaines intégrines. Un grand nombre d'inhibiteurs d'angiogénèse comprenant cette séquence RGD ont ainsi été développés.

L'invention concerne ainsi des produits (III) dont le biovecteur est un peptide RGD approprié pour des applications en oncologie.

Pour les peptides RGD ciblant l'αvβ3, les inventeurs préfèrent des biovecteurs qui permettent d'inhiber l'angiogénèse présentant une forte affinité pour l'αvβ3 (afin d'empêcher la fixation des protéines de la matrice) mais une faible affinité pour l'αIIbβ3. En effet, il a été montré que les antagonistes de l'αIIbβ3 pouvaient provoquer des problèmes de saignement indésirables. Les inventeurs préfèrent en particulier des antagonistes ayant une séquence peptidique RGD cyclique plus stable face à la dégradation enzymatique et une meilleure affinité et sélectivité, la conformation du peptide étant maintenue dans une position privilégiée en raison de la restriction des rotations.

Plus précisément, après une analyse structure activité des peptides RGD, les inventeurs préfèrent tout particulièrement, pour être dans des conditions favorables à un maintien de l'affinité pour l'αvβ3, des peptides RGD cycliques afin de prévenir la dégradation par les enzymes (exo- et endopeptidase), avec un cycle assez court afin qu'il soit plus rigide (cycle à 5 préférable à un cycle à 6 acides aminés), avec un acide aminé de configuration D. La distance entre les carbones β de l'acide aspartique et de l'arginine est typiquement inférieure à 6,6 Å pour avoir une sélectivité plus importante pour l'αvβ3 que pour l'αIIbβ3 (dont le site est plus large que celui de l'αvβ3). Un acide aminé hydrophobe voisin de l'acide aspartique favorise en outre une meilleure sélectivité. Une telle structure est optimale pour une bonne exposition des fonctions hydrophobes et hydrophiles dans le site du récepteur. Les inventeurs préfèrent en particulier le peptide cyclo(Arg-Gly-Asp-Dphe-Val) aussi noté cyclo(RGDfV), les lettres minuscules indiquent une configuration D de l'acide aminé correspondant. Il présente un IC₅₀ de l'ordre du nanomolaire (2 - 2,5nM). Ce peptide est particulièrement intéressant pour la fonction amine latérale de la lysine qui peut réagir avec un dérivé FR. Sa synthèse est décrite dans X. Dai, Z. Su, J.O. Liu ; Tetrahedron Letters (2000), 41, pp6295-6298.

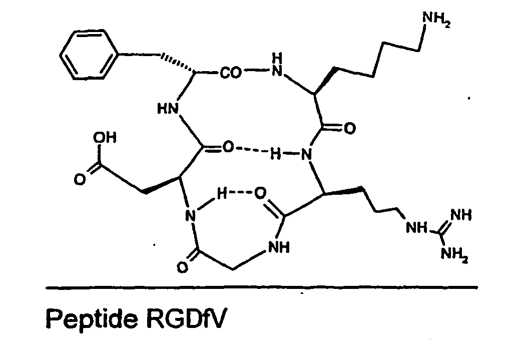

Il permet le couplage à des USPIO (particules fonctionnalisées) fonctionnalisés notamment COOH, squarate ou isothiocyanate. Les inventeurs ont eu à surmonter les difficultés techniques liées au choix des protections des acides aminés, au choix du milieu de couplage.

On pourra utiliser aussi des composés présentant une conformation de flexibilité restreinte, ces peptides ayant une bonne affinité et sélectivité pour αvβ3 :
- Des peptides dont le motif RGD est placé entre deux cystéines, l'activité pour l'αvβ3 augmentant, par exemple (cyclo(CRGDC)).
- Des peptides dont le groupement RGD n'est pas dans le cycle mais encadré par deux cycles dont au moins l'un d'entre eux était formé par un pont disulfure entre 2 cystéines (WO 02/26776).

On pourra utiliser aussi :
- des biovecteurs polypeptides choisis de manière telle qu'ils interagissent in vivo avec au moins une enzymes notamment MMP, cette interaction conduisant à une liaison plus forte avec une protéine cible ;
- des biovecteurs comprenant un site de clivage enzymatique, le clivage entraînant une modification de conformation ;
- des biovecteurs dérivés d'anticorps comme le LM609 (Nature Medicine, vol 4, 5, mai 1998, 623)
- des biovecteurs mimant les peptides RGD ayant une structure de type quinolone, benzodiazépine.

L'invention concerne aussi des produits (III) dont le biovecteur est un peptidomimétique du peptide RGD, approprié pour des applications en oncologie. On pourra utiliser :
- des peptides avec substitution d'une ou deux liaisons peptidiques par des liaisons thioamides (CSNH) ou par des groupements cétométhylene (COCH₂) dans la mesure où les substitutions n'induisent pas de changement de conformation importante ;
- des peptides (EMD 121974 aussi appelé Cilengitide : cyclo(RGDf-N(Me)V), qui présentent une N-méthylation de chacun des acides aminés du peptide cyclique (RGDfV), ce peptide présentant une très forte affinité (IC₅₀ = 0,58nM) et une sélectivité très importante (1500 fois plus importante que pour l'αIIbβ3) ; 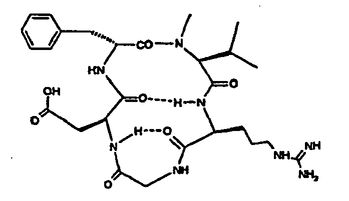
- des peptides possédant la séquence suivante cyclo(Xaa-Yaa-GD-Mamb), le groupement Mamb étant le N-aminométhyl acide benzoïque. Il est démontré que les DXaa-N-MeArg sont des antagonistes très actifs de l'αIIbβ3 alors que les antagonistes LXaa-Arg sont sélectifs de l'αvβ3 ; 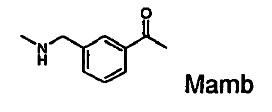
- le peptide cyclique (RGDD(tBuG)Mamb) présentant une affinité très importante pour l'αvβ3 (IC₅₀ = 0,6nM contre 14 µM pour αIIbβ3) due au caractère hydrophobe de l'acide aminé voisin de l'acide aspartique et au groupement Mamb qui rend le peptide moins flexible en solution aqueuse ;
- le peptide XJ735 (DUPONT) possédant le groupement : cyclo(ARGD-Mamb). Celui-ci permet d'inhiber la fixation du fibrinogène au récepteur αvβ3 (IC₅₀ = 70 nM) mais ne bloque pas les autres intégrines (αvβ5, α5β1);
- des peptides obtenus en introduisant différents groupements à la place de Dphe-Val (ou fV) dans le peptide de référence RGDfV.

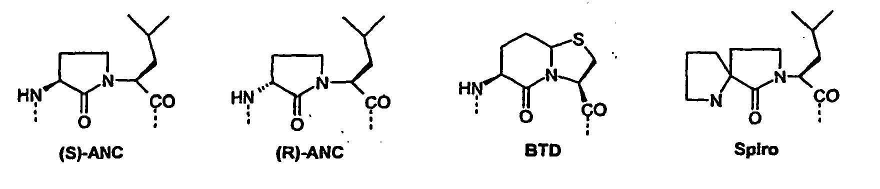

Ces groupements ont été synthétisés pour mimer le repliement βII' mais aussi pour réduire la flexibilité dans la région de rotation y. L'agent le plus actif est le cyclo(RGD-(R)-ANC) (IC₅₀ = 0,8nM). On peut aussi y introduire des sucres X à la place de ces deux acides aminés (fv) dans la séquence cyclo (RGDX) (Lohof E.et al; Angew. Chem. Int. Ed. Engl. (2000), 39 (15), pp. 2761-2764) ;
- des peptides avec introduction d'une double liaison dans le cycle (Kawaguchi, et al, Biochemical and Biophysical Research Communications (2001), 288(3), pp. 711-717) ; 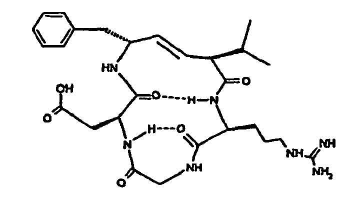
- des peptides décrits dans Chemlibrary.bri.nrc.ca.

L'invention concerne aussi des produits **(III)** dont le biovecteur est une molécule non peptidique, ciblant l'intégrine αvβ3, appropriée pour des applications en oncologie. On pourra utiliser les composés du tableau suivant, dont l'affinité nanomolaire est démontrée. 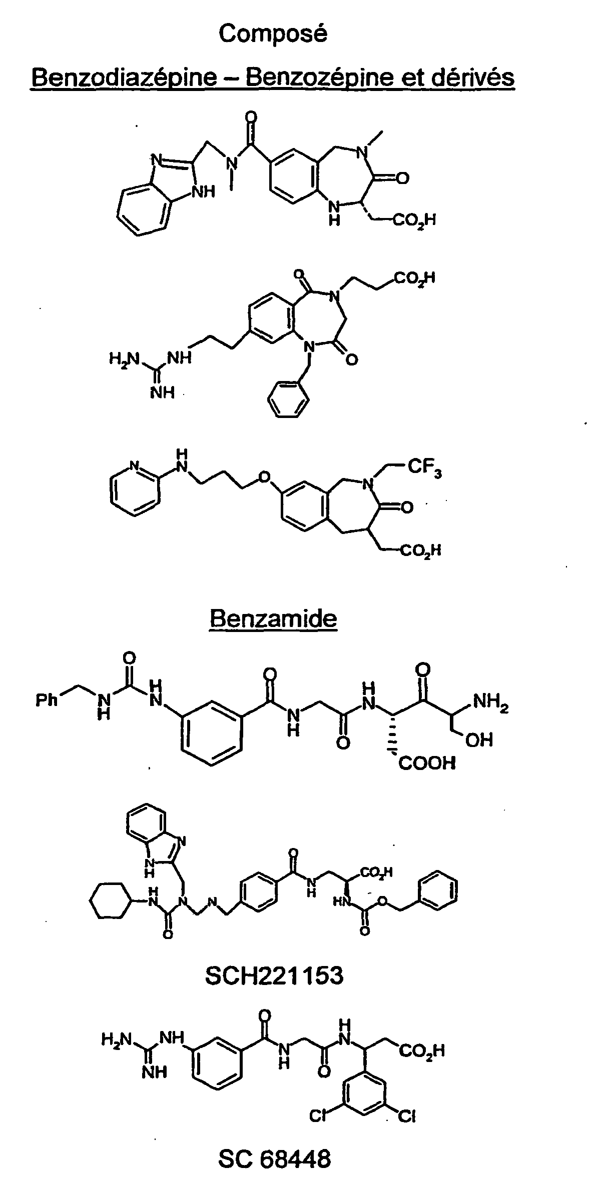 Et autres composés du document WO 01/97861 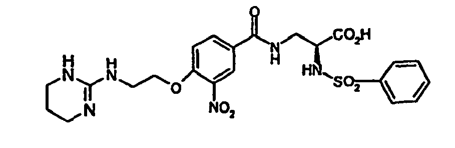 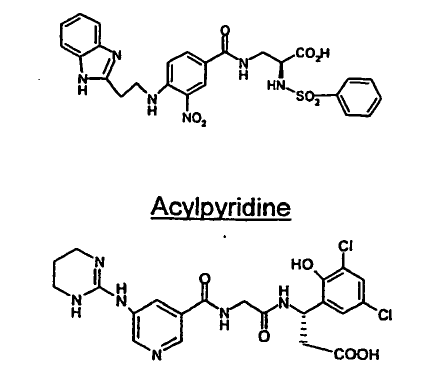 et autres composés du document WO 9952896 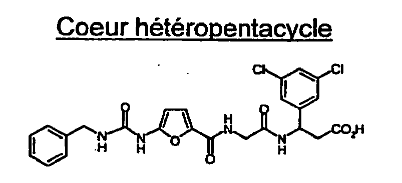 et autres composés du document Lohof et al., Angew. Chem. Int. Ed. Eng 2000, 39(15), pp. 2761-2764 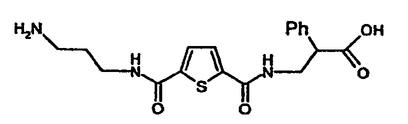 et autres composés à base d'acétylthiophène du document WO 0000486, 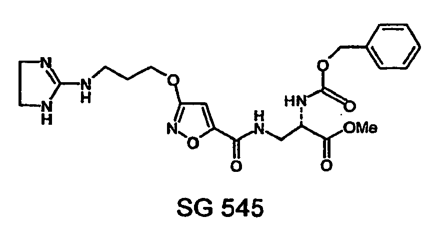 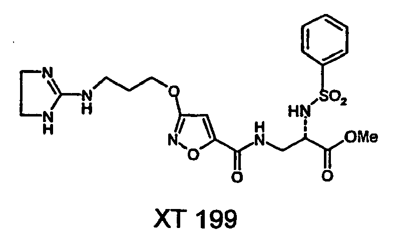 (et les composés SG256, SM256, XJ735 de Dupont Pharmaceuticals) 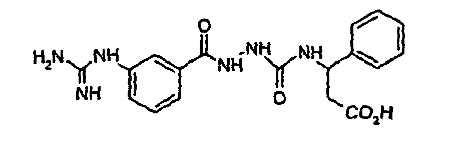 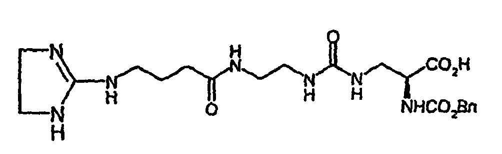 et analogues du document WO 0003973 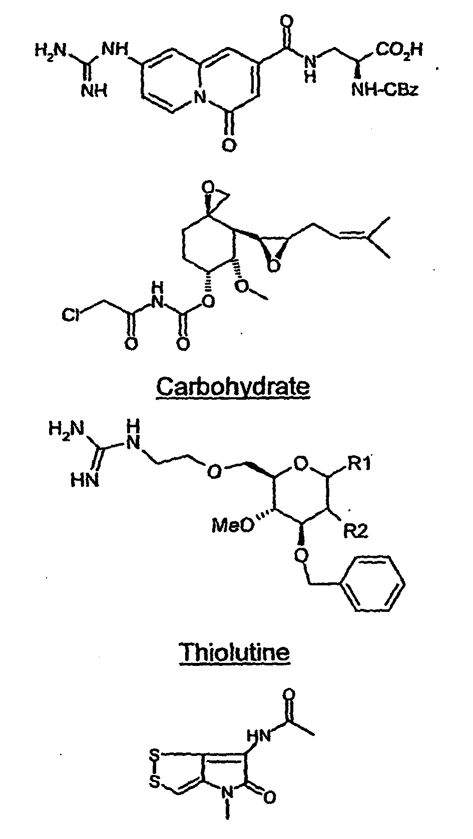 et autres composés à groupe pyrrothine

On pourra aussi utiliser des biovecteurs cités dans les documents US 6 537 520 (biovecteurs ciblant αvβ3 surexprimé lors de l'angiogenèse) et WO 01/198294 (coeur indazole). On pourra dans certains cas utiliser plusieurs biovecteurs différents dans un même composé pour augmenter les chances d'atteindre une même cible, par exemple un peptide RGD et une benzadiazépine pour l'αvβ3. En plus des biovecteurs de type RGD ou équivalents fonctionnels cités ci-dessus, on pourra utiliser les composés inhibiteurs de MMP suivants, sachant que la description détaillée donne des protocoles expérimentaux qui permettent de finaliser un criblage *in vitro* ou *in vivo :*
- des peptides, peptidomimétiques, non peptides fonctionnellement équivalents, efficaces sur le plan diagnostique ou thérapeutique, choisis parmi les inhibiteurs commercialisés, notamment des catalogues 2002 de Bachem, Amersham ;
- des inhibiteurs décrits dans les documents WO 01/60416, WO 01/60820, WO 2001/92244, EP 558 635, EP 663 823 ;
- des inhibiteurs de type hydroxamates, pyrrolidines hydroxamates, hydroxamates bicycliques, cyclobutyl hydroxamates, succinyl hydroxamates, sulfonamides hydroxamates, alanine hydroxamates, tels que décrits dans les documents EP 793 641, EP 766 665, EP 740 655, EP 689 538, EP 575 844, EP 634 998, WO 99/29667, EP 965 592, EP 922 702, WO 99/52889, WO 99/42443, WO 01/60416 (Dupont ; en particulier de formules Ia et Ib) ;
- des inhibiteurs à base d'acide phosphinique tels que décrits dans les documents EP 725 075, US 5 679 700, WO 98/03516, EP 716 086, WO 2000 74681, WO 2000 04030 ;
- des inhibiteurs à base d'imides cycliques (US 5 854 275) ;
- des inhibiteurs à base de sulfonamides tricycliques (WO 2000 06561) ;
- des inhibiteurs à base d'acide oxobutyrique ;
- des dérivés de TIMPS (Bioconjugate Chem, 2001,12, 964-971).

Toujours dans le domaine oncologique, des composés (III) dont le biovecteur est à base de phosphonates ou de bisphophonates sont très utiles pour le cancer des tissus osseux. Ces composés sont aussi utiles pour des maladies associées à des problèmes osseux, dues à des problèmes d'immunité (maladies auto-immunes comme l'arthrite rhumatoïde), à des maladies métaboliques (ostéoporose...), des maladies infectieuses.

Dans ces composés, le biovecteur est, par exemple, un biovecteur décrit dans le document WO 02/062398. On peut aussi utiliser un bisphosphonate décrit dans les brevets US 6 534 488, US 6 509 324, un bisphosphonate commercialisé tel que étidronate, clodronate, pamidronate, alendronate, ibandonate, YH 592, EB-1053 et analogues.

### C) Domaine des maladies inflammatoires et dégénératives

On ciblera notamment des biovecteurs ligands de récepteurs localisés sur les macrophages tels que les récepteurs SRA (Scavenger Receptor), les récepteurs Fc (US 2002/58284).

La progression de l'athérosclérose implique la captation des LDL puis leur oxydation au niveau des plaques. La phagocytose de ces LDL oxydées par les macrophages est médiée par un ensemble de récepteurs regroupés sous l'appellation de récepteurs éboueurs ou scavenger (SR). Cette famille de protéines membranaires joue donc un rôle majeur dans la transformation progressive des macrophages en cellules spumeuses. Les SR sont des protéines de surface membranaire capables de fixer les cellules sénescentes ainsi que les lipoprotéines modifiées chimiquement ou biologiquement. Les principaux groupes de SR se classent en différentes classes :
1/ Les SR de classe A : type I, type II et MARCO
2/ Les SR de classe B : type I, type II et CD36
3/ Les SR de classe D : CD68
4/ Les SR de classe E et F « lectin-like » : LOX-1
5/ Les SR récents non classés : SR-PSOX.

Comme rappelé dans le document US A 2002/0127181 et dans De Winther et al., ATVB 2000 ; 20: 290-297 ; Kunjathoor et al., J Biol. Chem. 2002 ; 277 : 49982-49988, le récepteur SRA est surexprimé par les macrophages lors de maladies cardiovasculaires (athérosclérose, plaque d'athérome, maladie des artères coronaires, thrombose, ischémie, infarctus du myocarde ...). L'utilisation de produits selon l'invention pour le ciblage de SRA associé à ces pathologies fait partie de l'invention.

L'invention couvre ainsi également, pour le diagnostic et/ou le traitement de maladies inflammatoires, des particules complexées par des composés de formule (I) associés :
1) à des biovecteurs ciblant le SR, notamment :
   1a) des biovecteurs décrits dans les documents US 6 255 298, US 6 458 845, WO 00/06147, WO 00/03704 ;
   1b) des lipoprotéines modifiées notamment des LDL acétylés (acLDL ; Gurudutta et al., Nucl. Med. Biol. 2001 28: 235-24) et des LDL oxides (oxLDL) ;
   1c) des ligands AcLDL, OxLDL, LPS décrits dans Esbach et al., Hepatology, 199318: 537; De Rijke et al., J. Biol. Chem, 1994, 269: 824; Van Oosten et al., Infect. Immun. 1998, 66 : 5107 ; Bijsterbosch et al., Nucleic Acids Res. 1997 25 : 3290; Biessen et al, Mol. Pharmacol. 53: 262, 1998
   1d) des peptides criblés par phage display capables de se lier au récepteur SR-AI;
2) à des biovecteurs ciblant des récepteurs aux folates (ces récepteurs aux folates sont surexprimés dans les macrophages activés) pour une utilisation dans des pathologies impliquant une activation de macrophages ;
3) à des peptides de ciblage de plaques amyloïdes entraînant notamment la maladie d'Alzheimer (par exemple décrits dans WO 01/74374, US 6 329 531);
4) à des biovecteurs de type CSF (GCSF, GM-CSF...) décrits par exemple dans le brevet US 6 491 893 ;
5) à des anticorps ou des fragments d'anticorps ciblant des récepteurs surexprimés sur les macrophages (CD68, MRP8-14 ...).

Dans le domaine des maladies inflammatoires, les inventeurs ont en particulier utilisé comme biovecteur la phosphatidylsérine ou un dérivé de la phosphatidylsérine, pour une utilisation dans le diagnostic de maladies liées aux macrophages.

La phosphatidylsérine (PS) est un phospholipide membranaire, principalement localisé sur la face interne de la cellule côté cytoplasmique. Sa charge globale négative stabilise sa polarité et l'empêche de diffuser au travers de la membrane plasmique. La PS sert de signal de reconnaissance pour le macrophage. La nature de ce signal reste encore inconnue (reconnaissance directe, densité de charges, récepteurs multiples, récepteur unique inductible). D'après les derniers travaux publiés, il s'agirait d'une interaction directe PS et récepteur à la PS (PSR) après induction de ce récepteur à la surface de certains macrophages présents dans la zone en rupture d'homéostasie. Chez certaines cellules macrophagiques, la PS interagit également avec les scavengers récepteurs via leur site d'attache pour les phospholipides anioniques. La PS est exprimée au niveau de la face interne de la membrane de toutes les cellules viables. En cas de souffrance cellulaire ou en début de processus apoptotique, une translocase membranaire provoque un basculement de la PS sur la face extracellulaire de la cellule. Cette expression extracellulaire constitue un signal de reconnaissance pour les macrophages qui reconnaissent et phagocytent la cellule en souffrance évitant ainsi une inflammation locale.

Les inventeurs ont préparé un agent de contraste lié à la PS ou dérivé, destiné à être activement capté par les macrophages afin d'imager ces diverses pathologies. Plusieurs difficultés techniques chimiques ont été surmontées, afin de préparer les biovecteurs PS suivants, le chélate étant couplé sur la fonction NH2 libre :

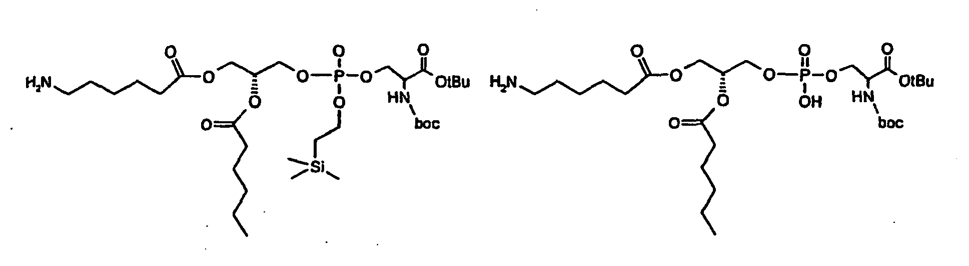

Afin de contrôler parfaitement la structure des produits finaux, il a été nécessaire de préparer des dérivés de PS correctement fonctionnalisés. La présence d'un résidu amino-acide sur la partie polaire de la PS est une source de difficultés supplémentaires qu'il a fallu gérer en pratiquant une chimie de protection/déprotection des fonctions amine et acide libres et/ou en utilisant des méthodes chimiques compatibles avec ces fonctions. Les groupes protecteurs du résidu amino-acide sont les groupements classiques utilisés en chimie des amino-acides (Boc, tBu, Z, Bn...). Les fonctions préférées pour assurer la liaison entre la PS et la particule superparamagnétique sont NH₂, COOH, SH.

Les inventeurs ont en outre préparé des produits vectorisés dans lesquels la phosphatidylsérine est dépourvue d'au moins une des chaînes grasses, l'affinité n'étant pas altérée de manière gênante.

En plus de ceux déjà cités plus haut dans la présente demande dans les domaines, on pourra aussi utiliser :
1) Les biovecteurs décrits dans les documents WO 01/97850 (ciblant des récepteurs VEGF et angiopoiétine), US 6,372,194 (polymère tel que polyhystidine), WO 2001/9188 (polypeptide ciblant la fibrine), WO 01/77145 (peptide de cibage d'intégrines), WO 02/26776 (peptide de ciblage d'intégrine αvβ3), WO 03/062198 (peptides de ciblage de métalloprotéases MMP), WO 99/40947 (peptides ciblant par exemple le récepteur KDR/Flk-l dont R-X-K-X-H et R-X-K-X-H, ou les récepteurs Tie-1 e 2), WO 02062810 et Müller et al, Eur. J. Org. Chem, 2002,3966-3973 (glycosides de sialyl Lewis), WO 02/40060 (antioxydants tels que l'acide ascorbique), US 6,524,554 (ciblage de la tuftsine), WO 02/094873 (ciblage de récepteurs à protéine G GPCR, en particulier la cholécystokinine), US 6,489,333 (association antagoniste d'intégrine et mime de la guanidine), US 6,511,648 (quinolone ciblant αvβ3 ou 5), US A 2002/0106325, WO01/97861 (benzodiazépines et analogues ciblant des intégrines), biovecteur de ciblage d'intégrine dont α5β1, WO 01/98294 (imidazoles et analogues), WO 01/60416 (inhibiteurs de MMP, notamment des hydroxamates), WO 02/081497 (peptides de ciblage d'αvβ3 tels que RGDWXE), WO 01/10450 (peptides RGD), US 6,261,535 (anticorps ou fragments d'anticorps (FGF, TGFb, GV39, GV97, ELAM, VCAM, inductible par TNF ou IL), US 5 707 605 (molécule de ciblage modifiée par interaction avec sa cible), WO 02/28441 (agents de ciblage de dépôts amyloïdes), WO 02/056670 (peptides clivés cathepsines), US 6,410,695 (mitoxantrone ou quinone), US 6,391,280 (polypeptides ciblant des cellules épithéliales), US 6,491,893 (GCSF),
   US 2002/0128553, WO 02/054088, WO 02/32292, WO 02/38546, WO20036059, US 6,534,038, WO 99/54317 (inhibiteurs de cystéines protéases), WO 0177102, EP 1 121 377, *Pharmacological Reviews* (52, n°2, 179 ; facteurs de croissance PDGF, EGF, FGF...), Topics in Current Chemistry (222, W. Krause, Springer), Bioorganic & Medicinal Chemistry (11, 2003, 1319-1341 ; dérivés tétrahydrobenzazépinones ciblant αvβ3).
2) Les inhibiteurs d'angiogenèse, notamment ceux testés en essais cliniques ou déjà commercialisés, notamment :
   - les inhibiteurs d'angiogenèse impliquant des récepteurs FGFR ou VEGFR tels que SU101, SU5416, SU6668, ZD4190, PTK787, ZK225846, des composés azacycles (WO 00244156, WO 02059110) ;
   - les inhibiteurs d'angiogenèse impliquant des MMP tels que le BB25-16 (marimastat), le AG3340 (prinomastat), le solimastat, le BAY12-9566, le BMS275291, le metastat, le neovastat ;
   - les inhibiteurs d'angiogenèse impliquant des intégrines tels que le SM256, le SG545, des molécules d'adhésion bloquant le EC-ECM (tels que le EMD 121-974, ou la vitaxine) ;
   - des médicaments à mécanisme d'action antiangiogenèse plus indirect tels que le carboxiamidotriazole, le TNP470, la squalamine, le ZD0101 ;
   - les inhibiteurs décrits dans le document WO 99/40947, les anticorps monoclonaux très sélectifs pour la liaison au récepteur KDR, les analogues de la somatostatine (WO 94/00489), les peptides de liaison à la sélectine (WO 94/05269), des facteurs de croissance (VEGF, EGF, PDGF, TNF, MCSF, interleukines); des biovecteurs de ciblage de VEGF décrits dans Nuclear Medicine Communications,1999, 20 ;
   - les peptides inhibiteurs du document WO 02/066512.
3) Des biovecteurs capables de cibler des récepteurs : CD36, EPAS-1, ARNT, NHE3, Tie-1, 1/KDR, Flt-1, Tek, neuropiline-1, endogline, pléientropine, endosialine, Axl., alPi, a2ssl, a4P1, a5pl, eph B4 (éphrine), récepteur laminine A, récepteur neutrophiline 65, récepteur leptine OB-RP, récepteur chimiokine CXCR-4 (et autres récepteurs cités dans le document WO99/40947), LHRH, bombésine/GRP, récepteurs gastrine, VIP, CCK, Tln4.
4) Des biovecteurs de type inhibiteurs de tyrosine kinase.
5) Les inhibiteurs du récepteur GPIIb/IIIa connus choisis parmi :
   (1) le fragment fab d'un anticorps monoclonal du récepteur GPIIb/IIIa, Abciximab (ReoPro^{™}),
   (2) les petites molécules peptidiques et peptidomimétiques injectées en intraveineuse telles que l'eptifibatide (Integrilin^{™}) et le tirofiban (Aggrastat^{™}).
6) Des peptides antagonistes de récepteurs au fibrinogène (EP 425 212), des peptides ligands de récepteurs IIb/IIIa, des ligands du fibrinogène, des ligands de la thrombine, des peptides capables de cibler la plaque d'athérome, les plaquettes, la fibrine, des peptides à base d'hirudine, des dérivés à base de guanine ciblant le récepteur IIb/IIIa.
7) D'autres biovecteurs ou fragments biologiquement actifs de biovecteurs connus de l'homme du métier comme médicaments, à action anti-thrombotique, anti-agrégation plaquettaire, antiathérosclérotique, antiresténotique, anticoagulante.
8) D'autres biovecteurs ou fragments biologiquement actifs de biovecteurs ciblant αvβ3, décrits en association avec des DOTA dans le brevet US 6 537 520, choisis parmi les suivants : mitomycine, tretinoine, ribomustine, gemcitabine, vincristine, étoposide, cladribine, mitobronitol, methotrexate, doxorubicine, carboquone, pentostatine, nitracrine, zinostatine, cetrorelix, letrozole, raltitrexed, daunorubicine, fadrozole, fotemustine, thymalfasin, sobuzoxane, nedaplatin, cytarabine, bicalutamide, vinorelbine, vesnarinone, aminoglutethimide, amsacrine, proglumide, elliptinium acétate, ketanserin, doxifluridine, etretinate, isotretinoine, streptozocine, nimustine, vindesine, flutamide, drogenil, butocin, carmofur, razoxane, sizofilan, carboplatine, mitolactol, tegafur, ifosfamide, prednimustine, picibanil, levamisole, teniposide, improsulfan, enocitabine, lisuride, oxymetholone, tamoxifen, progesterone, mepitiostane, epitiostanol, formestane, interferon-alpha, interferon-2 alpha, interferon-beta, interferon-gamma, colony stimulating factor-1, colony stimulating factor-2, denileukin diftitox, interleukin-2, leutinizing hormone releasing factor.
9) certains biovecteurs ciblant des types particuliers de cancers, par exemple des peptides ciblant le récepteur ST associé au cancer colo-rectal, ou le récepteur tachykinine.
10) des biovecteurs utilisant des composés de type phosphines.
11) des biovecteurs de ciblage de P-sélectine, E-sélectine ; par exemple le peptide de 8 acides aminés décrit par Morikawa et al, 1996, 951, ainsi que différents sucres.
12) l'annexine V ou des biovecteurs ciblant les processus apoptotiques.
13) tout peptide obtenu par des technologies de ciblage telles que le phage display, modifié éventuellement par des acides aminés non naturels (http//chemlibrary.bri.nrc.ca), par exemple des peptides issus de banques phage display : RGD, NGR, CRRETAWAC, KGD, RGD-4C, XXXY*XXX, RPLPP, APPLPPR.
14) d'autres biovecteurs peptidiques connus de ciblage de plaques d'athérome cités notamment dans le document WO 2003/014145.
15) des vitamines.
16) des ligands de récepteurs hormonaux dont les hormones et les stéroïdes.
17) des biovecteurs ciblant des récepteurs opioïdes.
18) des biovecteurs ciblant des récepteurs TKI.
19) des antagonistes LB4 et VnR.
20) des composés nitriimidazoles et benzylguanidines.
21) des biovecteurs rappelés dans Topics in Current Chemistry, vol.222, 260-274, Fundamentals of Receptor-based Diagnostic Metallopharmaceuticals, notamment :
   - des biovecteurs de ciblage de récepteurs peptidiques surexprimés dans les tumeurs (récepteurs LHRH, bombésine/GRP, récepteurs VIP, récepteurs CCK, récepteurs tachykinine par exemple), notamment les analogues de somatostatine ou de bombésine, des peptides dérivés octréotide éventuellement glycosylés, les peptides VIP, les alpha-MSH, les peptides CCK-B ;
   - des peptides choisis parmi : des peptides cycliques RGD, fibrine-chaîne alpha, CSVTCR, tuftsine, fMLF, YIGSR (récepteur : laminine).
22) des oligosaccharides, des polysaccharides et des dérivés d'oses, des dérivés ciblant Glu.
23) des biovecteurs utilisés pour des produits de type smart.
24) des marqueurs de la viabilité myocardique (tétrofosmine et hexakis2métoxy-2méthylpropylisonitrile).
25) des traceurs du métabolisme des sucres et des graisses.
26) des ligands de récepteurs de neurotransmetteurs (récepteurs D, 5HT, Ach, GABA, NA).
27) des oligonucléotides.
28) le facteur tissulaire.
29) des biovecteurs décrits dans WO 03/20701, en particulier le PK11195 ligand du récepteur périphérique aux benzodiazépines.
30) des peptides liant la fibrine, notamment les séquences peptidiques décrites dans WO 03/11115.
31) des inhibiteurs d'agrégation de plaques amyloïdes décrits dans WO 02/085903.
32) un biovecteur de type fluorochrome utilisable en imagerie optique.

Le cas échéant, on fera varier la longueur du linker L du composé (I) pour optimiser la biodistribution du composé (III) et/ou favoriser sa reconnaissance spécifique par la cible biologique.

### PARTICULES FINALES

Dans le cadre de l'invention, on privilégie notamment les particules finales, complexées et éventuellement couplées au(x) biovecteur(s) dont le diamètre hydrodynamique global est compris entre 5 nm et 200 nm, de préférence compris entre 5 et 60 nm.

Les compositions de particules magnétiques (p), éventuellement couplées aux biovecteurs sont généralement sous forme d'une suspension aqueuse éventuellement en présence d'un solvant organique miscible dans l'eau tel que le DMSO, l'acétone ou le méthanol, les suspensions biocompatibles étant particulièrement préférées.

Selon une variante privilégiée de l'invention, les compositions de particules magnétiques acides (p) complexées et éventuellement couplées à des biovecteurs comprennent un ou plusieurs véhicule(s) et/ou additif(s) pharmaceutiquement acceptables. Dans ce cadre, les compositions stériles sont particulièrement préférées.

### PROCÉDÉ DE PRÉPARATION DES PARTICULES

Selon un autre aspect, l'invention a pour objet un procédé de préparation de nanoparticules magnétiques (p) acides dont au moins 90% des sites protonés sont complexés par un ou plusieurs composés gem-bisphosphonates identiques ou différents de formule (I), les fonctions X étant susceptibles d'être couplées à au moins au biovecteur, ledit procédé comprenant la mise en contact d'une solution acide de particules magnétiques acides (p) à base d'un composé du fer, avec des composés de formule (I) tels que définis précédemment en quantité suffisante et la récupération des particules complexées obtenues.

Selon un autre aspect, l'invention a pour objet un procédé de préparation d'une composition d'une composition de nanoparticules magnétiques complexées par des composés (I) et recouvertes de biovecteurs, comprenant les étapes successives consistant à :
i) mettre en contact une solution acide de particules magnétiques acides (p) à base d'un composé du fer avec des composés de formule (I) tels que définis précédemment en quantité suffisante, et récupération des particules complexées obtenues ;
ii) réaliser le couplage de tout ou partie des fonctions X des composés de formule (I) complexés à la surface des particules magnétiques (p) avec des biovecteurs.

De préférence, l'étape i) comprend les étapes i1) et i2) suivantes :
i1) préparer une solution acide de particules magnétiques acides (p) à base d'un composé du fer ;
i.2) mettre en contact la solution du i.1) avec des composés de formule (I) tels que définis précédemment en quantité suffisante, et récupération des particules complexées obtenues.

Selon un mode de réalisation préféré du procédé selon l'invention, le ferrofluide acide mis en oeuvre à l'étape i) comprend des particules magnétiques à base de ferrite, de préférence de maghémite ou de magnétite.

Généralement, l'étape i) est réalisée en milieu aqueux, éventuellement en présence d'un solvant miscible dans l'eau, tel que le DMSO, l'acétone ou le méthanol, à un pH acide, de préférence à un pH de 1 à 3.

Sans vouloir se limiter à une quelconque théorie, lors de l'étape de complexation de particules magnétiques (p), les sites initialement protonés des particules magnétiques (p) interagissent avec l'extrémité *gem*-bisphosphonate des composés de formule (I) via l'expulsion de protons tel que décrit notamment dans la publication de N. Fauconnier et al. : Prog. Colloid Polym. Sci. (1996), 100, pp. 212-216. Les sites protonés des particules magnétiques acides (p) peuvent donc être considérés comme des « points d'accroche ou sites de fixation » des fonctions gem-bisphosphonates des composés de formule (I) à la surface de la particule magnétique.

Selon un mode de réalisation particulièrement préféré, on opère en présence d'une quantité allant de 1 à 3 équivalents molaires par rapport au nombre de moles de sites hydroxyles protonés à la surface de la surface de la particule (p), de manière à complexer au moins 70% et, de préférence, plus de 90% des sites protonés de la particule magnétique (p). Ceci représente, en général, environ 40 à 200 composés de formule (I) par particule selon la taille de celle-ci, le nombre de sites protonés en surface étant proportionnel à la surface de la particule magnétique acide (p).

De manière avantageuse, l'évaluation du taux de greffage qui définit le rapport du nombre de moles des composés de formule (I) au nombre de moles de sites protonés de la particule magnétique acide (p) peut être aisément déterminée par des méthodes conventionnelles telles que l'analyse élémentaire C, P, Fe. En effet, la particule magnétique acide (p) avant greffage du composé (I) ne comporte aucun atome de phosphore, ni de carbone. Or, après le greffage des composés (I) à la surface de la particule, la détermination du ratio phosphore/fer permet de connaître le nombre de mole de composé (I) greffé sur la particule magnétique acide (p).

Par ailleurs, le nombre de sites protonés par particule magnétique est accessible selon les techniques classiques connues de l'homme du métier, telle que la conductimétrie et les dosages acido-basiques.

De par ces outils analytiques, il est possible d'ajuster la quantité de composé de formule (I) en fonction du taux de greffage qu'on souhaite obtenir *in fine.*

Avantageusement, ce procédé permet donc de contrôler le greffage de la particule magnétique (p) par les composés de formule (I).

Contre toute attente, de façon particulièrement avantageuse par rapport à l'art antérieur qui n'utilisait pas de particules acides, les inventeurs ont de plus montré que, contrairement au cas d'un greffage sur des particules alcalines, la particule magnétique acide (p) greffée de composés de formule (I) est le plus souvent stable dans une gamme de pH allant de 3 à 12, et notamment au pH physiologique, ce qui constitue un avantage majeur pour réaliser le couplage ultérieur de biovecteurs, dont notamment de protéines ou d'anticorps sans les dégrader ou les dénaturer.

Cette propriété n'était pas évidente lorsqu'on sait que, de façon générale, le greffage d'un agent complexant, quel qu'il soit, à la surface d'une particule modifie la charge superficielle des particules et donc leur zone de stabilité en fonction du pH et de la force ionique.

De façon inattendue, les inventeurs ont observé en outre une très bonne sélectivité entre la fonction *gem*-bisphosphonate et la fonction réactive X : seules les fonctions *gem*-bisphosphonates sont complexées à la surface de la particule magnétique.

Avantageusement, contrairement à l'art antérieur, l'isolement des particules magnétiques complexées ne requiert pas d'étapes de purifications laborieuses, par exemple par dialyse ou traitement sur une résine.

Après complexation, les particules magnétiques acides (p) complexées par les composés de formule (I) forment un floculat qui peut être, selon un mode préférentiel, isolé du milieu réactionnel par simple décantation magnétique.

Préalablement à l'étape (ii), on procède de préférence à plusieurs lavages à l'eau du floculat récupéré de manière à éliminer les composés de formule (I) en excès n'ayant pas réagi.

A l'issue de l'étape (i), le floculat récupéré est généralement mis en suspension dans une solution aqueuse de pH compris entre 6 et 7, ceci conduisant à la désagrégation du floculat (peptisation) et à l'obtention d'une solution colloïdale.

Selon un mode de réalisation préféré, on réalise préalablement une étape consistant à suspendre le floculat formé à l'étape (i) dans une solution aqueuse de pH basique compris entre 10 et 11, avant de revenir à un pH compris entre 6 et 7. Ce traitement à pH basique vise, en effet, à renforcer la liaison du composé *gem*-bisphosphonate à la surface de la particule magnétique acide (p).

Selon un aspect avantageux, les particules magnétiques acides (p) complexées par les composés gem-bisphosphonates, obtenues à l'issue de l'étape (i) sont très peu polydispersées et présentent un profil en "taille hydrodynamique" très proche du ferrofluide acide de départ. Cette étape de complexation n'entraîne donc aucun phénomène d'agrégation, ce qui permet de s'affranchir de toute étape de filtration supplémentaire.

Selon un autre aspect avantageux, les particules magnétiques complexées par les composés *gem*-bisphosphonates sont obtenues avec de très bons rendements en fer, généralement compris entre 70 et 95 %.

Les particules magnétiques acides (p) greffées par des composés de formule (I) sont avantageusement stables au pH physiologique en présence, éventuellement, d'un solvant, ce qui permet de réaliser le couplage des biovecteurs directement en milieu ferrofluide.

L'art antérieur ne suggérait nullement de greffer des composés gem-bisphosphonates sur des particules acides.

Selon un autre aspect avantageux, la fonction réactive X du composé de formule (I) greffée à la surface de la particule possède une très bonne réactivité vis-à-vis des biovecteurs, et permet d'obtenir des rendements de couplage élevés. Ceci permet donc de diminuer les pertes en biovecteurs utilisés et de diminuer ainsi le coût de la particule magnétique spécifique obtenue *in fine.*

Un autre aspect particulièrement avantageux est la détermination du taux de couplage du biovecteur sur la particule magnétique (p) greffée par des composés de formule (I). En effet, la composition déterminée par analyse élémentaire (C, P, Fe) de la particule magnétique (p) greffée par des composés de formule (I) permet de connaître exactement le ratio Phosphore/Fer et l'analyse élémentaire des particules finales sur lesquelles est greffé un biovecteur permet de la même façon de déterminer le ratio Azote/Phosphore ou Carbone/Phosphore, ce qui permet de calculer le nombre de biovecteurs sur chaque particule. Ce type d'analyse est totalement impossible sur les particules magnétiques obtenues par synthèse dite « de Molday » enrobées avec des polymères comme le dextrane sur lesquelles ont été greffées des molécules d'affinité spécifique, à cause du caractère polydisperse du polymère d'enrobage.

L'étape (ii) consiste à mettre en contact les particules magnétiques (p) complexées par des composés *gem*-bisphosphonates avec une quantité suffisante de biovecteur, généralement en milieu ferrofluide, sous agitation à température ambiante pendant quelques heures, la quantité de biovecteur mise en oeuvre étant fixée en fonction du taux de couplage que l'on souhaite obtenir.

A l'issue de l'étape ii), une étape de filtration peut être réalisée de manière à séparer les particules magnétiques (p), couplées avec des biovecteurs, des éventuels biovecteurs n'ayant pas réagi.

Selon une variante préférée, les particules magnétiques (p) acides mises en oeuvre à l'étape (i) sont obtenues selon un procédé comprenant les étapes consistant à :
a) préparer une solution colloïdale de ferrofluide acide ;
b) traiter la solution obtenue à l'étape a) par une solution d'acide nitrique ;
c) isoler le floculat acide obtenu à l'étape b) ;
d) réaliser une peptisation.

Les étapes a) à c) correspondent dans cette variante aux étapes i.1) et i.2) décrites précédemment.

La préparation de la solution colloïdale de ferrofluide acide réalisée à l'étape a) est bien connue et décrite dans de nombreuses références. On pourra se référer notamment au brevet FR 7918842 et aux publications C. R. Acad. Sc. Paris (7/07/1980), t. 291- série C et IEE Transactions on Magnetics, 1981, vol. MAG-17, n ° 2, p. 1247*.*

A titre indicatif, les ferrofluides "acides" et "alcalins" sont généralement obtenus par mise en contact d'une solution aqueuse contenant des sels Fe²⁺ et Fe³⁺ en des proportions appropriées avec une solution basique, en général l'ammoniaque.

La peptisation ou désagrégation du précipité gélatineux obtenu par une solution acide, généralement d'acide perchlorique ou nitrique, conduit à l'obtention d'un ferrofluide "acide", tandis que la peptisation par une solution d'hydroxyde de tétraméthylammonium (TMAOH) conduit à l'obtention de ferrofluides "alcalins".

Les ferrofluides "alcalins" ne conviennent pas à l'invention en ce que leur procédé d'obtention est tel que l'on ne peut pas contrôler facilement leur taille hydrodynamique et qu'après greffage des composés de formule (I), ils nécessitent des étapes de purification longues et laborieuses de type dialyse ou filtration sur résines. En outre, le TMAOH étant toxique, l'application biomédicale des ferrofluides alcalins résultants nécessite de procéder à des traitements supplémentaires visant à éliminer toute trace de TMAOH.

En revanche, on peut envisager, dans le cadre de la présente invention, l'utilisation de ferrofluides "acides" résultant de la conversion de ferrofluides "alcalins" selon des procédés décrits dans la littérature, notamment dans les publications suivantes : R. Massart, V. Cabuil (Journal de Chimie-Physique, 1987, 84, n°7-8, p. 967-973) ; R. Massart, CR Acad. Sc. Paris, t. 291 (7 juillet 1980), série C1.

Selon un mode de réalisation préféré du procédé selon l'invention, le ferrofluide acide mis en oeuvre à l'étape a) comprend des particules magnétiques à base de ferrite, de préférence de maghémite ou de magnétite.

Dans ce cadre, on préfère particulièrement les ferrofluides acides à base de ferrite résultant d'un traitement supplémentaire par une solution de Fe(NO₃)₃. Ce traitement supplémentaire, effectué à l'issue de l'étape a), permet de réaliser une oxydation à coeur des particules magnétiques (p) à base de ferrite, et avantageusement de réduire les phénomènes de toxicité cellulaire liés à la présence d'ions Fe²⁺. En outre, ce traitement permet d'accroître la stabilité de la solution de ferrofluide finale obtenue à l'issue de l'étape d).

Avantageusement, le traitement mis en oeuvre à l'étape b) permet de contrôler la taille hydrodynamique des particules magnétiques obtenues à l'étape a).

Ainsi, les inventeurs ont découvert de façon inattendue que la taille des particules magnétiques obtenues à l'étape a) pouvait être diminuée sous réserve d'adapter les concentrations et la durée de traitement par l'acide nitrique.

De préférence, on met en oeuvre à l'étape b) une solution d'acide nitrique de concentration comprise entre 1,5 et 4 mol/L. En particulier, dans ce cas, le traitement est appliqué pendant une durée variant généralement de 1 heure à 48 heures et de préférence pendant une durée au moins égale à 2 H et de préférence inférieure à 24 H.

A l'issue de ce traitement, le floculat obtenu est isolé à l'étape c) avantageusement par simple décantation magnétique et lavé plusieurs fois avec un solvant organique tel que l'acétone.

Enfin, le floculat isolé à l'étape c) est mis en suspension dans un volume de solvant aqueux, éventuellement en présence d'un solvant organique miscible dans l'eau permettant la peptisation, c'est-à-dire la désagrégation du floculat et l'obtention d'une solution colloïdale de ferrofluide acide.

L'invention vise également, en tant que telles, les compositions comprenant des particules magnétiques acides (p) complexées par des composés de formule (I) et couplées à des biovecteurs, susceptibles d'être obtenues par le procédé comprenant les étapes (i) et (ii).

Selon un autre aspect, l'invention a pour objet en tant que telle une composition comprenant les particules magnétiques acides (p) complexées par des composés de formule (I), susceptible d'être obtenue à l'étape (i) du procédé selon l'invention.

Sont également comprises dans l'invention les compositions comprenant un mélange de ces particules magnétiques avec les particules magnétiques couplées à des biovecteurs.

### APPLICATIONS

L'invention concerne aussi des produits de contraste pour l'imagerie médicale par résonance magnétique qui comprennent les compositions de particules magnétiques acides (p) éventuellement couplées à des biovecteurs, éventuellement associées à un véhicule et à des additifs pharmaceutiquement acceptables.

Dans le cas où la particule n'est pas couplée à un biovecteur, la fonction X du composé *gem*-bisphosphonate est choisie de telle sorte qu'elle n'induise pas de réaction toxique *in vivo.* Dans ce cadre, on préfère particulièrement que X représente -COOH ou -NH₂. En l'absence de couplage à un biovecteur, les compositions de particules magnétiques (p) complexées aux composés de formule (I) peuvent, à titre d'exemple, être utilisées comme agent de contraste pour l'Angiographie par IRM, ainsi que pour d'autres applications IRM.

Les compositions de particules magnétiques (p) complexées aux composés de formule (I) couplés à des biovecteurs sont particulièrement utiles pour une imagerie spécifique, par résonance magnétique (IRM), notamment, l'imagerie spécifique d'un organe ou d'une pathologie par résonance magnétique.

En l'absence de couplage à un biovecteur, les compositions de particules magnétiques (p) complexées aux composés de formule (I) peuvent, à titre d'exemple, être utilisées comme agent de contraste pour l'angiographie par IRM, ainsi que pour d'autres applications IRM non spécifiques.

Les relaxivités r₁ et r₂ d'un produit de contraste magnétique donnent la mesure de son efficacité magnétique et permettent d'apprécier son influence sur le signal enregistré.

Dans ce cadre, les inventeurs ont montré que les compositions de particules magnétiques (p) complexées par les composés gem-bisphosphonates présentaient des relaxivités r₁ et r₂ très avantageuses, permettant d'obtenir une forte augmentation des vitesses de relaxation des protons (R₁ = 1/T₁ et R₂ = 1/T2). Cet effet sur les vitesses de relaxation permet alors d'obtenir un très bon contraste en IRM, dans les zones ciblées.

Dans le cas où les particules magnétiques sont couplées à des biovecteurs, notamment à des ligands spécifiques d'un récepteur biologique cible donné, les compositions de l'invention pourront être utilisées comme agents de contraste pour l'Imagerie spécifique par IRM, en particulier pour la caractérisation et/ou le suivi thérapeutique dans de nombreuses pathologies : à titre indicatif, nous pouvons citer les maladies cardiovasculaires (imagerie de la plaque d'athérome), les cancers (tumeurs, métastases), les maladies inflammatoires et dégénératives (sclérose en plaque, polyarthrite rhumatoïde, maladie d'Alzheimer).

Ainsi, selon un autre aspect, l'invention a pour objet une méthode de diagnostic ou de suivi thérapeutique dans laquelle on administre à un patient une composition comprenant des particules magnétiques acides (p) éventuellement couplées à au moins un biovecteur, ce qui permet de visualiser un organe ou une pathologie chez le patient par résonance magnétique.

A titre d'exemple, les compositions de particules finales pour lesquelles le biovecteur utilisé est un dérivé de l'acide folique, un inhibiteur de métalloprotéases ou un anticorps, par exemple de type anti-CEA ou antimucines, pourront être particulièrement utiles comme agent de contraste IRM en oncologie pour la détection, la caractérisation des tumeurs et le bilan d'extension de cancers (métastases).

De la même façon, les compositions de particules finales sur lesquelles sont couplés des biovecteurs tels que les dérivés de phosphatidylsérine permettent de cibler les maladies inflammatoires et dégénératives, les plaques d'athérome ou le stress.

De la même façon, les compositions de particules finales sur lesquelles sont couplés des biovecteurs tels que des peptides contenant la séquence RGD (Arginine-Glycine-Acide-Aspartique) permettent de cibler des intégrines exprimées au cours de l'angiogénèse et dans les phénomènes de thrombose et inflammatoires. Elles pourront donc être utilisées pour suivre, par IRM, l'efficacité de traitement anti-angiogénique en cancérologie ou anti-thrombotique en cardiologie.

De manière générale, l'utilisation de biovecteurs tels que les anticorps monoclonaux ou leurs fragments (Fab, Fab'₂, sFv) permet d'atteindre une très haute spécificité *in vivo* pour cibler des zones pathologiques où sont surexprimés les récepteurs correspondants aux anticorps utilisés.

De manière avantageuse, il a été montré que les relaxivités des particules (p) complexées par des composés de formule (I) et couplées avec un biovecteur ne sont pas altérées en comparaison aux relaxivités de l'intermédiaire sans biovecteur. En outre, il a été montré que l'efficacité de ces compositions ne dépend pas de la nature du biovecteur couplé sur les particules (p) complexées par des composés de formule (I).

Les compositions de particules magnétiques couplées ou non couplées à des biovecteurs sont également utiles pour la médecine nucléaire, en utilisant un isotope du fer radioactif lors de la synthèse.

Selon un autre aspect, les compositions de particules magnétiques acides (p) couplées à des biovecteurs selon l'invention peuvent être utilisées pour délivrer un médicament par ciblage magnétique. Dans ce cadre, les particules magnétiques (p) sont couplées à des biovecteurs possédant des propriétés pharmacologiques et/ou cytotoxiques pouvant être éventuellement libérées sous l'action d'un champ radiofréquence dans la zone ciblée.

L'invention a ainsi pour objet une méthode de traitement thérapeutique dans laquelle on administre à un patient nécessitant un tel traitement une composition comprenant des particules magnétiques acides (p) couplées à au moins un biovecteur, en association avec un véhicule pharmaceutiquement acceptable.

Les compositions de particules magnétiques acides (p) couplées à des biovecteurs selon l'invention sont également utiles pour le traitement par hyperthermie de territoires pathologiques. Dans ce cadre, on choisit généralement un biovecteur ayant une affinité particulière pour un récepteur présent dans la zone ciblée et on applique un champ de radiofréquence, de préférence un champ de radiofréquence alternatif extérieur, en regard de ladite zone ciblée.

Les compositions de particules magnétiques (p) couplées à des biovecteurs selon l'invention sont également utiles pour le marquage cellulaire et, en particulier, le marquage des cellules souches, lequel peut être réalisé *in vitro* ou *ex vivo.*

Ainsi, selon un autre aspect, l'invention a pour objet une méthode de détection et/ou de séparation *in vitro* des cellules comprenant :
i) la mise en contact de cellules avec une composition comprenant des particules magnétiques acides (p) couplées à au moins un biovecteur capable de marquer les cellules ; et
ii) la détection et/ou la séparation des cellules marquées obtenues.

De préférence, le biovecteur est un ligand spécifique d'un récepteur biologique exprimé par les cellules à marquer.

Dans ce cadre, un biovecteur susceptible de se lier spécifiquement à un récepteur des cellules souches est couplé sur les particules magnétiques (p). Les cellules souches ainsi marquées administrées à un patient, permettent alors de suivre, sous l'application d'un champ magnétique, le mouvement, la localisation et la survie des cellules exogènes par visualisation IRM. Cette application est particulièrement utile pour détecter des pathologies liées à un dysfonctionnement cellulaire.

L'invention a ainsi pour objet une méthode de diagnostic de pathologies liées à un dysfonctionnement cellulaire, comprenant :
i) le marquage *ex vivo* de cellules souches d'un patient selon la méthode précitée ;
ii) l'administration des cellules souches marquées audit patient ;
ce qui permet, sous l'application d'un champ magnétique, de suivre le mouvement, la localisation et la survie des cellules exogènes par visualisation IRM et ainsi de détecter les pathologies liées à un dysfonctionnement cellulaire.

Enfin, les compositions de particules magnétiques (p) couplées à des biovecteurs sont particulièrement utiles pour le tri magnétique cellulaire, réalisé in vitro. Dans ce cas, le biovecteur est généralement un ligand choisi de manière à différencier et/ou séparer les composés portant un récepteur dudit biovecteur de ceux ne portant pas ledit récepteur. A titre d'exemple, le biovecteur peut représenter une annexine spécifique, ligand capable de se lier à la phosphatidyl sérine (PS) ou la phosphatidyl éthanolamine (PE), en présence de Ca²⁺. Dans ce cas, l'utilisation selon l'invention permet de différencier et/ou de séparer les cellules présentant un état anormal, i. e. capables de se lier aux particules magnétiques (p) couplées à des biovecteurs annexines, des cellules saines incapables de lier ces particules.

Les termes "pharmaceutiquement acceptable" font référence à une composition ou une entité moléculaire ne produisant pas de réaction allergique, d'effets secondaires ou indésirables lorsqu'elle est administrée à un animal ou à un être humain de façon appropriée.

L'expression "véhicules pharmaceutiquement acceptables" peut désigner au sens de la présente description un solvant, un milieu de dispersion, des agents antibactériens et antifongiques. L'utilisation de tels milieux et agents relève des compétences de l'homme du métier.

A moins que les milieux ou agents conventionnels ne soient incompatibles avec les particules magnétiques (p) complexées par des composés de formule (I), et couplées à des biovecteurs, leur utilisation dans les compositions de diagnostique peut être envisagée. Des ingrédients actifs supplémentaires peuvent également être incorporés dans les compositions selon l'invention.

Il est entendu que, dans les méthodes de traitement de l'invention, les compositions sont administrées en une quantité thérapeutique efficace, qui peut être ajustée par l'homme du métier selon la pathologie visée, l'état du patient, son poids, son âge, etc.

Les compositions de l'invention sont préférablement administrées par voie parentérale, par voie orale, les autres voies d'administration n'étant cependant pas exclues, telles que par exemple l'administration par voie rectale, l'administration sous forme d'une injection intraveineuse étant particulièrement préférée.

Lorsque l'administration par voie orale est envisagée, les compositions de l'invention se trouvent sous la forme de gélules, comprimés effervescents, comprimés nus ou enrobés, sachets, dragées, ampoules ou solutés buvables, microgranules ou formes à libération prolongée ou contrôlée.

Lorsque l'administration par voie parentérale est envisagée, les compositions de l'invention se trouvent sous la forme de lyophilisats prêts à être reconstitués ou de solutés et suspensions injectables conditionnées en ampoules ou flacons ou seringues pré-remplies, pour perfusion veineuse lente ou injection intra-veineuse en bolus.

Les formes pour l'administration orale sont préparées par mélange des particules magnétiques (p) éventuellement couplées à des biovecteurs, notamment à une substance active, avec différents types d'excipients ou de véhicules tels que des charges, des agents de délitement, des liants, des colorants, des agents correcteurs de goût et analogues, et puis, mise en forme du mélange en une gélule à libération contrôlée notamment.

La solubilité aqueuse et la faible osmolalité des particules de l'invention permettent de préparer des solutions aqueuses isotoniques, de forte concentration et de viscosité acceptable pour l'injection.

Les formes pour l'administration parentérale sont obtenues de façon conventionnelle par mélange des particules magnétiques (p) éventuellement couplées à des biovecteurs avec des tampons, des agents stabilisants, des conservateurs, des agents solubilisants, des agents isotoniques et des agents de mise en suspension. Conformément aux techniques connues, ces mélanges sont ensuite stérilisés puis conditionnés sous la forme d'injections intraveineuses ou de lyophilisats prêts à être reconstitués dans un véhicule stérile pharmaceutiquement acceptable.

Les solutions peuvent être préparées extemporanément à partir de la poudre lyophilisée contenant les particules magnétiques (p) éventuellement couplées aux biovecteurs et éventuellement des additifs et un solvant stérile ou, du fait de la grande stabilité des particules magnétiques couplées aux biovecteurs en solution, les solutions peuvent être fournies au radiologue en flacons, ampoules seringues ou poches.

Pour la préparation de suppositoires, les particules magnétiques selon l'invention sont mélangées de façon connue en soi à un constituant de base approprié tel que du polyéthylèneglycol ou des glycérides hémi-synthétiques.

Les doses unitaires seront fonction de la composition des particules magnétiques (p), couplées à des biovecteurs, de la voie d'administration, du type de diagnostic à établir, ainsi que du patient. Les doses unitaires seront en général de 0,01 µmole à 100 µmole de particules magnétiques pour un homme de taille moyenne (75 kg).

Comme démontré dans les exemples, les inventeurs ont réussi à obtenir des composés à la fois stables et spécifiques utilisant comme revêtement des composés *gem*-bisphosphonates. Il est précisé que, de manière plus large, le demandeur étudie des revêtements capables de se lier d'une part à des particules magnétiques et d'autre part à au moins un biovecteur. Différents revêtements sont étudiés de formule Y1-Y2-Y3, Y1 étant au moins un groupe capable de se coupler à des nanoparticules, Y3 étant au moins un groupe capable d'interagir avec au moins un biovecteur, Y2 présent ou absent étant un linker entre Y1 et Y3. Parmi les Y1 on étudie notamment les phosphonates, phosphates, bisphosphonates et analogues autres que gem-bisphosphonates, hydroxamates ou gem-hydroxamates.

Dans ce qui suit, des exemples de préparation des compositions selon l'invention sont décrits à titre d'illustration.

Dans les exemples qui suivent, on précise les généralités suivantes.

Dans ce qui suit, les abréviations M, M/L, M théorique, N et M/z, ES⁺ , ES, kD et CCM ont les significations suivantes :
M ou M/L : concentration molaire (mole/litre).
M théorique : masse moléculaire théorique.
N : normalité.
M/z : masse sur charge déterminée par spectrométrie de masse.
ES⁺ : électrospray mode positif.
ES⁻: électrospray mode négatif.
TFA : acide trifluoroacétique
kD : unité de masse moléculaire (kiloDalton)
CCM : Chromatographie sur Couche Mince
Z ave : diamètre hydrodynamique mesuré par PCS
Poly σ : polydispersité mesurée par PCS.

La nomenclature chimique qui suit est issue du logiciel ACD/NAME (société Advanced Chemistry Development Inc, Toronto, Canada), selon les règles IUPAC.

### Dosage du fer total :

Le fer est dosé par spectroscopie d'absorption atomique (Spectrophotomètre VARIAN AA10) après minéralisation par HCl concentré et dilution par rapport à une gamme étalon d'ions ferrique (0, 5, 10, 15 et 20 ppm).

### Taille des particules :

### - Diamètre hydrodynamique de la particule greffée (Z ave) = Taille PCS:

Déterminé par PCS (appareil Malvern 4700, laser 488 nm à 90°) sur un échantillon dilué à ∼ 1 millimolaire avec de l'eau PPI filtrée sur 0.22 µm.

PCS = Photon Correlation Spectroscopy = Technique par Diffusion de Lumière Dynamique - Référence : R. Pecora dans J. of Nano. Res (2000), 2, p. 123-131.

### - Diamètre de la particule magnétique (p) (avant greffage)

Déterminé par déconvolution des courbes d'aimantation (mesures effectuées sur un magnétomètre SQUID) à différentes températures. [Référence: R.W. Chantrell dans IEEE Transactions on Magnetics (1978), 14(5), p. 975-977.

### Détermination du taux de greffage par microanalyse :

Composé A : exprimé en mole pour cent mole de fer total.

Autres composés : exprimé en mole pour cent mole de fer et en mole pour cent mole de composé A.

### Analyses structurales :

Par spectroscopie de masse (appareil MICROMASS VG Quattro II) avec une source Etectrospray.

### Mesures de relaxivité :

Les temps de relaxation T1 et T2 ont été déterminés par des procédures standards sur un appareil Minispec 120 (Bruker) à 20 Mhz (0,47T) et 37°C. Le temps de relaxation longitudinal T₁ est mesuré en utilisant une séquence d'Inversion Récupération et le temps de relaxation transverse T2 est mesuré par une technique CPMG.

Les vitesses de relaxation R1 (=1/T1) et R2 (=1/T2) ont été calculées pour différentes concentrations en métal total (variant de 0,1.10⁻³ à 1.10⁻³ mole/L) en solution aqueuse à 37°C. La corrélation entre R1 ou R2 en fonction de la concentration est linéaire, et la pente représente la relaxivité r1 (R1/C) ou r2 (R2/C) exprimées en (1 / seconde) x (1/mmol/L) soit (mM⁻¹.s⁻¹).

### Exemple de composés de formule (I)

### Exemple 1 : Préparation du composé A :

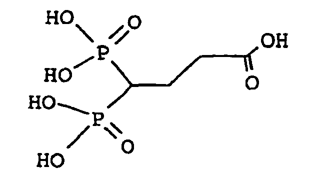

### 1) Diéthyl-1-[éthoxyphosphoryl]vinylphosphonate

13 g (0,433 mole) de paraformaldéhyde et 10 ml (0,097 mole) de diéthylamine sont solubilisés à chaud dans 250 ml de méthanol. 24 g (8,67 10⁻² mole) de diéthyl[éthoxy(propyl)phosphoryl]méthylphosphonate sont ensuite additionnés. L'ensemble est porté à reflux pendant 24 heures. Le milieu réactionnel est concentré sous vide. Le concentrat est repris 2 fois par 250 ml de toluène et est ensuite concentré sous vide.

L'huile obtenue est solubilisée dans 125 ml de toluène. 0,14 g d'acide para toluène sulfonique sont ajoutés. Le mélange est porté à reflux 24 heures avec un Dean-Stark puis est concentré à sec sous vide.

Le produit est extrait avec 500 ml de CH₂Cl₂ puis est lavé 2 fois avec 250 ml d'eau. La phase organique est séchée sur MgSO₄ et concentrée sous vide.

Le produit brut est purifié sur 625 g de gel de silice Merck Geduran® (40-63 µm). Elution : CH₂Cl₂/Acétone - 50/50 (CCM - SiO₂ : Rf = 0,45)

18,4 g sont isolés avec un rendement de 71 %.
Spectre de masse : M/z = 301,4 (ES+) M théorique = 300,2
Spectre C¹³: (s) 148,8 ppm, (t) 134,8 -131,.5 - 128,2 ppm, (s) 62,2 ppm, (s) 16,7 ppm
Spectre H¹: (t) 6,9 - 6,8 - 6,6 ppm, (massif) 3,9 ppm, (t) 1,15 ppm.

### 2) Diéthyl 2-[2.2-bis(diéthylphosphoryl)éthyl]malonate

1,6 g (0,01 mole) de diéthyle malonate, 0,07 g (0,001 mole) d'éthylate de sodium et 3 g (0,01 mole) de diéthyl[éthoxy(propyl)phosphoryl]vinylphosphonate sont agités 15 mn dans 15 ml d'éthanol. [CCM : SiO₂ ; éluant CH₂Cl₂ /Acétone 50/50 - Rf = 0,6].

5 ml d'une solution saturé de NH₄Cl sont ajoutés à la solution éthanolique. L'ensemble est concentré sous vide. Le résidu est extrait avec 30 ml d'acétate d'éthyle et lavé deux fois avec 5 ml d'eau. La phase organique est séchée sur MgSO₄ puis est évaporée à sec.

L'huile obtenue est purifiée sur 200 g de silice Merck Geduran^{®}
(40-63 µm). Elution CH₂Cl₂ /Acétone 50/50 Rf = 0,6
3,8 g sont isolés avec un rendement de 82 %.
Spectre de masse : M/z 460,9 (ES+), M théorique = 460.

### 3) Acide 4,4-diphosphonobutanoique

7 g (15.7 10⁻² mole) de diéthyl2-[2.2-bis (diéthylphosphoryl) éthyl] malonate sont portés à reflux pendant 8 heures dans 350 ml d'HCl [5N].

L'huile brune obtenue est purifiée sur 60 g de gel de silice 60 silanisée (0,063-0,200 mm) avec une élution eau [suivi HPLC].

3,6 g sont isolés avec un rendement de 92 %.
Spectre de masse : M/z 249 (ES+), M théorique = 248
HPLC : Colonne : Hypercarb^{®} 250 X 4 mm Détection : 202 nm
Eluant Isocratique 99/1 : H₂SO₄ 0,034 N / CH₃CN - Tr = 8 min

### Exemples 2 à 7 : Préparation de biovecteurs

### Exemple 2 : Préparation du composé B :

### 1) Éthyl-2,5-dioxo-2.5-dihydro-1H-pyrrole-1-carboxylate

9,7 g (0,1 mole) de maléimide sont solubilisés dans 50 ml d'acétate d'éthyle et 1,1 ml de N-méthyl morpholine à 5°C.

1,1 ml d'éthyle chloroformate est additionné goutte à goutte. L'ensemble est agité ½ heure. L'insoluble est éliminé par filtration. Le filtrat est lavé avec 50 ml d'eau. La phase organique est séchée sur MgSO₄ puis est concentrée sous vide. L'huile obtenue est purifiée sur gel de silice Merck Geduran® (40-63µm). Elution : CH₂Cl₂ / ACOEt 66/34.

16,9 g sont isolés avec un rendement de 50 %.
Spectre de masse : M/z = 170,1 (ES+) M théorique = 169

### 2) ter-butyl 3-(2.5-dioxo-2,5dihydro1H-pyrrol-1yl)propylcarbamate

0,515 g (2,96 10⁻³ mole) du composé B 1) est solubilisé dans 20 ml de NaHCO₃ saturée. La solution est filtrée sur un filtre de porosité 0,45 µm puis est refroidie à 0°C.

0,5 g (2,96 10⁻³ mole) d'éthyl carbonyl maléimide en solution dans 25 ml de tétrahydrofuranne sont additionnés goutte à goutte. L'ensemble est agité pendant 15 minutes.

25 ml de tétrahydrofuranne sont additionnés et le milieu réactionnel est agité 45 mn. Le pH est ajusté à 6 avec qsp d'HCl [1N]. Le produit est extrait avec 2 x 200 ml d'acétate d'éthyle. La phase organique est séchée sur MgSO₄ puis est concentrée sous vide.

737 mg sont isolés avec un rendement de 98 %.

### 3) 1-(3-aminopropyl)1H-pyrrol-2,5-dione

0,7 g (2,75 10⁻³ mole) du composé B 2) est mis en agitation dans 25 ml d'HCl [2 N] pendant 24 heures.

La solution est concentrée sous vide. 500 mg sont obtenus avec un rendement de 88 %
Spectre de masse : M/z = 155,1 (ES+) M théorique = 154,1

### Exemple 3 : Préparation du composé C :

### 1-déoxy-1-[(2,3-dihydropropyl)amino]hexitol

173 g (1,9 mole) de 3-amino-1,2-propanediol sont solubilisés dans 1 litre de méthanol. 360 g (2 mole) de glucose sont ajoutés. L'ensemble est agité 24 heures à température ambiante. Cette solution est réduite sous 20 bars d'hydrogène à 60°C avec 50 g de palladium sur Charbon et 450 ml d'eau pendant 6 heures.

Le milieu réactionnel est filtré sur clarcel à 35 °C. Les jus de filtration sont concentrés jusqu'à un volume final de 850 ml. Le concentrat est versé sur 3 litres d'alcool isopropylique maintenus à 35°C par un bain d'eau chaude. Le précipité est filtré et séché sous vide. 272 g sont isolés avec un rendement de 53%.
Spectre de masse : M/z = 256,4 (ES+) M théorique = 253,3

### Exemple 4 : Préparation du composé D : N-[(2,3-dihydroxypropyl)(2,3,4,5,6-pentahydroxyhexyl)]2,4,6-tribromo 5-(glycylamino)isophtalamide

255 g (1,9 mole) de composé C sont dissous à 80°C dans 2,5 litres de 1-méthyl-2-pyrolidinone pendant 30 minutes. Le milieu réactionnel est refroidi à 40°C avant l'addition de 67,1 g (0 ,635 mole) de Na₂CO₃ séché à 170°C et de 407,8 g (0,635 mole) du chlorure de l'acide 5-phtalamido(acétamido) 2,4,6 triaminoisophtalique en maintenant la température inférieure à 45°C.

Le milieu est maintenu 2 heures à 40°C puis est refroidi à 20°C avant filtration des minéraux sur Clarcel.

610 ml d'eau sont ajoutés au filtrat et l'ensemble est chauffé à 70°C avant l'addition de 44,8 ml d'hydrate d'hydrazine (0,889 mole). Le milieu réactionnel est chauffé 2 heures à 90°C.

Le pH est ajusté à 1 avec QSP d'HCl [12 N]. Le précipité formé est éliminé par filtration. Les jus de filtration sont versés dans 30 litres d'éthanol.

Le produit obtenu est dissous dans 3,6 litres d'eau, le pH est ajusté à 6,5 avec QSP de soude [2N] et la solution est diafiltrée. Suivi HPLC de la purification. Pureté HPLC = 98.2 %.

Conditions HPLC :
Colonne : Symetry^{®} C18 250x 4 mm (5 µm). Détection : 254 nm
Eluant isocratique : 99/1 : H₂SO₄ 0,034 N/CH₃CN
Dosages de Brome = 23,6% (soit une pureté de 97,2%)

### Exemple 5 : Préparation du composé E : N-N'-[bis(2,3,4,5,6-pentahydroxyhexyl)]2,4,6-tribromo 5-(glycylamino)isophtalamide

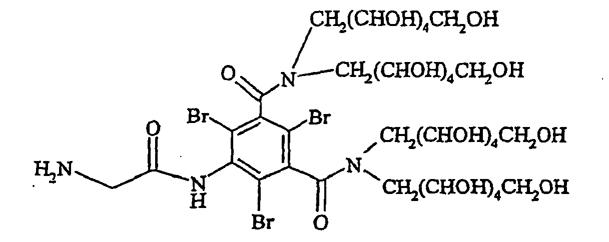

Le composé E peut être préparé selon le mode opératoire décrit dans le Brevet : EP 0 922 700 A1.

### Exemple 6 : Préparation du composé F : Acide (18S)-1-amino-18{[(2-amino-4-oxo-3,4-dihydropteridin-6-yl)méthyl]amino}benzoyl)amino]-15-oxo-4-trioxa-14 azanonadécan-19-oique

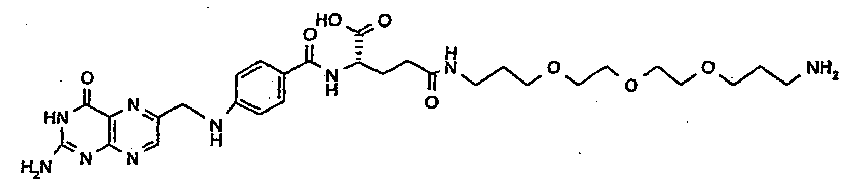

9,74 g (0,0214 mole) de l'acide (2S)-2-[(4-{[(2-amino-4-oxo-3,4-dihydroptéridin-6-yl)méthyl]amino}benzoyl)amino]-5-méthoxy-5-oxopentanoique (pouvant être préparé selon le mode opératoire décrit dans J. Am. Chem. Soc., 1997, 119, 10004-10013) sont solubilisés dans 60 ml de DMSO à température ambiante. 235 ml (1,07 mole) de 4,7,10-trioxa-1,13-tridécanediamine sont ajoutés. L'ensemble est agité 48 h à température ambiante.

Le milieu réactionnel est versé dans un mélange de 1,5 L de CH₃CN et de 1,5 L d'éther éthylique. Le précipité obtenu est filtré et séché sous vide.

Le produit brut est solubilisé dans 100ml de pyridine [0,1 N] et est purifié sur 1 Kg de gel de silice 60 silanisée (0,063-0,200 mm) avec une élution pyridine [0,1 N] - méthanol 9/1 [suivi HPLC]. 2,7 g sont isolés avec un rendement de 26 % et une pureté HPLC de 97,3 %.
Conditions HPLC :
Colonne : Symetry^{®} C18 250x 4 mm (5 µm). Détection : 254 nm
Eluant isocratique : 99/1 : H₂SO₄ 0,034 N/CH₃CN

### Exemple 7 : Préparation du composé G :

### L-propyl-L-Leucyl-N¹-hydroxy glycinamide

1 g (2,3 10⁻³ mole) de 1-[(benzyloxy)carbonyl]-L-propyl-L-leucyl-N¹-hydroxy glycinamide est dissous dans 100 ml de méthanol.

100 mg de Pd/C 50% hydraté sont ajoutés à la solution. L'ensemble est agité 6 heures à température ambiante sous 50 PSI d'hydrogène.

Le catalyseur est éliminé par filtration et le filtrat est concentré sous vide. Le produit est stocké à 4°C.
Rendement quantitatif
Spectre de masse : M/z = 371,2 (ES-) M théorique = 372,4

### Exemples 8 et 9: Exemples de préparation de solutions colloïdales de particules magnétiques acides (p)

### Exemple 8 :

Une solution de 36 g (0,181 mole) de FeCl₂, 4H₂O, 20 ml de HCl à 37% dans 150 ml de H₂O est introduite dans un mélange constitué de 3 litres d'eau et 143 ml (0,302 mole) de FeCl₃ à 27%. 250 ml de NH₄OH à 25 % sont introduits rapidement sous forte agitation. L'ensemble est agité 30 mn. Les jus sont éliminés par décantation magnétique. Le ferrofluide est lavé 3 fois de suite avec 2 litres d'eau.

Le ferrofluide nitrique est mis en agitation 15 mn avec 200 ml de HNO₃ [2M], le surnageant est éliminé par décantation magnétique.

Le ferrofluide nitrique est porté à reflux avec 600 ml d'eau et 200 ml de Fe(NO₃)₃ [1M] pendant 30 mm. Le surnageant est éliminé par décantation magnétique.

Le ferrofluide nitrique est mis en agitation 15 mn avec 200 ml de HNO₃ [2M], le surnageant étant éliminé par décantation magnétique.

Le ferrofluide nitrique est lavé 3 fois avec 3 litres d'acétone, puis est repris par 400 ml d'eau. La solution est évaporée sous vide jusqu'à un volume final de 250 ml.

| Concentration M/L | Z ave nm | Poly σ | Diamètre SQUID | Ms emu/cm³ |
|---|---|---|---|---|
| 4,85 | 40 nm | 0,22 | 8,5 nm | 275 |

| | | | | |
|---|---|---|---|---|
| Ms (emu/cm³) = aimantation à saturation Z ave = Taille hydrodynamique par PCS en mode unimodal Poly σ : polydispersité du pic en PCS SQUID = diamètre de la particule (p) non greffée estimé par déconvolution des courbes d'aimantation mesurées sur un magnétomètre SQUID | | | | |

### Exemple 9 :

108 g (0,543 mole) de FeCl₂, 4 H₂O dans 450 ml de H₂O est introduit dans une solution de 4 litres d'eau et 429ml (0,906 mole) de FeCl₃ à 27%. 750 ml de NH₄OH à 25 % sont introduits rapidement sous agitation (1200 tr/mn). L'ensemble est agité 30 mn. Les jus sont éliminés par décantation magnétique. Le ferrofluide est lavé 2 fois de suite avec 3 litres d'eau.

Le ferrofluide nitrique est mis en agitation ¼ h avec 3 litres de HNO₃ [2M], le surnageant est éliminé par décantation magnétique.

Le ferrofluide nitrique est porté à reflux avec 1300 ml d'eau et 700 ml de Fe(NO₃)₃ [1M] pendant 30 mm (600 tr/mn). Le surnageant est éliminé par décantation magnétique.

Le ferrofluide nitrique est mis en agitation 15 mn avec 3 litres de HNO₃ [2M], le surnageant étant éliminé par décantation magnétique.

Le ferrofluide nitrique est lavé 3 fois avec 3 litres d'acétone, puis est repris par 600 ml d'eau. La solution est évaporée sous vide jusqu'à un volume final de 250 ml.

### ❖ A ce stade, on obtient comme caractéristiques :

| Rendement % | Concentration M/L | Z ave (nm) | Poly σ |
|---|---|---|---|
| 81,8 | 4,45 | 31,3 | 0,21 |

| | | | |
|---|---|---|---|
| Z ave = Taille hydrodynamique par PCS en mode unimodal. | | | |

### ❖ Traitement :

200 ml de la solution précédente sont mis en agitation dans 2,4 litres de HNO₃ pendant 4 heures. Le surnageant est éliminé par décantation magnétique. Le ferrofluide nitrique est lavé 2 fois avec 3 litres d'acétone, puis est repris par 400 ml d'eau. La solution est évaporée sous vide jusqu'à un volume final de 250 ml.

| Rendement % | Concentration M/L | Z ave (nm) | Poly σ |
|---|---|---|---|
| 77 | 2,742 | 23, 3 | 0,20 |

### Exemples 10 à 12: Exemples de complexation des particules magnétiques (p) par des composés de formule (I)

### Exemple 10 :

50 ml de l'exemple 8 à 4,85 M/L sont dilués dans 3 litres d'eau. Une solution de 1,3 g (5,24 10⁻³ mole) du composé A de l'exemple 1 dans 100 ml d'eau est introduite goutte à goutte. L'agitation est maintenue pendant 30 minutes. Le floculat est isolé par décantation magnétique puis est lavé 3 fois par 3 litres d'eau.

Il est remis en solution avec 700 ml d'eau à pH 7,2 avec QSP de NaOH [1N]. La solution finale est filtrée sur membrane 0,22 µm.

| | |
|---|---|
| Dosage de Fer = 0,252 M/L | Taille PCS = 67,9 nm |
| Fe = 61,7 % Masse/Masse | |
| P = 1,21 % Masse/Masse | |
| C = 1,04 % Masse/Masse | |
| Taux de greffage [composé A/Fe] = 1,86 % mole/mole | |

### Exemple 11 :

50 ml de l'exemple 8 à 4,73 M/L sont dilués dans 3 litres d'eau. Une solution de 1,3 g (5,24 10⁻³ mole) du composé A de l'exemple 1 dans 80 ml d'eau est introduite goutte à goutte. L'agitation est maintenue 30 mn. Le floculat est isolé par décantation magnétique puis est lavé 3 fois par 3 litres d'eau.

Il est remis en solution avec 700 ml d'eau à pH 11 avec QSP de NaOH [1N] puis stabilisé à pH 7,2 avec QSP de HCl [1 N]. La solution finale est filtrée sur membrane 0,22 µm.

| | | |
|---|---|---|
| Dosage de Fer = 0,279 M/L | Taille PCS = 40,3 nm | Poly σ = 0,19 |
| Fe = 63,9 % Masse/Masse | | |
| P = 1,38 % Masse/Masse | | |
| C = 1,07 % Masse/Masse | | |
| Taux de greffage [composé A / Fe] = 1,95 % mole/mole | | |

### Exemple 12 :

100 ml de l'exemple 9 à 2,742 M/L (taille PCS = 21,3 nm) sont dilués dans 3 litres d'eau.

Une solution de 1,5 g (6,03.10⁻³ mole) du composé A de l'exemple 1 dans 100 ml d'eau est introduite goutte à goutte. L'agitation est maintenue pendant 30 minutes. Le floculat est isolé par décantation magnétique puis est lavé 3 fois par 3 litres d'eau.

Il est remis en solution avec 700 ml d'eau à pH 11 avec QSP de NaOH [1 N] puis stabilisé à pH 7,2 avec QSP de HCl [1 N]. La solution finale est filtrée sur membrane 0,22 µm.

| | |
|---|---|
| Dosage de Fer = 0,285 M/L | Taille PCS = 25,6 nm |
| Fe = 62,9 % Masse/Masse | |
| P = 1,32 % Masse/Masse | |
| C = 1,22 % Masse/Masse | |
| Taux de greffage [composé A / Fe] = 1,90 % mole/mole | |

### Relaxivités :

### 20MHz (0,47 T) - 37°C - en solution aqueuse

| r₁ (mM⁻¹.s⁻¹) | r₂ (mM⁻¹.s⁻¹) |
|---|---|
| 35±2 | 103±5 |

### Exemples 13 à 18: Couplage des particules magnétiques acides (p) complexées par des composés de formule (I) avec des biovecteurs

### Exemple 13 :

Dans 50 ml de l'exemple 10 à 0,252 M/L, 36 mg (1,88 10⁻⁴ mole) du composé B sont dissous. Le pH est ajusté à 5,8 avec QSP d'HCl [1 N].

Une goutte de triéthylamine et 72 mg de chlorhydrate de 1-(3-diméthylaminopropyl)-3-éthylcarbodiimide sont ajoutés au milieu réactionnel et l'ensemble est agité 18 heures à température ambiante.

La solution est ultrafiltrée sur une cellule à agitation PALL^{®} ayant un seuil de coupure de 30 kD. 600 ml de filtrat sont éliminés pour obtenir une solution finale de 35 ml.

| | |
|---|---|
| [Fe] = 0,212 M/L | Taille PCS = 69,2nm |
| Fe = 62,8 % Masse/Masse | |
| P =1,21 % Masse/Masse | |
| C = 2,23 % Masse/Masse | |
| N = 0,48 % Masse/Masse | |
| Taux de greffage [composé A / Fe] = 1,74 % mole/mole | |
| Taux de greffage [composé B / Fe] = 1,37% mole/mole | |
| Taux de greffage [composé B / composé A] = 78,6 % | |

### Exemple 14 :

0,193 g (7,6 10⁻⁴ mole) du composé C de l'exemple 3 est dissous dans 13,55 ml de l'exemple 11 à 0,279 M/L. Le pH est ajusté à 6,2.

171 mg de chlorhydrate de 1-(3-diméthylaminopropyl)-3-éthylcarbodiimide sont ajoutés. L'ensemble est agité à température ambiante pendant 24 heures. La solution est ultrafiltrée sur une cellule à agitation PALL^{®} ayant un seuil de coupure de 30 kD. 500 ml de filtrat sont éliminés. Le rétentat est ajusté à 30 ml

| | |
|---|---|
| [Fe] = 0,155 M/L | Taille PCS = 43,7 nm |
| Fe = 58,4 % Masse/Masse | |
| P = 1,17 % Masse/Masse | |
| C = 2,80 % Masse/Masse | |
| Taux de greffage [composé A / Fe] = 1,80 % mole/mole | |
| Taux de greffage [composé C / Fe] = 1,67% en mole/mole | |
| Taux de greffage [composé C / composé A] = 93 % | |

### Exemple 15 :

0,358 g (3,78 10⁻⁴ mole) du composé D de l'exemple 4 sont dissous dans 13,55 ml de l'exemple 11 à 0,279 M/L.

86 mg de chlorhydrate de 1-(3-diméthylaminopropyl)-3-éthylcarbodiimide sont ajoutés. L'ensemble est agité à température ambiante pendant 24 heures. La solution est ultrafiltrée sur une cellule PALL^{®} à agitation ayant un seuil de coupure de 30 kD. 500 ml de filtrat sont éliminés. Le rétentat est ajusté à 30 ml

| | |
|---|---|
| [Fe] = 0,134 M/L | Taille PCS = 41, 3 nm |
| Fe = 51,2 % Masse/Masse | |
| P = 1,16 % Masse/Masse | |
| C = 5,79 % Masse/Masse | |
| Br = 3,07 % Masse/Masse | |
| Taux de greffage [composé A / Fe] = 2 % mole/mole | |
| Taux de greffage [composé DI Fe] = 1,4% mole/mole | |
| Taux de greffage [composé D/ composé A] = 68,4 % | |

### Exemple 16 :

0,853 g (7,6 10⁻⁴ mole) du composé E de l'exemple 5 sont dissous dans 13,55 ml de l'exemple 11 à 0,279 M/L. Le pH est ajusté à 6,2.

171 mg de chlorhydrate de 1-(3-diméthylaminopropyl)-3-éthylcarbodiimide sont ajoutés. L'ensemble est agité à température ambiante pendant 24 heures. La solution est ultrafiltrée sur une cellule à agitation PALL^{®} ayant un seuil de coupure de 30 kD. 500 ml de filtrat sont éliminés. Le rétentat est ajusté à 30 ml.

| | | |
|---|---|---|
| [Fe] = 0,132 M/L | Taille PCS = 41,6 nm | Poly σ = 0,22 |
| Fe = 52,3 % Masse/Masse | | |
| P = 0,77 % Masse/Masse | | |
| C = 5,86 % Masse/Masse | | |
| Br = 2,65 % Masse/Masse | | |
| Taux de greffage [composé A / Fe] = 1,33 % mole/mole | | |
| Taux de greffage [composé E / Fe] = 1,18% mole/mole | | |
| Taux de greffage [composé E / composé A] = 89 % | | |

### Exemple 17 :

90 ml d'une solution à 1,35 g/L du composé F de l'exemple 6 sont introduits dans 66,3 ml de l'exemple 12 à 0,285 M/L. 121,7 mg de chlorhydrate de 1-(3-diméthylamino-propyl)-3-éthylcarbodiimide sont ajoutés. Le pH est ajusté à 6,8 et l'ensemble est agité 18 heures à température ambiante.

Le milieu réactionnel est directement ultrafiltré sur cellule à agitation PALL^{®} ayant un seuil de coupure de 30 kD. Le rétentat est ajusté à 100 ml puis est filtré sur 0,22 µm.

| | |
|---|---|
| [Fe]: 0,213 M/L | Taille PCS = 29 nm |
| Fe = 59,6 % Masse/Masse | |
| P = 1,29 % Masse/Masse | |
| C = 2,67 % Masse/Masse | |
| N = 0,38 % Masse/Masse | |
| Taux de greffage [composé A / Fe] = 1,95 % mole/mole | |
| Taux de greffage [composé F / Fe] = 0,28 % mole/mole | |
| Taux de greffage [composé F / composé A] = 14,5 % | |

### Relaxivités :

### 20MHz (0,47 T) - 37°C - en solution aqueuse

| r₁ (mM⁻¹.s⁻¹) | r₂ (mM-1.s⁻¹) |
|---|---|
| 36 ± 2 | 105 ± 5 |

Ces résultats montrent que le couplage du biovecteur ne modifie pas les relaxivités de l'intermédiaire non couplé (exemple 12).

### Exemple 18 :

100 ml d'une solution de l'exemple 12 à 0,285 M/L sont ultrafiltrés sur une cellule à agitation de 30 KD PALL^{®}. A cette solution, 202 mg du composé G de l'exemple 7 sont ajoutés. Le pH est ajusté à 6,1 avec QSP de HCl [0,1 N].

203 mg de chlorhydrate de 1-(3-diméthylaminopropyl)-3-éthylcarbodiimide sont ajoutés et l'ensemble est agité 6 heures à température ambiante.

Le milieu réactionnel est ultrafiltré sur cellule à agitation PALL^{®} ayant un seuil de coupure de 30 kD. 800 ml de filtrat sont éliminés. Le rétentat est ajusté à 140 ml puis est filtré sur 0,22 µm.

| | |
|---|---|
| [Fe] : 0,223 M/L | Taille PCS = 28,4 nm |
| Fe = 57,9 % Masse/Masse | |
| P = 1,37 % Masse/Masse | |
| C = 3,49 % Masse/Masse | |
| N = 0,89 % Masse/Masse | |
| Taux de greffage [composé A / Fe] = 2,13 % mole/mole | |
| Taux de greffage [composé G / Fe] = 1,5 % mole/mole | |
| Taux de greffage [composé G / composé A] = 71 % | |

### Exemples 19 à 21 : Comparaison des particules magnétiques complexées par un composé gem-bisohosohonate et couplées à un biovecteur, dérivant d'un précurseur ferrofluide "alcalin" avec celles dérivant d'un précurseur ferrofluide "acide"

### Exemple 19 : Préparation d'un ferrofluide « alcalin » en milieu TMAOH

Un mélange constitué d'une solution de 2,96 ml de FeCl₃ à 27 %, et d'une solution de 0,6 g de FeCl₂, 4 H₂O dans 40 ml d'eau, est versée rapidement dans 200 ml de tétraméthylammonium hydroxyde [1 M]. L'ensemble est agité 1 heure à température ambiante. La solution est filtrée sur une membrane 0,22 µm et maintenue sous azote à 4 °C.
[Fe] : 0,0403 M/L Taille PCS = 68,2 nm Polydispersité σ = 0,48

### Exemple 20 : Complexation du ferrofluide "alcalin" de l'exemple 19 par le composé gem-bisphosphonate de l'exemple 1

Une solution constituée de 416 mg du composé A de l'exemple 1 en solution dans 5 ml d'eau est introduite dans 25 ml de l'exemple 19. L'ensemble est agité 1 heure puis ultrafiltré sur cellule à agitation PALL^{®} ayant un seuil de coupure de 30 kD.

600 ml de filtrat sont éliminés. Le rétentat est ajusté à 40 ml puis est filtré sur membrane 0,22 µm.

### Exemple 21 : Couplage des particules magnétiques complexées de l'exemple 20 avec le composé E de l'exemple 5

1,7 g de composé E sont introduits dans les 40 ml de solution de l'exemple 20. Le pH est ajusté à 6,2. 345 mg de chlorhydrate de 1-(3-diméthylaminopropyl)-3-éthylcarbodiimide sont alors ajoutés et l'ensemble est agité 24 heures à température ambiante.

Le milieu réactionnel est ultrafiltré sur cellule à agitation PALL^{®} ayant un seuil de coupure de 30 kD.

700 ml de filtrat sont éliminés. Le rétentat est ajusté à 40 ml puis est filtré sur 0,22 µm.

| | |
|---|---|
| Taille PCS = 60 nm | Polydispersité σ = 0,60 |
| Fe = 37,9 % Masse/Masse | |
| P = 1,70 % Masse/Masse | |
| C = 12,96 % Masse/Masse | |
| N= 1,84 % Masse/Masse | |
| Br = 6,42 % Masse/Masse | |
| Taux de greffage [composé A / Fe] = 4,04 % mole/mole | |
| Taux de greffage [composé E / Fe] = 3,95 % mole/mole | |
| Taux de greffage [composé E / composé A] = 97,7 % | |

### Précurseurs

| | N° | Taille Z ave (nm) | Polydispersité σ |
|---|---|---|---|
| Ferrofluide acide | Exemple 8 | 40 | 0,22 |
| Ferrofluide alcalin | Exemple 19 | 68,2 | 0,48 |

### Molécules finales (ferrofluide acide ou basique + composé gem-biphosphonate (la) + biovecteur composé E) :

| | N° | Taille Z ave (nm) | Polydispersité σ |
|---|---|---|---|
| Procédé acide | Exemple 16 | 41,6 | 0,22 |
| Procédé alcalin | Exemple 21 | 60 | 0,60 |

### Conclusions :

Pour les exemples 19 et 21, les inventeurs ont avantageusement démontré que lorsqu'on utilise un ferrofluide alcalin comparable à celui mis en oeuvre dans le document J. of Coll. and Interf., Science, 2001, 238, pp. 37-42, la taille hydrodynamique est plus élevée dès l'étape du précurseur (ferrofluide alcalin - exemple 19) et la polydispersité est forte (σ = 0,48) comparé au ferrofluide acide (σ = 0,22) ; cela se répercute sur la taille hydrodynamique du produit final (après greffage du composé la et du biovecteur - composé E), avec une polydispersité encore plus grande (σ = 0,6). La différence de % (composé A / Fer) entre les deux types de particules finales issues respectivement d'un ferrofluide acide ou basique indique la formation certaine d'une double couche de composé I (*gem*-biphosphonate) dans le cas de la mise en oeuvre d'un précurseur "alcalin", ce qui est préjudiciable à une bonne stabilité. Dans le procédé selon l'invention, nous avons 90 % des sites greffés avec un composé de formule (I), formant avantageusement une mono-couche.

### Exemples 22 à 25 : Préparation d'autres biovecteurs

### Exemple 22 : Préparation du composé H :

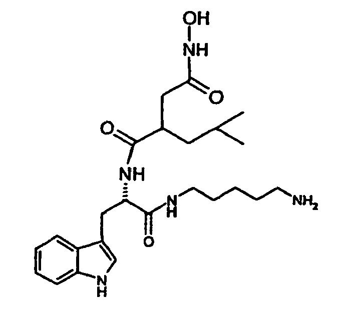

### 1) Benzyl 5-{[(2R)-2-{[(9H-fluorèn-9-ylméthoxy)carbonyl]amino}3-(1H-indol-3-yl)propanoyl]amino}pentylcarbamate

15,63 g de (acide(2R)-2-{[(9H-fluorèn-9-ylméthoxy)carbonyl]amino}-3-(1H-indol-3-yl) propanoïque) sont solubilisés à température ambiante dans 300 ml de tétrahydrofuranne.

10 g de (benzyl-5-aminopentylcarbamate) puis 5,1 ml de triéthylamine sont additionnés. L'ensemble est agité 5 minutes à température ambiante.

5,94 g d'hydroxy-1-benzotriazole hydraté puis 8,43 g de chlorhydrate de 1-(3-diméthyl-aminopropyl)-3-éthylcarbodiimide sont ensuite ajoutés au milieu réactionnel. L'ensemble est agité 24 heures à température ambiante.

L'insoluble est éliminé par filtration. Le filtrat est concentré sous vide.

L'huile obtenue est versée dans 150 ml d'eau et est agité pendant 1 heure à température ambiante.

Le précipité obtenu est filtré, et lavé sous forte agitation dans 100 ml d'eau pendant 30 minutes à température ambiante.

Le précipité est filtré puis lavé par 200 ml d'éther éthylique puis séché sous vide. 22,78 g sont isolés avec un rendement de 96,4%.
Rf = 0,94 sur silice (MERCK) dans CH₂Cl₂ / CH₃OH (8/2)
HPLC : Colonne Symmetry Waters C18, 5 µm, (250 mm x 4,6 mm) Gradient : Eau-TFA (pH 2,95) / CH₃CN : λ = 210 nm

| Temps en min. | Débit (ml/min.) | % Eau-TFA pH 2,95 | %CH₃CN |
|---|---|---|---|
| 0 | 1 | 98 | 2 |
| 50 | 1 | 10 | 90 |
| 55 | 1 | 5 | 95 |

### Temps de rétention du produit attendu : 42 min

Spectre de masse : m/z = 645,3 (mode ES⁺) (M théorique = 644)

### 2) Benzyl 5-{[(2R)-2-amino-3-(1H-indol-3-yl)propanoyl]amino} pentylcarbamate

20 g de (benzyl 5-{[(2R)-2-{[(9H-fluorèn-9-ylméthoxy)carbonyl]amino}-3-(1H-indol-3-yl)propanoyl]amino} pentylcarbamate) sont solubilisés à température ambiante dans 280 ml de tétrahydrofuranne.

41,4 ml de pipéridine et 20 ml d'eau sont ensuite additionnés. L'ensemble est agité 3 heures à température ambiante. Le milieu réactionnel est concentré sous vide.

L'huile obtenue est purifiée sur 1400 g de silice Merck Geduran ® (40-63 µm). Elution : CH₂Cl₂ / CH₂OH (95/5).
12,77 g sont isolés avec un rendement de 97,4%.
Rf = 0,64 sur silice (MERCK) dans CH₂Cl₂/ / CH₃OH (95/5)
HPLC : Colonne Symmetry Waters C18, 5 µm, (250 mm x 4,6 mm) Gradient : Eau-TFA (pH 2,95) / CH₃CN :
λ = 210 nm

| Temps en min | Débit (ml/min) | % Eau-TFA pH 2,95 | %CH₃CN |
|---|---|---|---|
| 0 | 1 | 98 | 2 |
| 17 | 1 | 66 | 34 |
| 19 | 1 | 64 | 36 |
| 50 | 1 | 0 | 100 |

Temps de rétention du produit attendu : 18,2 min
Spectre de masse : m/z = 423,2 (mode ES⁺) (M théorique = 422)

### 3) Tert-butyl (12R)-12-(1H-indol-3-ylméthyl)-15-isobutyl-3, 11, 14-trioxo-1-phényl-2-oxa-4,10,13-triazaheptadécan-17-oate

9,16 g de (acide 2-(2-tert-butoxy-2-oxoéthyl)-4-méthylpentanoïque), synthétisé selon le mode opératoire décrit dans (J.Med.Chem.1998, Vol 41 (2) : p 209), sont solubilisés à température ambiante dans 160 ml de tétrahydrofuranne.

Une solution homogène formée par 16,81 g (0,039 mole) de (benzyl 5-{[(2R)-2-amino-3-(1H-indol-3-yl)propanoyl]amino}pentylcarbamate) dans 165 ml de tétrahydrofuranne est additionnée en une seule fois suivi par, successivement, 8,3 ml de triéthylamine, 6,45 g d'hydroxy-1-benzotriazole hydraté et 9,5 g de chlorhydrate de 1-(3-diméthylaminopropyl)-3-éthylcarbodiimide.

L'ensemble est agité 12 heures à température ambiante. L'insoluble est éliminé par filtration. Le filtrat est concentré sous vide.

L'huile obtenue est purifiée sur 1900 g de silice Merck Geduran^{®} (40-63 µm). Elution du bon produit par CH₂Cl₂ / CH₃OH (98/2).

24 g sont isolés avec un rendement de 95,3%.
Rf = 0,42 sur silice (MERCK) dans CH₂Cl₂ / CH₃OH (95/5)
HPLC : Colonne Symmetry Waters C18, 5 µm, (250 mm x 4,6 mm) Gradient : Eau-TFA (pH 2,95) / CH₃CN :
λ = 220 nm

| Temps en min | Débit (ml/min) | % Eau-TFA pH 2,95 | %CH₃CN |
|---|---|---|---|
| 0 | 1 | 98 | 2 |
| 30 | 1 | 0 | 100 |

Temps de rétention du produit attendu : 25,7 min
Spectre de masse : m/z = 635,6 (mode ES⁺) (M théorique = 634)

### 4) Acide(12R)-12-(1H-indol-3-ylméthyl)-15-isobutyl-3,11,14-trioxo-1-phényl-2-oxa-4, 10, 13-triazaheptadécan-17-oïque

1,4 g de dithioérythritol sont additionnés dans une solution composée de 100 ml de CH₂Cl₂ et de 100 ml d'acide trifluoroacétique. 10 g de (tert-butyl (12R)-12-(1H-indol-3-ylméthyl)-15-isobutyl-3,11,14-trioxo-1-phényl-2-oxa-4,10,13-triazaheptadécan-17-oate) sont ensuite ajoutés. L'ensemble est agité 30 minutes à température ambiante puis concentré sous vide.

L'huile obtenue est purifiée sur 700 g de silice Merck Geduran^{®} (40-63 µm). Elution du bon produit par CH₂Cl₂ / CH₃OH (98/2).
6,19 g sont isolés avec un rendement de 68%.
Rf = 0,57 sur silice (MERCK) dans CH₂Cl₂ / CH₃OH (8/2)
HPLC : Colonne Symmetry Waters C18, 5 µm, (250 mm x 4,6 mm) Gradient : Eau-TFA (pH 2,95) / CH₃CN : λ = 210 nm

| Temps en min. | Débit (ml/min.) | % Eau-TFA pH 2,95 | %CH₃CN |
|---|---|---|---|
| 0 | 1 | 98 | 2 |
| 30 | 1 | 0 | 100 |

Temps de rétention du produit attendu : 20,8 min
Spectre de masse : m/z = 579,0 (mode ES⁺) (M théorique = 578)

### 5) Benzyl (8R)-8-(1H-indol-3-ylméthyl)-11-isobutyl-7,10,13-trioxo-16-phényl-15-oxa-6,9,14-triazahexadéc-1-ylcarbamate

6,11 g d'acide(12R)-12-(1H-indol-3-ylméthyl)-15-isobutyl-3,11,14-trioxo-1-phényl-2-oxa-4,10,13-triazaheptadécan-17-oïque sont solubilisés à température ambiante dans 160 ml de tétrahydrofuranne. Le milieu réactionnel est refroidi à 0°C.

1,69 g de chlorhydrate de O-benzylhydroxylamine, 3 ml de triéthylamine, 1,86 g de d'hydroxy-1-benzotriazole hydraté puis 2,63 g de chlorhydrate de 1-(3-diméthyl-aminopropylp)-3-éthylcarbodiimide sont successivement ajoutés au milieu réactionnel et l'ensemble est agité 24 heures à température ambiante.

L'insoluble est éliminé par filtration. Le filtrat est concentré sous vide.

L'huile obtenue est versée dans 200 ml d'eau. Le précipité obtenu est filtré, relavé dans 100 ml d'eau à température ambiante.

Le précipité est filtré et séché sous vide.
5,7 g sont isolés avec un rendement de 79,2%.
Rf = 0,29 sur silice (MERCK) dans CH₂Cl₂ / CH₃CN (5/5)
HPLC : Colonne Symmetry Waters C18, 5 µm, (250 mm x 4,6 mm) Gradient : Eau-TFA pH (2,95) / CH₃CN : λ = 210 nm

| Temps en min | Débit (ml/min) | % Eau-TFA pH 2,95 | %CH₃CN |
|---|---|---|---|
| 0 | 1 | 98 | 2 |
| 30 | 1 | 0 | 100 |

Temps de rétention du produit attendu : 22,7 min
Spectre de masse : m/z = 684,3 (mode ES⁺) (M théorique = 683)

### 6) N¹-[(1R)-2-[(5-aminophentyl)amino]-1-(1H-indol-3-ylméthyl)-2-oxoéthyl]-N⁴-hydroxy-2-isobutylsuccinamide

0,32 g de benzyl (8R)-8-(1H-indol-3-ylméthyl)-11-isobutyl-7,10,13-trioxo-16-phényl-15-oxa-6, 9,14-triazahexadéc-1-ylcarbamate est dissous dans 30 ml de méthanol. Une demi-spatule de Pd/C 50% hydraté est ajoutée à la solution.

L'ensemble est agité 4 heures et trente minutes à température ambiante sous 50 PSI d'hydrogène. Le catalyseur est éliminé par filtration sur clarcel et le filtrat est concentré sous vide. Rendement quantitatif.

Le produit obtenu est purifié sur 5 g de gel de silice 60 silanisé Merck (63-200 µm). Elution du produit attendu par H₂O.
0,11 g sont isolés avec un rendement de 51%.
HPLC : Colonne Symmetry Waters C18, 5 µm, (250 mm x 4,6 mm) Gradient : Eau-TFA (pH 2,95) / CH₃CN
λ = 220 nm

| Temps en min. | Débit (ml/min.) | % Eau-TFA pH 2,95 | %CH₃CN |
|---|---|---|---|
| 0 | 1 | 98 | 2 |
| 30 | 1 | 0 | 100 |

Temps de rétention du produit attendu : 9,4 min
Spectre de masse : m/z = 460,3 (mode ES⁺) (M théorique = 459)

### Exemple 23 : Préparation du composé I :

### 1) O-(2-paratoluènesulfonyléthyl)-O'-méthylpolyéthlylène glycol 1100

16,2 g de polyéthylèneglycolmonométhyléther de masse moyenne 1100 (Fluka ®) sont dissous dans 25 ml de dichloromethane. Cette solution est refroidie à une température de 5 °C.

0,4 g de chlorure de triéthylbenzylammonium, 10,4 ml d'une solution aqueuse à 30% d'hydroxyde de sodium puis 2,94 g de chlorure de paratoluènesulfonyl en solution dans 15 ml de CH₂Cl₂ sont ensuite additionnés à 5 °C.

La réaction est poursuivie 24 heures à température ambiante. L'extraction du milieu réactionnel conduit, après séchage sur sulfate de magnésium et évaporation, à 17,2 g de solide. Le rendement est de 95 %.
CCM : SiO₂ Merck® Eluant : dichlorométhane / méthanol : 9/1.
Révélation : UV 254 nm et Draggendorf _{:} Rf = 0,4
◆ Infra Rouge : 1356 cm⁻¹ (v : R-O-SO₂-R)

### 2) O-(2-azidoéthyl)-O'-méthylpolyéthylène glycol 1100

12,4 g de O-(2- paratoluènesulfonyléthyl)-O'-méthylpolyéthlylène glycol 1100 sont dissous à température ambiante dans 90 ml de DMF. 2 g d'azide de sodium sont ensuite introduits. L'agitation est maintenue 18 heures à température ambiante. L'extraction du milieu réactionnel par le CH₂Cl₂ conduit, après évaporation, à 4,4 g de composé. Le rendement est de 40 %

CCM : SiO₂ Merck® Eluant : dichlorométhane / méthanol : 9/1.
Révélation : UV 254 nm et Draggendorf Rf = 0,39
◆ Infra Rouge : 2104 cm⁻¹ (v : N₃)

### 3) O-(2-Aminoéthyl)-O'-méthylpolyéthylène glycol 1100

4,3 g de O-(2-azidoéthyl)-O'-méthylpolyéthylène glycol 1100 et 0,4 g de Pd/C (5%) en solution dans 100 ml d'éthanol sont introduits dans l'autoclave. La réaction est conduite sous une pression d'hydrogène de 2,5 bars, à 25 °C, pendant 4 heures.

Le catalyseur est éliminé par filtration sur célite. Après évaporation, 3,5 g sont ainsi isolés avec un rendement de 85 %.

CCM : SiO₂ Merck® Eluant : dichlorométhane / méthanol : 9/1.
Révélation : Nihydrine Spécifique « Amine primaire »
◆ Infra Rouge : 3390 cm⁻¹ (v NH₂)

### Exemple 24 : Préparation du composé J :

### 1) Tert-butyl 3-{2-[2-(3-aminopropoxy)éthoxy]éthoxy}propylcarbamate

A 15 g de 3-{2-[2-(3-aminopropoxy)éthoxy]éthoxy}-1-propanamine dissout dans 100 ml de CH₂Cl₂ est ajouté, au moyen d'une ampoule à brome, une solution de 5 g de di(*tert*-butyl) dicarbonate dissout dans 50 ml de CH₂Cl₂.

Le milieu réactionnel est agité à température ambiante pendant 18 heures puis est concentré à moitié pour être lavé à l'eau (50 ml), séché et concentré. La solution obtenue est filtrée sur silice MERCK Geduran^{®} (40-63 µm) avec de l'acétate d'éthyle puis du méthanol.

4,7g de produit sont obtenus avec un rendement de 64%.
Spectre de masse : M/z = 321,3 (ES+) M théorique = 320,4
CCM : Rf=0,05 ; Silice SiO₂ ; éluant = CH₂Cl₂/MeOH/NH₄OH (93/7/traces)

### 2)Tert-butyl 16-chloro-15-oxo-4,7,10-trioxa-14-azahexadéc-1-ylcarbamate

Dans un tricol muni d'un bain d'acétone et de glace est mis en solution 10 g de tert-butyl 3-{2-[2-(3-aminopropoxy)éthoxy]éthoxy}propylcarbamate dans 50 ml de CH₂Cl₂. Au moyen d'une ampoule à brome sont ajoutés goutte à goutte et simultanément 5 g de K₂CO₃ dissout dans 50 ml d'eau et 5 g de chlorure de chloroacétyle dissout dans 50 ml de CH₂Cl₂. Au cours de l'addition, la température du milieu est maintenue à 0°C.

Quand l'addition des réactifs est terminée, le milieu réactionnel est maintenu sous agitation magnétique, à température ambiante pendant 1 heure.

Les 2 phases obtenues sont séparées puis la phase chlorométhylénique est lavée à l'eau jusqu'à pH neutre, séchée sur Na₂SO₄, filtrée puis concentrée.

11,2 g de produit sont obtenus avec un rendement de 90%.
Spectre de masse : M/z = 397,4 (ES +) M théorique = 396,9
CCM : Rf = 0,6 ; Silice SiO₂ ; éluant = CH₂Cl₂/MeOH (9/1)

### 3) Tert-butyl 30-amino-15-oxo- 4,7,10,21,24,27-hexaoxa-14,17-diazatriacont-1-ylcarbamate

6,8 g de 3-{2-[2-(3-aminopropoxy)éthoxy]éthoxy}propylamine sont dilués dans 50 ml de CH₃CN en présence de K₂CO₃ A cette suspension sont ajoutés, au moyen d'une ampoule à Brome, 3,5 g de tert-butyl 16-chloro-15-oxo-4,7,10-trioxa-14-azahexadéc-1-ylcarbamate dissout dans 25 ml de CH₃CN.

Le milieu réactionnel est chauffé au reflux pendant 2 heures puis est mis à température ambiante pour une filtration sur verre fritté. Le filtrat est ensuite évaporé à sec.

Le résidu obtenu est dissout dans 50 ml de CH₂Cl₂ pour faire des lavages avec 4 fois 20 ml d'eau. La phase chlorométhylénique est séchée sur Na₂SO_{4,} filtrée et concentrée suffisamment pour envisager directement une purification sur Silice Merck Geduran^{®} (40-63 µm) avec une élution CH₂Cl₂ / MeOH (50/50) (facteur de rétention Rf = 0,15) .

1,8 g sont obtenus avec un rendement de 50%.
HPLC : Colonne Symmetry C18 (3,5 µm) 50x2,1 mm
- Détecteur : UV 201 nm
- Phase mobile :
A : Eau +TFA (pH 2,8)
B : CH₃CN

| Temps (min.) | Débit (ml/min.) | %A % A | %B % B |
|---|---|---|---|
| 0 | 1 | 98 | 2 |
| 4 | 1 | 0 | 100 |
| 5 | 1 | 0 | 100 |

Le produit attendu a un temps de rétention = 1,8min.
Spectre de masse : M/z = 581 (ES+) M théorique = 580,7

### 4) Tert-butyl 30-[(2-éthoxy-3,4-dioxo-1-cyclobutèn-1-yl)amino]-15-oxo-4,7,10,21,24,27-hexaoxa-14,17-diazatriacont-1-ylcarbamate

0,25 g de tert-butyl 30-amino-15-oxo-4,7,10,21,24,27-hexaoxa-14,17-diazatriacont-1-ylcarbamate est mis en solution dans 1,5 ml de CH₂Cl₂. On additionne 57,5 µl de 3,4-diéthoxy-3-cyclobutène-1,2-dione

Le milieu réactionnel est mis sous agitation magnétique pendant 18 heures à température ambiante. Cet intermédiaire n'est pas isolé.
HPLC : Colonne Symmetry C18 (3,5µm) 50x2,1 mm
- Détecteur : UV 201 nm
- Phase mobile :
A : Eau + TFA (pH 2,8)
B : CH₃CN

| Temps (min) | Débit (ml/min) | %A | %B |
|---|---|---|---|
| 0 | 1 | 98 | 2 |
| 4 | 1 | 0 | 100 |
| 5 | 1 | 0 | 100 |

Le produit attendu a un temps de rétention de 2,30 min.

### 5) Tert-butyl 17-acétyl-30-[(2-éthoxy-3,4-dioxo-1-cyclobutèn-1-yl)amino]-15-oxo-4,7,10,21,24,27-hexaoxa-14,17-diazatriacont-1-ylcarbamate

Au milieu réactionnel obtenu à l'étape 4) est ajouté 40,6µl d'anhydride acétique. Le milieu réactionnel est agité à température ambiante puis est purifié directement sur 5g de Silice Merck Geduran^{®} (40-63µm) à pression atmosphérique, avec un éluant CH₂Cl₂/EtOH (9/1).
0,27g de produit sous forme d'huile est obtenu avec un rendement de 60%.
Spectre de masse : M/z =748 (ES +) M théorique = 746,9

### 6) Acide 1-({2-[(14-acétyl-34,34-diméthyl-16,32-dioxo-4,7,10,21,24,27,33-heptaoxa-14,17,31-triazapentatriacont-1-yl)amino]-3,4-dioxo-1-cyclobutèn-1-yl}amino)-18-[(4-{[(2-amino-4-oxo-3,4-dihydro-6-ptéridinyl)méthyl]amino}benzoyl)amino]-15-oxo-4,7,10-trioxa-14-azanonadécan-19-oïque

0,16 g de tert-butyl 17-acétyl-30-[(2-éthoxy-3,4-dioxo-1-cyclobutèn-1-yl)amino]-15-oxo-4,7,10,21,24,27-hexaoxa-14,17-diazatriacont-1-ylcarbamate et 0,205 g de l'exemple 6 (composé F) sont solubilisés dans 3 ml d'eau .

Des gouttes d'eau saturée en Na₂CO₃ sont ajoutées dans le milieu pour obtenir un pH=9,2. Le milieu est maintenu sous agitation magnétique pendant 18 heures puis est filtré sur papier Whatmann, neutralisé à l'aide d'HCl (1 N) et repris dans 15 ml de CH₂Cl₂.

La phase chlorométhylénique est lavée avec 1 ml d'eau puis séchée sur Na₂SO₄, filtrée et évaporée. Le résidu est repris dans 10 ml d'EtOH pour être précipité dans 100 ml d'Et₂.

0,2 g de cristaux (couleur orange safran) sont obtenus avec un rendement de 69%.
HPLC : Colonne Symmetry 18 (3,5 µm) 50x2,1 mm
- Détecteur : UV 201 nm
- Phase mobile :
A : Eau +TFA (pH 2,8)
B : CH₃CN

| Temps (min) | Débit (ml/min) | % A | % B |
|---|---|---|---|
| 0 | 1 | 98 | 2 |
| 4 | 1 | 0 | 100 |
| 5 | 1 | 0 | 100 |

Le produit attendu a un temps de rétention de 2,40 min.
Spectre de masse : M/z = 673 (z= 2 ; ES +) Masse théorique = 1344,5

### 7) Acide 1-({2-[(14-acétyl-30-amino-16-oxo-4,7,10,21,24,27-hexaoxa-14,17-diazatriacont-1-yl)amino]-3,4-dioxo-1-cyclobutèn-1-yl}amino)-18-[(4-{[(2-amino-4-oxo-3,4-dihydro-6-ptéridinyl)méthyl]amino}benzoyl)amino]-15-oxo-4,7,10-trioxa-14-azanonadécan-19-oïque

0,2 g de l'acide 1-({2-[(14-acétyl-34,34-diméthyl-16,32-dioxo-4,7,10,21,24, 7,33-heptaoxa-14,17,31-triazapentatriacont-1-yl)amino]-3,4-dioxo-1-cyclobutèn-1-yl}amino)-18-[(4-{[(2-amino-4-oxo-3,4-dihydro-6ptéridinyl)méthyl]amino}benzoyl) amino]-15-oxo-4,7,10-trioxa-14-azanonadécan-19-oïque sont mis en solution dans 2 ml d'acide trifluoroacétique à température ambiante.

Le milieu réactionnel est ensuite précipité dans 20 ml d'Et₂O, filtré et lavé abondamment à l'éther éthylique.

0,18 g de cristaux sont obtenus avec un rendement de 78%.
HPLC : Colonne Symmetry C18 (3,5 µm) 50 x 2,1 mm
- Détecteur : UV 201 nm
- Phase mobile :
   A : Eau +TFA (pH 2,8)
   B : CH₃CN

| Temps (min) | Débit (ml/min) | %A | %B |
|---|---|---|---|
| 0 | 1 | 98 | 2 |
| 4 | 1 | 0 | 100 |
| 5 | 1 | 0 | 100 |

Le produit attendu a un temps de rétention de 1,9 min.
Spectre de masse : M/z = 1245 (ES+) M théorique = 1244,4

### Exemple 25 : Préparation du composé K :

### 1) tert-butyl 35-[(2-éthoxy-3,4-dioxo-1-cyclobutèn-1-yl)amino]-3,6,9,12,15,18,21,24,27,30,33 -undécaoxapentatriacont-1-ylcarbamate

0,8 g de tert-butyl 35-amino-3,6,9,12,15,18,21,24,27,30,33-undécaoxapentatriacont-1-ylcarbamate sont solubilisés à température ambiante dans 4 ml d'EtOH . A cette solution sont additionnés 165µl de 3,4-diéthoxy-3-cyclobutène-1,2-dione.

L'ensemble est agité, à température ambiante pendant 18 heures. Après évaporation du solvant, le résidu obtenu est dissout dans 5 ml de CH₂Cl₂ pour être purifié sur 30 g de silice MERCK Geduran^{®} (4-63 µm) avec un éluant composé de CH₂Cl_{2/}MeOH (95/5).

0,6 g d'huile est obtenue avec un rendement de 70%.
CCM : Rf = 0,5 ; silice SiO₂ ; éluant = CH₂Cl₂/MeOH (9/1)
HPLC : Colonne Symmetry C18 (3,5µm) 50 x 2,1 mm
- Détecteur : UV (201 nm)
- Phase mobile :
A : Eau + TFA (pH 2,8)
B : CH₃CN

| Temps (min) | Débit (ml/min) | %A | %B |
|---|---|---|---|
| 0 | 1 | 98 | 2 |
| 4 | 1 | 0 | 100 |
| 5 | 1 | 0 | 100 |

Le produit attendu a un temps de rétention de 2,78 min.
Spectre de masse : M/z = 769,5 (ES+) M théorique=768,9

### 2) Acide 18-[(4-{[(2-amino-4-oxo-3,4-dihydro-6-ptéridinyl)méthyl]amino} benzoyl)amino]-1-({2-[(39,39-diméthyl-37-oxo-3,6,9,12,15,18,21,24,27,30,33,38 -dodécaoxa-36-azatétracont-1-yl)amino]-3,4-dioxo-1-cyclobutèn-1-yl}amino)-15-oxo-4,7,10-trioxa-14-azanonadécan-19-oïque

0,3 g de tert-butyl 35-[(2-éthoxy-3,4-dioxo-1-cyclobutèn-1-yl)amino]-3,6,9,12,15,18,21,24,27,30,33-undecaoxapentatriacont-1-ylcarbamate et 0,34 g de l'exemple 6 (composé F) sont solubilisés dans 4,5 ml d'eau et 0,7 ml d'EtOH.

Le pH du milieu est augmenté à 9,2 avec des gouttes d'eau saturée en Na₂CO₃. Le milieu réactionnel est agité à température ambiante pendant 48 heures avec maintien du pH à 9,2.

Après évaporation de l'éthanol, la solution aqueuse obtenue est purifiée sur un système de colonne chromatographique « Supervarioflash^{®}» avec une cartouche K026033 (RP 18 ; 25-40 µm) avec un éluant Eau/CH₃CN (98/2) et un débit de 20 ml/min.

100 mg de produit sont obtenus avec un rendement de 25%.
HPLC : Colonne Symmetry C18 (3,5µm) 50x2,1 mm
- Détecteur : UV (275 nm)
- Phase mobile :
A : Eau + TFA (pH 2,8)
B : CH₃CN

| Temps (min) | Débit (ml/min) | %A | %B |
|---|---|---|---|
| 0 | 1 | 98 | 2 |
| 4 | 1 | 0 | 100 |
| 5 | 1 | 0 | 100 |

Le produit attendu a un temps de rétention de 2,40 min.
Spectre de masse : M/z = 684 (z=2; ES+) M théorique = 1366,5

### 3) Acide 18-[(4-{[(2-amino-4-oxo-3,4-dihydro-6-ptéridinyl)méthyl]amino} benzoyl)amino]-1-({2-[(35-amino-3,6,9,12,15,18,21,24,27,30,33-undécaoxapentatriacont-1-yl)amino]-3,4-dioxo-1-cyclobutèn-1-yl}amino)-15-oxo-4,7,10-trioxa-14-azanonadécan-19-oïque

0.3 g de l'acide 18-[(4-{[(2-amino-4-oxo-3,4-dihydro-6-ptéridinyl)méthyl] amino}benzoyl)amino]-1-({2-[(39,39-diméthyl-37-oxo-3,6,9,12,15,18,21,24,27,30, 33,38-dodécaoxa-36-azatétracont-1-yl)amino]-3,4-dioxo-1-cyclobutèn-1-yl}amino)-15-oxo-4,7,10-trioxa-14-azanonadécan-19-oïque sont solubilisés dans 0,3 ml d'acide trifluoroacétique.

Après 15 minutes d'agitation à température ambiante, le milieu réactionnel est évaporé puis repris dans 30 ml d'Et₂O.

Après filtration, lavage à l'Et₂O et séchage, 0,29 g de produit sont obtenus.
HPLC : Colonne Symmetry C18 (3,5µm) 50x2,1 mm
- Détecteur : UV (275 nm)
- Phase mobile :
A : Eau + TFA (pH 2,8)
B : CH₃CN

| Temps (min) | Débit (ml/min) | % A | % B |
|---|---|---|---|
| 0 | 1 | 98 | 2 |
| 4 | 1 | 0 | 100 |
| 5 | 1 | 0 | 100 |

Le produit attendu a un temps de rétention de 1,87 min.
Spectre de masse : M/z = 1267 (ES+) M théorique = 1266,4

### Exemples 26 à 37 : Couplage des particules magnétiques acides (p) complexées par des composés de formule (I) avec des biovecteurs

### Exemple 26 :

1) 102,5 mg du composé F de l'exemple 6 sont introduits dans 50 ml de l'exemple 12 à 0,285 M/L. 76,6 mg de chlorhydrate de 1-(3-diméthylaminopropyl)-3-éthylcarbodiimide sont ajoutés. Le pH est ajusté à 6,2 et l'ensemble est agité 18 heures à température ambiante.
Le milieu réactionnel est directement ultrafiltré sur cellule à agitation PALL^{®} ayant un seuil de coupure de 30 kD. Le rétentat est ajusté à 65 ml puis est filtré sur 0,22 µm donnant la solution A.

| |
|---|
| Fe = 67 % Masse/Masse |
| P = 1,29 % Masse/Masse |
| C = 1,95 % Masse/Masse |
| N = 0,26 % Masse/Masse |
| Taux de greffage [composé A / Fe] = 1,74 % mole/mole |
| Taux de greffage [composé F / Fe] = 0,17 % mole/mole |
| Taux de greffage [composé F / composé A] = 9,8 %. |

2) 3 g du composé E de l'exemple 5 sont introduits dans 50 ml de la solution A obtenue à l'étape 1). 600 mg de chlorhydrate de 1-(3-diméthylaminopropyl)-3-éthylcarbodiimide sont ajoutés. Le pH est ajusté à 6,2 et l'ensemble est agité 18 heures à température ambiante.
Le milieu réactionnel est directement ultrafiltré sur cellule à agitation PALL^{®} ayant un seuil de coupure de 30 kD. Le rétentat est ajusté à 40 ml puis est filtré sur 0,22 µm.

| |
|---|
| [Fe]: 0,289 M/L Taille PCS = 29,6 nm |
| Fe = 56,6 % Masse/Masse |
| P = 1,1 % Masse/Masse |
| C = 7,27 % Masse/Masse |
| N = 1,1 % Masse/Masse |
| Br = 2,6 % Masse/Masse |
| Taux de greffage [composé A / Fe] = 1,75 % mole/mole |
| Taux de greffage [composé E / Fe] = 1,07 % mole/mole |
| Taux de greffage [composé E / composé A] = 61 % |

### Exemple 27 :

36,5 mg du composé H de l'exemple 22 sont introduits dans 30 ml de l'exemple 12 à 0,285 M/L. Le pH est ajusté à 6,2 et 18 mg de chlorhydrate de 1-(3-diméthyl-amino-propyl)-3-éthylcarbodiimide sont ajoutés. L'ensemble est agité 18 heures à température ambiante.

Le milieu réactionnel est directement ultrafiltré sur cellule à agitation PALL^{®} ayant un seuil de coupure de 30 kD. Le rétentat est ajusté à 65 ml puis est filtré sur 0,22 µm.

| |
|---|
| [Fe] : 0,292 M/L Taille PCS = 32,2 nm |
| Fe = 66,0 % Masse/Masse |
| P = 1,48 % Masse/Masse |
| C = 3,35 % Masse/Masse |
| N = 0,69 % Masse/Masse |
| Taux de greffage [composé A / Fe] = 2,02 % mole/mole |
| Taux de greffage [composé H / Fe] = 0,83 % mole/mole |
| Taux de greffage [composé H / composé A] = 41 % |

### Exemple 28 :

### 1) 9H-fluorèn-9-ylméthyl 6-(phosphonooxy)hexylcarbamate

1,33 ml de POCl₃ et 1,83 ml de triétylamine sont mis en solution dans 9 ml d'heptane. 3 g de 6-(FMOC-amino)-1-hexanol en solution dans 30 ml de CH₂Cl₂ sont introduits goutte à goutte à la solution précédente en maintenant une température de 0°C.

Le milieu réactionnel est agité 1 heure à 0°C puis 18 heures à température ambiante. 30 ml d'eau sont ajoutés et l'ensemble est agité 2 heures à température ambiante.

La phase organique est lavée à l'eau puis concentrée sous vide. Le solide résultant est repris à l'eau, filtré et séché.

3 g de produit sont obtenus avec un rendement de 81% et une pureté HPLC de 60 %.
HPLC : Colonne X-TERRA C18 (5µm) 250x4,6 mm
- Détecteur : UV 201 nm
- Phase mobile :
A : Solution de Carbonate d'ammonium pH = 9
B : CH₃CN

| Temps (min) | Débit (ml/min) | % A | % B |
|---|---|---|---|
| 0 | 1 | 98 | 2 |
| 30 | 1 | 2 | 98 |

Le produit attendu a un temps de rétention de 14,1 min.

### 2) tert-butyl 15-[(tert-butoxycarbonyl)amino]-1-(9H-fluorèn-9-yl)-12-hydroxy-3-oxo-2,11,13-trioxa-4-aza-12-phosphahexadécan-16-oate 12-oxide

3 g de 9*H*-fluorèn-9-ylméthyl 6-(phosphonooxy)hexylcarbamate et 3,73 g de Boc-Serine-OtBu sont dissous dans 54 ml de pyridine. Une solution de 6,49 g de triisopropylbenzènesulfochloride dans 42 ml de pyridine est additionnée goutte à goutte. L'ensemble est agité à température ambiante 48 heures.

30 ml d'eau sont ajoutés et l'ensemble est agité pendant 4 heures puis est concentré sous vide. Le résidu obtenu est repris dans 100 ml d'ether éthylique.

L'insoluble est éliminé par filtration. Le filtrat est concentré sous vide et est ensuite purifié sur Silice (élution : CH₂Cl₂ 95 % - MeOH 5 %).
HPLC : Colonne X-TERRA C18 (5µm) 250x4,6 mm
- Détecteur : UV 201 nm
- Phase mobile :
   A : Solution de Carbonate d'ammonium pH = 9
   B: CH₃CN

| Temps (min.) | Débit (ml/min.) | % A | % B |
|---|---|---|---|
| 0 | 1 | 98 | 2 |
| 30 | 1 | 2 | 98 |

Le produit attendu a un temps de rétention de 20 min.

### 3) tert-butyl 3-{[[(6-aminohexyl)oxy](hydroxy)phosphoryl]oxy}-2-[(tert-butoxycarbonyl)amino]propanoate

3 g de *tert*-butyl 15-[(*tert*-butoxycarbonyl)amino]-1-(9*H*-fluorèn-9-yl)-12-hydroxy-3-oxo-2,11,13-trioxa-4-aza-12-phosphahexadecan-16-oate-12-oxide sont agités dans 0,62 ml de pipéridine et 3 ml d'eau pendant 12 heures à température ambiante. Le milieu réactionnel est filtré et les cristaux obtenus sont lavés 2 fois avec 50 ml d'acétonitrile puis est recristallisé dans 100 ml d'acétonitrile.
Spectre de masse : M/z = 441,3 (ES+) M théorique = 440

### 4) Acide 16-(tert-butoxycarbonyl)-13-hydroxy-20,20-diméthyl-13-oxido-4,18-dioxo-1-phosphono-12,14,19-trioxa-5,17-diaza-13-phosphahénicos-1-ylphosphonique

106 mg de *tert*-butyl 3-{[[(6-aminohexyl)oxy](hydroxy)phosphoryl]oxy}-2-[(*tert*-butoxycarbonyl)amino]propanoate en solution dans 2 ml d'eau sont introduits dans une solution de 50 mg du composé A de l'exemple 1 dans 4 ml d'eau. Le pH est ajusté à 6,2 et 60 mg de chlorhydrate de 1-(3-diméthylaminopropyl)-3-éthylcarbodiimide sont ajoutés. L'ensemble est agité 24 heures à température ambiante.

Le produit obtenu est purifié sur Silice 60 silanisé Merck (63-200 µm) avec une élution en gradient (eau/méthanol) avec un suivi par HPLC.

70 mg sont obtenus avec un rendement de 52 %.
Spectre de masse : M/z = 671,2 (ES+) M théorique = 670

### 5) Acide 17-amino-1,1,14-trihydroxy-5-oxo-2-phosphono-13,15-dioxa-6-aza-1λ⁵,14-diphosphaoctadécan-18-oïque 1,14-dioxide

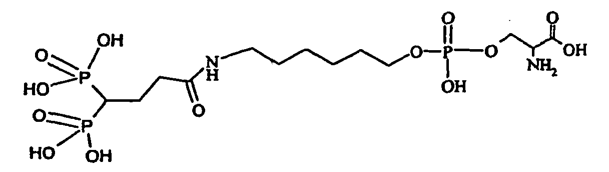

60 mg d'acide 16-(*tert*-butoxycarbonyl)-13-hydroxy-20,20-diméthyl-13-oxido-4,18-dioxo-1-phosphono-12,14,19-trioxa-5,17-diaza-13-phosphahénicos-1-yl-phosphonique sont mis en agitation dans 1 ml de TFA pendant 2 heures.

La solution est évaporée sous vide et 46 mg de produit sont obtenus avec un rendement quantitatif.
Spectre de masse : M/z = 515,1 (ES+) M théorique = 514

6) 31 mg d'acide 17-amino-1,1,14-trihydroxy-5-oxo-2-phosphono-13,15-dioxa-6-aza-1λ⁵,14-diphosphaoctadécan-18-oiique 1,14-dioxide en solution dans 10 ml d'eau est ajouté goutte à goutte à une solution de 1 ml de l'exemple 9 (ferrofluide acide) à 2,742 M/L dilué dans 100 ml d'eau. L'ensemble est agité pendant 20 minutes à température ambiante et le pH est ajusté à 7,2.

La solution obtenue est ultrafiltrée sur une cellule à agitation PALL^{®} ayant un seuil de coupure de 30 kD. 300 ml de filtrat sont éliminés pour obtenir une solution finale de 10 ml.

| | |
|---|---|
| [Fe] = 0,262 M/L | Taille PCS = 28,1 nm |
| Fe = 67,6 % Masse/Masse | |
| P = 1,9 % Masse/Masse | |
| C = 3,15 % Masse/Masse | |
| N = 0,57 % Masse/Masse | |

### Exemple 29 :

1,75 g du composé I de l'exemple 23 sont dissous dans 30 ml de l'exemple 12 à 0,285 M/L et le pH est ajusté à 6,5. 368 mg de chlorhydrate de 1-(3-diméthylaminopropyl)-3-éthylcarbodiimide sont ajoutés. L'ensemble est agité à température ambiante pendant 24 heures. La solution est ultrafiltrée sur une cellule PALL® à agitation ayant un seuil de coupure de 30 kD. 800 ml de filtrat sont éliminés. Le rétentat est ajusté à 15 ml.

| | |
|---|---|
| [Fe] = 0,496 M/L | Taille PCS = 27,1 nm |
| Fe = 59,2 % Masse/Masse | |
| P = 1,09 % Masse/Masse | |
| N = 0,32 % Masse/Masse | |
| C = 6,8 % Masse/Masse | |
| Taux de greffage [composé A / Fe] = 1,66 % mole/mole | |
| Taux de greffage [composé I / Fe] = 0,97 % mole/mole | |
| Taux de greffage [composé I / composé A] = 54 % | |

### Exemple 30 :

0,9 g du composé O-(2-Aminoéthyl)-O'-méthylpolyéthylène glycol 750 [Amino-PEG750 ; Fluka] sont dissous dans 22 ml de l'exemple 12 à 0,285 M/L et le pH est ajusté à 6,5. 280 mg de chlorhydrate de 1-(3-diméthylaminopropyl)-3-éthylcarbodiimide sont ajoutés. L'ensemble est agité à température ambiante pendant 24 heures. La solution est ultrafiltrée sur une cellule PALL® à agitation ayant un seuil de coupure de 30 kD. 800 ml de filtrat sont éliminés. Le rétentat est ajusté à 20 ml.

| | |
|---|---|
| [Fe] = 0,285 M/L | Taille PCS = 24,9 nm |
| Fe = 53,2 % Masse/Masse | |
| P = 1,30 % Masse/Masse | |
| N = 0,35 % Masse/Masse | |
| C = 5,87 % Masse/Masse | |
| Taux de greffage [composé A / Fe] = 2,2 % mole/mole | |
| Taux de greffage [composé Amino-PEG750 / Fe] = 1,33% mole/mole | |
| Taux de greffage [composé Amino-PEG750 / composé A] = 60,3 % | |

### Exemple 31 :

3,15 g du composé O-(2-aminoéthyl)-O'-méthylpolyéthylène glycol 2000 [Amino-PEG2000; Fluka] sont dissous dans 30 ml de l'exemple 12 à 0,285 M/L et le pH est ajusté à 6,2. 368 mg de chlorhydrate de 1-(3-diméthylaminopropyl)-3-éthylcarbodiimide sont ajoutés. L'ensemble est agité à température ambiante pendant 24 heures. La solution est ultrafiltrée sur une cellule PALL® à agitation ayant un seuil de coupure de 30 kD. 800 ml de filtrat sont éliminés. Le rétentat est ajusté à 25 ml.

| | |
|---|---|
| [Fe] = 0,290 M/L | Taille PCS = 32,2 nm |
| Fe = 46,3 % Masse/Masse | |
| P = 1,07 % Masse/Masse | |
| N = 0,3 % Masse/Masse | |
| C = 10,3 % Masse/Masse | |
| Taux de greffage [composé A / Fe] = 2,08 % mole/mole | |
| Taux de greffage [composé Amino-PEG2000 / Fe] = 1,07% mole/mole | |
| Taux de greffage [composé Amino-PEG2000 / composé A] = 50,4 % | |

### Exemple 32 :

180 mg du composé J de l'exemple 24 sont introduits dans 46 ml de l'exemple 12 (concentration : 0,285 mole/L en fer) et le pH est ajusté à 6,2.

50 mg de chlorhydrate de 1-(3-diméthylamino-propyl)-3-éthylcarbodiimide sont ajoutés à cette solution. L'ensemble est agité 8 heures à température ambiante. 50 mg de chlorhydrate de 1-(3-diméthylamino-propyl)-3-éthylcarbodiimide sont rajoutés au milieu réactionnel et l'agitation est maintenue 16 heures.

Le milieu réactionnel est directement ultrafiltré sur cellule à agitation PALL^{®} ayant un seuil de coupure de 30 kD.

Le rétentat est ajusté à 55 ml puis est filtré sur 0,22 µm.

| | |
|---|---|
| [Fe] : 0,273 M/L | Taille PCS = 72,4 nm |
| Fe = 62,4 % Masse/Masse | |
| P = 1,16 % Masse/Masse | |
| C = 2,31 % Masse/Masse | |
| N = 0,37 % Masse/Masse | |
| Taux de greffage [composé A / Fe] = 1,68 % mole/mole | |
| Taux de greffage [composé J / Fe] = 0,185 % mole/mole | |
| Taux de greffage [composé J / composé A] = 11,0 % | |

### Exemple 33 :

750 mg de O-(2-aminoéthyl)-O'-méthylpolyéthylène glycol 750 (Amino-PEG méthylester 750 ^{®} - Fluka) sont introduits dans 20 ml de l'exemple 32 à 0,273 M/L en Fer et le pH est ajusté à 6,2.

200 mg de chlorhydrate de 1-(3-diméthylamino-propyl)-3-éthycarbodiimide sont ajoutés à la solution précédente. L'ensemble est agité 8 heures à température ambiante. 200 mg de chlorhydrate de 1-(3-diméthylamino-propyl)-3-éthyl-carbodiimide sont alors rajoutés au milieu réactionnel et l'agitation est maintenue 16 heures.

Le milieu réactionnel est directement ultrafiltré sur cellule à agitation PALL^{®} ayant un seuil de coupure de 30 kD. Le rétentat est ajusté à 25 ml puis est filtré sur 0,22 µm.

| | |
|---|---|
| [Fe] : 0,268 M/L | Taille PCS = 69 nm |
| Fe = 59,7 % Masse/Masse | |
| P = 1,06 % Masse/Masse | |
| C = 6,3 % Masse/Masse | |
| N = 0,65 % Masse/Masse | |
| Taux de greffage [composé A / Fe] = 1,6 % mole/mole | |
| Taux de greffage [Amino-PEG 750 / Fe] = 1,9 % mole/mole | |
| Taux de greffage [composé J / Fe] = 0,19 % mole/mole | |

### Exemple 34 :

1,13 g de l'exemple 5 (composé E) sont introduits dans 20 ml de l'exemple 32 à 0,273 M/L en fer et le pH est ajusté à 6,2.

200 mg de chlorhydrate de 1-(3-diméthylamino-propyl)-3-éthylcarbodiimide sont ajoutés à cette solution. L'ensemble est agité 8 heures à température ambiante.

200 mg de chlorhydrate de 1-(3-diméthylamino-propyl)-3-éthylcarbodiimide sont alors rajoutés au milieu réactionnel et l'agitation est maintenue 16 heures.

Le milieu réactionnel est directement ultrafiltré sur cellule à agitation PALL^{®} ayant un seuil de coupure de 30 kD. Le rétentat est ajusté à 25 ml puis est filtré sur 0,22 µm.

| | |
|---|---|
| [Fe] : 0,188 M/L | Taille PCS = 79 nm |
| Fe = 55,1 % Masse/Masse | |
| P = 0,91 % Masse/Masse | |
| C = 7,21 % Masse/Masse | |
| N = 1,09 % Masse/Masse | |
| Br = 3,08 % Masse/Masse | |
| Taux de greffage [composé A / Fe] = 1,5 % mole/mole | |
| Taux de greffage [composé E / Fe] = 1,3 % mole/mole | |
| Taux de greffage [composé J / Fe] = 0,19 % mole/mole | |

### Exemple 35 :

0,29 g du composé K de l'exemple 2 sont introduits dans 74 ml de l'exemple 12 (concentration : 0,285 mole/L en fer) et le pH est ajusté à 6,2.

82 mg de chlorhydrate de 1-(3-diméthylamino-propyl)-3-éthylcarbodiimide sont ajoutés à cette solution et l'ensemble est agité 8 heures à température ambiante. Au bout de 8 heures, 82 mg de chlorhydrate de 1-(3-diméthylamino-propyl)-3-éthylcarbodümide sont alors rajoutés au milieu réactionnel et l'agitation est maintenue 16 heures.

Le milieu réactionnel est directement ultrafiltré sur cellule à agitation PALL^{®} ayant un seuil de coupure de 30 kD. Le rétentat est ajusté à 75 ml puis est filtré sur 0,22 µm.

| | |
|---|---|
| [Fe] : 0,273 M/L | Taille PCS = 60,8 nm |
| Fe = 63,1 % Masse/Masse | |
| P = 1,23 % Masse/Masse | |
| C = 4,1 % Masse/Masse | |
| N = 0,37 % Masse/Masse | |
| Taux de greffage [composé A / Fe] = 1,76 % mole/mole | |
| Taux de greffage [composé K / Fe] = 0,22 % mole/mole | |
| Taux de greffage [composé K / composé A] = 12,5 % | |

### Exemple 36 :

1 g de O-(2-aminoéhyl)-O'-méthylpolyéthlylène glycol 750 (Amino-PEG méthylester 750 ^{®}- Fluka) sont introduits dans 25 ml de l'exemple 35 (C= 0,273 M/L en fer) et le pH est ajusté à 6,2.

274 mg de chlorhydrate de 1-(3-diméthylamino-propyl)-3-éthylcarbodiimide sont ajoutés à cette solution. L'ensemble est agité 8 heures à température ambiante. Au bout de 8 heures, 274 mg de chlorhydrate de 1-(3-diméthylamino-propyl)-3-éthylcarbodiimide sont alors rajoutés au milieu réactionnel et l'agitation est maintenue 16 heures.

Le milieu réactionnel est directement ultrafiltré sur cellule à agitation PALL^{®} ayant un seuil de coupure de 30 kD. Le rétentat est ajusté à 26 ml puis est filtré sur 0,22 µm.

| | |
|---|---|
| [Fe] : 0,261 M/L | Taille PCS = 59 nm |
| Fe = 62,4 % Masse/Masse | |
| P = 1,13 % Masse/Masse | |
| C = 7,56 % Masse/Masse | |
| N = 0,8 % Masse/Masse | |
| Taux de greffage [composé A / Fe] = 1,63 % mole/mole | |
| Taux de greffage [Amino-PEG 750 / Fe] = 1,59 % mole/mole | |
| Taux de greffage [composé K / Fe] = 0,22 % mole/mole | |

### Exemple 37 :

1,61 g de l'exemple 5 (composé E) sont introduits dans 25 ml de l'exemple 35 (C=0,273 M/L en fer) et le pH est ajusté à 6,2.

274 mg de chlorhydrate de 1-(3-diméthylamino-propyl)-3-éthylcarbodiimide sont ajoutés à cette solution. L'ensemble est agité 8 heures à température ambiante. Au bout de 8 heures, 274 mg de chlorhydrate de 1-(3-diméthylamino-propyl)-3-éthylcarbodiimide sont alors rajoutés au milieu réactionnel et l'agitation est maintenue 16 heures.

Le milieu réactionnel est directement ultrafiltré sur cellule à agitation PALL^{®} ayant un seuil de coupure de 30 kD. Le rétentat est ajusté à 28 ml puis est filtré sur 0,22 µm.

| | |
|---|---|
| [Fe] : 0,240 M/L | Taille PCS = 65,6 nm |
| Fe = 61,5 % Masse/Masse | |
| P = 1,02 % Masse/Masse | |
| C = 8,0 % Masse/Masse | |
| N = 1,21 % Masse/Masse | |
| Br = 3,8 % Masse/Masse | |
| Taux de greffage [composé A / Fe] = 1,5 % mole/mole | |
| Taux de greffage [composé E / Fe] = 1,4 % mole/mole | |
| Taux de greffage [composé K / Fe] = 0,22 % mole/mole | |

### Tests d'affinité des particules magnétiques acides (p) complexées par des composés de formule (I) et couplées avec des biovecteurs, pour certaines cibles biologiques.

### 1/ Test d'affinité de l'exemple 17 pour la Folate Binding Protein (FBP) par la technique BiaCore

### Réactifs

| Produits | Fournisseur | Référence |
|---|---|---|
| Acide folique | Avogado | 14300 |
| FBP | Sigma | F-0504 |
| Chips CM5 | BIAcore. | Lots 1121065 et 11210007 |

Tampon HBS : Hépès 10mM pH7,4, NaCl 0,15 M, EDTA 4,3mM et NP20 à 0,005%
Tampon PGM : KH₂PO₄ 100mM pH 7, glycérol 10% et β-mercaptoéthanol 4mM.

L'immobilisation de la FBP a été réalisée à 1 mg/ml dans le tampon PGM selon les protocoles prescrits par BIAcore pour le couplage aux amines. Après, les groupements carboxylés actifs restants sont saturés par 1M d'éthanolamine pH=8. La quantité fixée de FBP correspond approximativement à 1,5 ng/mm².

L'exemple 17 était à une concentration de 0,170 mole/L en fer ce qui correspond à 1,9.10⁻⁵ mole de particules/L (en considérant un cristal de 7,5 nm soit 8900 atomes de fer/particule).

La fixation du folate a été suivie à quatre concentrations différentes (125, 250, 500 et 1000 µM) alors que celle de l'exemple 17 a été réalisée à 10 µM et à 10 nM (exprimée en mole de particules/L). L'association et la dissociation ont été étudiées avec un flux à 25 µl/min. Les phases d'association et de dissociation sont de 5 minutes.

### Résultats

| Produits | Vitesse d'association ka | Vitesse de dissociation kd | Constante d'affinité KD |
|---|---|---|---|
| Acide folique | 10³ M⁻¹ s⁻¹ | 1.6 10⁻³ s⁻¹ | 1,6 µM |
| Exemple 17 | > 10⁶ M⁻¹ s⁻¹ | < 10⁻⁶ s⁻¹ | < 1 pM |

Ces résultats démontrent que l'affinité de l'exemple 17 pour la Folate Binding Protein (FBP) est très élevée avec une constante d'affinité KD inférieure à la picomolaire (exprimés en mole de particule/L).

Sachant que le récepteur à l'acide folique (représenté ici par la FBP) est très fortement sur-exprimé au niveau de certaines cellules tumorales (cancer de l'ovaire, des poumons, du colon et du sein) et dans des sites inflammatoires (en particulier dans la polyarthrite rhumatoïde), l'exemple 17 peut avantageusement être utilisé comme agent de contraste IRM spécifique en oncologie et dans les maladies inflammatoires.

### 2/ Activité inhibitrice de l'exemple 27 pour des métalloprotéases MMP-1 et MMP-3 humaines.

L'exemple 27 est couvert à sa surface par un biovecteur (exemple 22 : composé H) dérivé de l'Ilomastat, pseudo-peptide reconnu comme étant un inhibiteur puissant de métalloprotéases à Zinc (Matrix metalloproteinase : MMP).

L'activité inhibitrice de l'exemple 27 sur les métalloprotéases de la matrice MMP-1 et MMP-3 humaines a été évaluée *in vitro.* Elle a été comparée à celle de l'exemple 12 (particule non fonctionnalisée par le composé H) et à celle du peptide de référence, l'Ilomastat. Le protocole de mesure de l'effet inhibiteur des différents produits sur MMP-1 a été le suivant :
- Principe : évaluation de l'effet inhibiteur du produit testé sur l'activité d'une MMP-1 humaine (isolée de fibroblastes humains de synovie rhumatoïde), quantifié par fluorimétrie (λex=340 nm, λem=440 nm ; mesure de la formation de Cys(Me)-His-Ala-Lys-(n-Me-Abz)-NH2 par clivage du substrat DNP-Pro-Cha-Gly-Cys(Me)-His-Ala-Lys-(n-Me-Abz)-NH2) ; protocole décrit dans la référence bibliographique Bickett and al. (1993) Anal. Biochem. ; 212 :58.

Le produit de référence utilisé dans ce test a été le GM6001 (Ilomastat, Galardin IC₅₀ =1,9.10⁻⁹ M).

Plus précisément, le produit à tester, ou le produit de référence ou de l'eau, a été ajouté à une solution tampon à pH=7, contenant 7 nM en MMP-1. L'intensité en fluorescence de ce milieu a immédiatement été évaluée à λex=340 nm et λem=440 nm au moyen d'un lecteur de plaques (GeminiXS, Molecular Devices). Cette mesure à t=0 sert à vérifier toute interférence éventuelle d'un produit avec la détection fluorescente. La réaction enzymatique a ensuite été initiée en ajoutant le substrat DNP-Pro-Cha-Gly-Cys(Me)-His-Ala-Lys-(n-Me-Abz)-NH2 à une concentration de 10 µM. Après une incubation de 40 min à 37°C, la fluorescence a de nouveau été évaluée dans les mêmes conditions que précédemment (t=40 min). L'activité enzymatique a été déterminée en soustrayant le signal mesuré à t=0 du signal mesuré à t=40 min. Le résultat final est exprimé en pourcentage d'inhibition de l'activité enzymatique.

Le protocole de mesure de l'effet inhibiteur des différents produits sur MMP-3 a été le suivant :
- Principe : évaluation de l'effet inhibiteur du produit testé sur l'activité d'une MMP-3 humaine (utilisation d'une enzyme recombinante exprimée par la lignée cellulaire sF9), quantification par fluorimétrie (λex=340 nm, λem=405 nm ; mesure de la formation de Mca-Arg-Pro-Lys-Pro-Tyr-Ala par clivage du substrat Mca-Arg-Pro-Lys-Pro-Tyr-Ala-Nva-Trp-Met-Lys(DNP)-NH2 (NFF2) - protocole décrit dans la référence: Nagase et al. (1994) J. Biol. Chem. ; 269 :20952.

Le produit de référence utilisé dans ce test a été le GM6001 (Ilomastat, Galardin IC₅₀ =1,3.10⁻⁷ M).

Plus précisément, le produit à tester, ou le produit de référence ou de l'eau, a été ajouté à une solution tampon à pH=6, contenant 6 nM en MMP-3. La fluorescence de ce milieu a été évaluée à λex=340 nm et λem=405 nm au moyen d'un lecteur de plaques (GeminiXS, Molecular Devices), après une pré-incubation de 30 min à 37°C (t=0). Cette mesure à t=0 sert à vérifier toute interférence éventuelle d'un produit avec la détection fluorescente. La réaction enzymatique a ensuite été initiée en ajoutant 10 µM du substrat NFF-2. Après une incubation de 90 min à 37°C, la fluorescence a à nouveau été évaluée dans les mêmes conditions que précédemment (t=90 min). L'activité enzymatique a ensuite été déterminée en soustrayant le signal mesuré à t=0 du signal mesuré à t=90 min.

Le résultat final est exprimé en pourcentage d'inhibition de l'activité enzymatique.

Les résultats d'inhibition des MMP-1 et -3 humaines par l'exemple 27 sont représentés dans le tableau 1.

**Tableau 1**

| MMP | Concentration en fer [M] | Concentration en biovecteur : composé H [M] | % inhibition de la valeur contrôle |
|---|---|---|---|
| MMP-1 | 1,03 x 10⁻⁸ | 0,86 x 10⁻¹⁰ | 9 |
| | 1,03 x 10⁻⁶ | 0,86 x 10⁻⁸ | 86 |
| | 1,03 x 10⁻⁴ | 0,86 x 10⁻⁶ | 103 |
| MMP-3 | 1,03 x 10⁻⁸ | 0,86 x 10⁻¹⁰ | 0 |
| | 1,03 x 10⁻⁶ | 0,86 x 10⁻⁸ | -1 |
| | 1,03 x 10⁻⁴ | 0,86 x 10⁻⁶ | 68 |

L'exemple 27 inhibe MMP-1 entre 0,87 x 10⁻¹⁰ et 0,87 x 10⁻⁸ M (exprimé en concentration de composé H), ce qui est en accord avec la valeur expérimentale obtenue pour le peptide de référence - Ilomastat -(IC50 = 1,9 x 10⁻⁹ M)_{.}

L'exemple 27 inhibe MMP-3 entre 0,87 x 10⁻⁸ et 0,87 x 10⁻⁶ M (exprimé en concentration de composé H), ce qui est en accord avec la valeur expérimentale obtenue pour le peptide de référence - Ilamastat- (IC50 = 1,3 x 10⁻⁷ M).

Les résultats obtenus avec l'exemple 27 sur les MMP-1 et MMP-3 montrent que les particules d'oxyde de fer préparées par le demandeur se comportent de manière très similaire au peptide de référence (Ilomastat). Le composé de l'exemple 27 peut être utilisé comme inhibiteur à large spectre vis à vis des métalloprotéases (MMP-1, MMP2, MMP-3, MMP-7, MMP-9), et ainsi comme agent de contraste IRM spécifique très utile pour imager des zones pathologiques dans lesquelles ces différentes MMP sont sur-exprimées.

## Revendications

1. Composition comprenant des particules magnétiques acides (p) à base d'un composé du fer, lesdites particules magnétiques acides (p) étant complexée par un ou plusieurs composés gem-bisphosphonate, de formule I :
X-L-CH (PO₃H₂)₂ (I)
dans laquelle :
- L représente un groupement organique reliant la fonction X à la fonction gem-bisphosphonate -CH(PO₃H₂)₂ ; choisi parmi
un groupe -(CH₂)ₚ- où p est un entier de 1 à 5 ;
un groupe phénylène éventuellement substitué, les groupes X et gem-bisphosphonates pouvant être en position ortho, méta, para ;
une chaîne aliphatique linéaire comprenant de 1 à 6 atomes de carbone substituée par un groupement aromatique ;
un groupe L₁-NHCO-L₂ où L₁ et L₂ sont soit identiques soit différents et représentent un groupe aliphatique linéaire comprenant de 1 à 6 atomes de carbone ou une chaîne aliphatique linéaire comprenant de 1 à 6 atomes de carbone substituée par un groupement aromatique,
lesdits groupes pouvant être éventuellement substitués par un groupe méthyle, hydroxy, méthoxy, acétoxy, amido ou un atome de chlore, d'iode ou de brome,
- X représente une fonction chimique susceptible de former une liaison covalente avec un biovecteur choisie parmi -COOH, -NH₂, -NCS, -NH-NH₂, -CHO, maléimidyle, alkylpyrocarbonyle, acylazidyle, iminocarbonate, vinylsulfuryle, pyridyl-disulfuryle, haloacétyle, dichlorotriazinyle, halogène,
lesdites fonctions X desdites particules étant éventuellement couplées en tout ou partie à un biovecteur.

2. Composition selon la revendication 1, dans laquelle tout ou partie des fonctions X sont couplées à un biovecteur.

3. Composition selon la revendication 1, dans laquelle L représente un groupe -(CH₂)ₘ-CONH-(CH₂)_{n,} où m et n représentent un entier de 0 à 5.

4. Composition selon la revendication 1, dans laquelle X représente -COOH ou -NH₂.

5. Composition selon les revendications 1 ou 4, dans laquelle tout ou partie des composés de formule (I) a la formule (la) suivante :
HOOC-(CH₂)₂-CH(PO₃H₂)₂ (Ia)

6. Composition selon l'une quelconque des revendications précédentes, dans laquelle les particules (p) sont, en moyenne, complexées sur au moins 90% de leurs sites protonés par des composés de formule (I).

7. Composition selon l'une des revendications précédentes, dans laquelle les sites protonés sont complexés par au moins deux composés gem-bisphosphonates de formule (I) de structure différente.

8. Composition selon l'une quelconque des revendications précédentes, dans laquelle les sites protonés sont complexés par au moins deux composés de formulé (I) de structure identique.

9. Composition selon l'une quelconque des revendications précédentes, dans laquelle les particules magnétiques (p) sont composées en tout ou partie d'hydroxyde de fer, d'oxyde de fer hydraté, de ferrites, d'oxydes de fer mixtes ou d'un mélange de ceux-ci.

10. Composition selon la revendication 9, dans laquelle les particules magnétiques (p) sont composées en tout ou partie d'une ferrite.

11. Composition selon la revendication 10, dans laquelle la ferrite est la maghémite ou la magnétite ou un mélange de celles-ci.

12. Composition selon l'une des revendications précédentes, dans laquelle les particules (p) sont superparamagnétiques.

13. Composition selon l'une quelconque des revendications précédentes, dans laquelle entre 1 et 70%, de préférence entre 1 et 50 % des fonctions X des composés de formule (I) sont couplées à un biovecteur.

14. Composition selon l'une quelconque des revendications précédentes, dans laquelle les fonctions X des composés de formule (I) sont couplées à au moins deux biovecteurs différents.

15. Composition selon l'une quelconque des revendications précédentes, dans laquelle le biovecteur est choisi parmi les protéines, éventuellement recombinantes ou mutées, les glycoprotéines, les lectines, la biotine, les vitamines, les phospholipides, les dérivés de l'acide folique, les anticorps ou fragments d'anticorps, les peptides et leurs dérivés, les mono- ou polysaccharides, l'avidine, la streptavidine, les inhibiteurs ou substrats de récepteurs, les stéroïdes et leurs analogues, les oligonucléotides, tes séquences d'acide ribonucléique, les séquences d'acide désoxyribonucléique, les hormones ou substances analogues aux hormones, les acides aminés et dérivés, les molécules organiques à activité pharmacologique, les aminoalcools, les agents de ciblage d'intégrines, les agents de ciblage de MMP.

16. Composition selon la revendication 15, dans laquelle le biovecteur représente un dérivé de peptide, de préférence un agent de ciblage d'intégrine ou de MMP.

17. Composition selon la revendication 15, dans laquelle le biovecteur représente un dérivé de l'acide folique ou antifolique.

18. Composition selon la revendication 15, dans laquelle le biovecteur représente un dérivé de phospholipide, en particulier un dérivé de phosphatidylsérine, de phosphatidyléthanolamine, de phosphatidylcholine, de phosphatidylinositol, de sphingomyéline ou les dérivés de céramides.

19. Composition selon la revendication 15, dans laquelle le biovecteur représente une protéine, éventuellement recombinante ou mutée.

20. Composition selon la revendication 15, dans laquelle le biovecteur représente un anticorps ou un fragment d'anticorps éventuellement fonctionnalisés.

21. Composition selon la revendication 15, dans laquelle le biovecteur représente un pharmacophore éventuellement fonctionnalisé.

22. Composition selon la revendication 15, dans laquelle le biovecteur est choisi parmi les aminoalcools de formule générale (II) 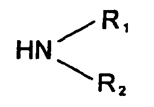 dans laquelle R₁ et R₂ sont identiques ou différents et représentent une chaîne hydrocarbonée aliphatique comportant de 2 à 6 atomes de carbone, substituée de préférence par 6 à 10 groupements hydroxyles, ou bien par 4 à 8 groupements hydroxyles dans le cas où R₁ et/ou R₂ est interrompu par un atome d'oxygène.

23. Composition selon la revendication 15, dans laquelle le biovecteur est choisi parmi les aminopolyéthylèneglycols.

24. Composition selon la revendication 22, dans laquelle R₁ représente un groupe -(CH₂)-(CHOH)₄-CH₂OH ou -(CH₂)-CHOH-CH₂OH et R₂ un groupe -CH₂-(CHOH)₄-CH₂OH.

25. Composition selon la revendication 1, dans laquelle le biovecteur est le composé N-[(2,3-dihydroxypropyl)(2,3,4,5,6-pentahydroxyhexyl)]2,4,6-tribromo 5-(glycylamino)isophtalamide ou le composé N-N'-[bis(2,3,4,5,6-pentahydroxyhexyl)]2,4,6-tribromo 5-(glycylamino)isophtalamide.

26. Composition selon l'une quelconque des revendications précédentes, dans laquelle les fonctions X forment une liaison covalente avec le biovecteur de type -CONH-, -COO-, -NHCO-, -OCO-, -NH-CS-NH-, -C-S-, -N-NH-CO-, -CO-NH-N-, -CH₂-NH-, -N-CH₂- , -N-CS-N-, -CO-CH₂-S-, -N-CO-CH₂-S-, -N-CO-CH₂-CH₂-S, -CH=NH-NH-, -NH-NH=CH-, -CH=N-O-, -O-N=CH- ainsi que les formules suivantes

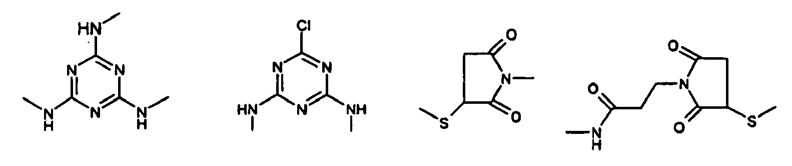

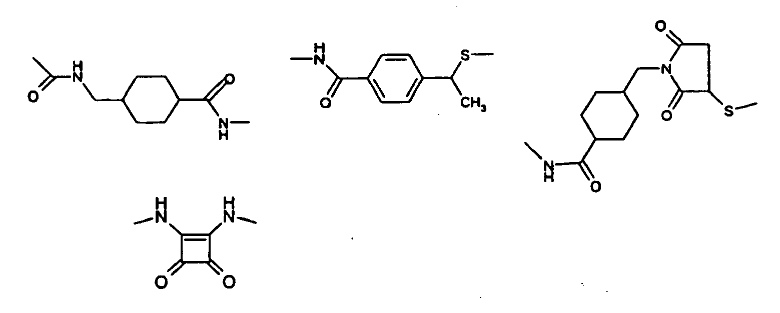

27. Composition selon l'une des revendications précédentes, dans laquelle les particules complexées et couplées au(x) biovecteur(s) ont un diamètre hydrodynamique global compris entre 5 nm et 200 nm, de préférence compris entre 5 et 60 nm.

28. Composition selon l'une quelconque des revendications précédentes, dans laquelle la concentration des particules (p) dans la suspension est comprise entre 2 µmoles L⁻¹ et 4 moles L⁻¹ , exprimée en nombre de moles d'ions métalliques total.

29. Composition selon l'une quelconque des revendications précédentes, comprenant un ou plusieurs véhicule(s) et/ou additif(s) pharmaceutiquement acceptables.

30. Composition selon la revendication 29, **caractérisée en ce qu'**elle est stérile.

31. Procédé de préparation d'une composition selon les revendications 1 à 30, ledit procédé comprenant les étapes successives consistant à :
(i) mettre en contact une solution acide de particules magnétiques acides (p) à base d'un composé de fer, avec des composés de formule (I) tels que définis dans la revendication 1 en quantité suffisante et récupération des particules complexées obtenues; le cas échéant,
ii) réaliser le couplage de tout ou partie des fonctions X des composés de formule (I) complexés à la surface des particules magnétiques (p) avec des biovecteurs.

32. Procédé selon la revendication 31, dans lequel les particules magnétiques (p) mises en oeuvre à l'étape (i) sont obtenues selon un procédé comprenant les étapes consistant à :
a) préparer une solution colloïdale de ferrofluide acide ;
b) traiter la solution obtenue à l'étape a) par une solution d'acide nitrique ;
c) isoler le floculat obtenu à l'étape b) ; et
d) réaliser une peptisation.

33. Produit de contraste pour l'imagerie par résonance magnétique comprenant des particules magnétiques éventuellement couplées à des biovecteurs telles que définies dans les revendications 1 à 30, éventuellement associées à un véhicule pharmaceutiquement acceptable.

34. Utilisation des compositions selon les revendications 1 à 30 pour la fabrication d'un agent de contraste utile pour l'Imagerie par Résonance Magnétique.

35. Utilisation des compositions selon les revendications 1 à 30 pour la fabrication d'un agent de contraste utile pour la médecine nucléaire.

36. Utilisation des compositions selon les revendications 2 à 30, dans laquelle le biovecteur est un ligand spécifique d'un récepteur biologique, pour la fabrication d'un agent de contraste utile pour la caractérisation et/ou le suivi thérapeutique, notamment dans les maladies cardiovasculaires, les cancers ou les maladies inflammatoires et dégénératives.

37. Utilisation des compositions selon les revendications 2 à 30, dans laquelle le biovecteur est un ligand spécifique d'un récepteur biologique, pour la différenciation et/ou la séparation *in vitro,* au sein d'un mélange, de composants portant une cible dudit biovecteur et de composants ne portant pas ladite cible.

38. Utilisation des particules magnétiques couplées à des biovecteurs selon les revendications 2 à 30, dans laquelle le biovecteur des particules magnétiques (p) possède des propriétés pharmacologiques et/ou cytotoxiques, ledit biovecteur pouvant éventuellement être libéré sous l'action d'un champ radiofréquence dans la zone ciblée.

39. Utilisation des compositions selon les revendications 2 à 30, dans laquelle le biovecteur est un ligand spécifique d'un récepteur biologique, pour le traitement par hyperthermie de territoires pathologiques.

40. Utilisation des compositions selon les revendications 2 à 30 pour le marquage cellulaire, et en particulier le marquage des cellules souches.

## Claims

1. A composition comprising acid magnetic particles (p) based on an iron compound, said acid magnetic particles (p) being complexed by one or more gem-bisphosphonate compounds, of formula I:
X-L-CH(PO₃H₂)₂ (I)
in which :
• L represents an organic group connecting the X group to the gem-bisphosphonate group -CH(PO₃H₂)₂ ; chosen among :
- a group -(CH₂)ₚ, where p is an integer from 1 to 5
- an optionally substituted phenylene group, it being possible for the X and gem-bisphosphonate groups to be in the ortho, meta or para position
- a linear aliphatic group comprising 1 to 6 carbon atoms substituted by an aromatic group
- a group L₁-CONH-L₂, where L₁ and L₂ are either identical or different and represent a linear aliphatic group comprising 1 to 6 carbon atoms or a linear aliphatic group comprising 1 to 6 carbon atoms substituted by an aromatic group
it being optionally possible for said groups to be substituted by methyl, hydroxyl, methoxy, acetoxy, amido group or a chlorine, iodine or bromine atom.
• X represents a chemical group capable of forming a covalent link with a biovector selected from -COOH, -NH₂, -NCS, -NH-NH₂, -CHO, maleimidyl, alkylpyrocarbonyl, acylazidyl, iminocarbonate, vinylsulfuryl, pyridyldisulfuryl, haloacetyl, dichlorotriazinyl and halogen;
all or some of said X groups of said particles optionally being coupled to a biovector.

2. The composition as claimed in claim 1, in which all or some of the X groups are coupled to a biovector.

3. The composition as claimed in claim 1, in which L represents a group -(CH₂)ₘ-CONH-(CH₂)ₙ where m and n represent an integer from 0 to 5.

4. The composition as claimed in claim 1, in which X represents -COOH or -NH₂.

5. The composition as claimed in claim 1 or 4, in which all or some of the compounds of formula (I) have the formula (Ia) below:
HOOC-(CH₂)₂-CH(PO₃H₂)₂ (Ia)

6. The composition as claimed in any of the previous claims, in which the particles (p) are, on average, complexed on at least 90% of their protonated sites by compounds of formula (I).

7. The composition as claimed in as claimed in any of the previous claims, in which the protonated sites are complexed by at least two gem-bisphosphonate compounds of formula (I) having a different structure.

8. The composition as claimed in as claimed in any of the previous claims, in which the protonated sites are complexed by at least two compounds of formula (I) having an identical structure.

9. The composition as claimed in any of the previous claims, in which all or some of the magnetic particles (p) are composed of iron hydroxide, of iron hydroxide hydrate, of ferrite, of mixed iron oxides or of a mixture thereof.

10. The composition as claimed in claim 9, in which all or some of the magnetic particles (p) are composed of a ferrite.

11. The composition as claimed in claim 10, in which the ferrite is maghemite or magnetite or a mixture thereof.

12. The composition as claimed in any of the previous claims, in which the particles (p) are superparamagnetic.

13. The composition as claimed in any of the previous claims, in which between 1 and 70%, preferably between 1 and 50% of the X groups of compounds of formula (I) are coupled to a biovector.

14. The composition as claimed in any of the previous claims, in which the X groups of compounds of formula (I) are coupled to at least two different biovectors.

15. The composition as claimed in any of the previous claims, in which the biovector is selected from proteins, optionally recombinant or mutated, glycoproteins, lectins, biotin, vitamins, phospholipids, folic acid derivatives, antibodies or antibody fragments, peptides and derivatives thereof, monosaccharides or polysaccharides, avidin, streptavidin, receptor substrates or inhibitors, steroids and analogs thereof, oligonucleotides, ribonucleic acid sequences, deoxyribonucleic acid sequences, hormones or hormone-like substances, amino acids and derivatives, organic molecules with pharmacological activity, amino alcohols, integrin-targeting agents and MMP-targeting agents.

16. The composition as claimed in claim 15, in which the biovector represents a peptide derivative, preferably an integrin-targeting agent or a MMP-targeting agent.

17. The composition as claimed in claim 15, in which the biovector represents an antifolic or folic acid derivative.

18. The composition as claimed in claim 15, in which the biovector represents a phospholipid derivative, in particular a derivative of phosphatidylserine, of phosphatidylethanolamine, of phosphatidylcholine, of phosphatidylinositol or of sphingomyelin or ceramide derivatives.

19. The composition as claimed in claim 15, in which the biovector represents a protein, optionally recombinant or mutated.

20. The composition as claimed in claim 15, in which the biovector represents an antibody or an antibody fragment which is optionally functionalized.

21. The composition as claimed in claim 15, in which the biovector represents an optionally functionalized pharmacophore.

22. The composition as claimed in claim 15, in which the biovector is selected from the amino alcohols of formula (II) 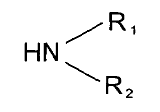 in which R₁ and R₂ are identical or different and represent an aliphatic hydrocarbon chain comprising from 2 to 6 carbon atoms, preferably substituted by 6 to 10 hydroxyl groups, or else by 4 to 8 hydroxyl groups when R₁ and/or R₂ is interrupted by an oxygen atom.

23. The composition as claimed in claim 15, in which the biovector is selected from aminopolyethylene glycols.

24. The composition as claimed in claim 22, in which R₁ represents a group -(CH₂)-(CHOH)₄-CH₂OH or -(CH₂)-CHOH-CH₂OH and R₂ represents a group -CH₂-(CHOH)₄-CH₂OH.

25. The composition as claimed in claim 1, in which the biovector is the compound N-[(2,3-dihydroxypropyl)-(2,3,4,5,6-pentahydroxyhexyl)2,4,6-tribromo-5-(glycylamino)isophthalamide or the compound N-N'-[bis(2,3,4,5,6-pentahydroxyhexyl)]2,4,6-tribromo-5-(glycylamino) isophthalamide.

26. The composition as claimed in any of the previous claims, in which the X groups form a covalent bond with the biovector, of type -CONH-, -COO-, - NHCO-, -OCO, -NH-CS-NH-, -C-S-, -N-NH-CO-, -CO-NH-N-, -CH₂-NH-, -N-CH₂-, -N-CS-N-, -CO-CH₂-S-, -N-CO-CH₂-S-, -N-CO-CH₂-CH₂-S, -CH=NH-NH-, -NH-NH=CH-, -CH=N-O- or -O-N=CH-, and also the formulae below

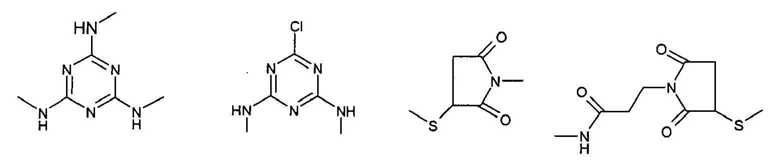

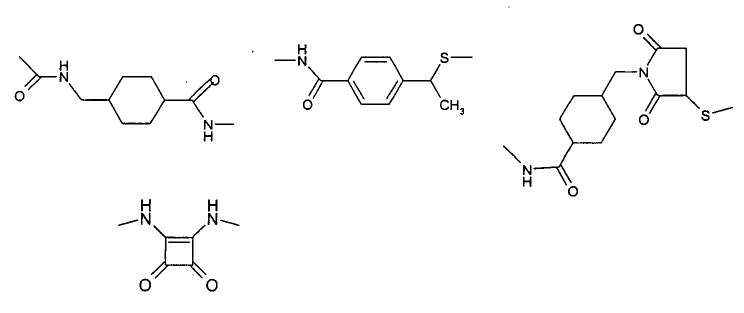

27. The composition as claimed in any of the previous claims, in which the particles complexed and coupled to the biovector(s) have an overall hydrodynamic diameter of between 5 nm and 200 nm, preferably between 5 and 60 nm.

28. The composition as claimed in any of the previous claims, in which the concentration of the particles (p) in the composition is between 2 µmol L⁻¹ and 4 mol L⁻¹, expressed as total number of moles of metal ions.

29. The composition as claimed in any of the previous claims, comprising one or more pharmaceutically acceptable vehicle(s) and/or additive(s).

30. The composition as claimed in claim 30, which is sterile.

31. A process for the preparation of a composition as claimed in claim 1 to 30, said process comprising the successive steps consisting in:
(i) bringing an acid solution of acid magnetic particles (p) based on an iron compound into contact with compounds of formula (I) as defined in claim 1 in sufficient amount, and recovering the complexed particles obtained;
where appropriate,
ii) coupling all or some of the X groups of the compounds of formula (I) complexed at the surface of the magnetic particles (p) with biovectors.

32. The process as claimed in claim 31, in which the magnetic particles (p) used in step (i) are obtained according to a process comprising the steps consisting in:
a) preparing a colloidal solution of acid ferrofluid;
b) treating the solution obtained in step a) with a nitric acid solution;
c) isolating the flocculate obtained in step b); and
d) carrying out a peptization.

33. A contrast product for magnetic resonance imaging, comprising magnetic particles optionally coupled to biovectors as defined in the claims 1 to 30, optionally combined with a suitably acceptable vehicle.

34. Use of compositions coupled to biovectors according to claims 1 to 30 for the preparation of a contrast agent for Magnetic Resonance imaging.

35. Use of compositions according to claims 1 to 30 for the preparation of a contrast agent for nuclear medicine.

36. Use of compositions according to claims 2 to 30, in which the biovector is a ligand specific of a biological receptor, for the preparation of a contrast agent for the characterization and/or the monitoring of a pathology, notably for the cardiovascular diseases, cancers, and inflammatory and degenerative diseases.

37. Use of compositions according to claims 2 to 30, in which the biovector is a ligand specific for a biological receptor, for the differentiation and/or the separation *in vitro,* within a mixture, of components carrying a target of said biovector and of components not carrying said target.

38. Use of compositions coupled to biovectors according to claims 2 to 30, in which the biovector of the magnetic particles (p) has pharmacological and/or cytotoxic properties, said biovector being able to be released under the action of a radiofrequency field in a targeted area.

39. Use of compositions according to claims 2 to 30, in which the biovector is a ligand specific for a biological receptor, for the treatment by hyperthermia of pathological areas.

40. Use of compositions according to claims 2 to 30, for cellular labelling, and in particular for labelling stem cells.

## Patentansprüche

1. Zusammensetzung, umfassend saure magnetische Partikel (p) auf Basis einer Eisenverbindung, wobei die sauren magnetischen Partikel (p) mit einer oder mehreren gem-Bisphosphonat-Verbindungen der Formel I:
X-L-CH(PO₃H₂)₂ (I)
komplexiert sind, worin:
- L für eine organische Gruppe steht, welche die *gem-*Bisphosphonat-Funktion -CH(PO₃H₂)₂ an die Funktion X bindet; ausgewählt aus
einer Gruppe -(CH₂)ₚ-, worin p eine ganze Zahl von 1 bis 5 ist;
einer gegebenenfalls substituierten Phenylengruppe, wobei die X- und gem-Bisphosphonat-Gruppen in ortho-, meta- oder para-Position stehen können;
einer geraden aliphatischen Kette mit 1 bis 6 Kohlenstoffatomen, die mit einer aromatischen Gruppe substituiert ist;
einer Gruppe L₁-NHCO-L₂, worin L₁ und L₂ gleich oder verschieden sind und eine gerade aliphatische Gruppe mit 1 bis 6 Kohlenstoffatomen oder eine gerade aliphatischen Kette mit 1 bis 6 Kohlenstoffatomen, die mit einer aromatischen Gruppe substituiert ist, darstellen,
wobei diese Gruppen gegebenenfalls mit einer Methyl-, Hydroxy-, Methoxy-, Acetoxy-, Amidogruppe oder einem Chlor-, Iod- oder Bromatom substituiert sind,
- X. eine chemische Funktion darstellt, die eine kovalente Bindung mit einem Biovektor eingehen kann, ausgewählt aus -COOH, -NH₂, -NCS, -NH-NH₂, -CHO, Maleinimidyl, Alkylpyrocarbonyl, Acylazidyl, Iminocarbonat, Vinylsulfuryl, Pyridyldisulfuryl, Halogenacetyl, Dichlortriazinyl, Halogen,
wobei die Funktionen X der Partikel gegebenenfalls ganz oder teilweise an einen Biovektor gekuppelt sind.

2. Zusammensetzung nach Anspruch 1, wobei die ganzen oder ein Teil der Funktionen X an einen Biovektor gekuppelt sind.

3. Zusammensetzung nach Anspruch 1, wobei L eine Gruppe -(CH₂)ₘ-CONH-(CH₂)ₙ darstellt, wobei m und n eine ganze Zahl von 0 bis 5 sind.

4. Zusammensetzung nach Anspruch 1, wobei X für -COOH oder -NH₂ steht.

5. Zusammensetzung nach den Ansprüchen 1 oder 4, wobei die gesamten oder ein Teil der Verbindungen der Formel (I) die folgende Formel (Ia) haben:
HOOC-(CH₂)₂-CH(PO₃H₂)₂ (Ia)

6. Zusammensetzung nach einem der vorhergehenden Ansprüche, wobei die Partikel (p) im Durchschnitt an mindestens 90% ihrer protonierten Stellen durch Verbindungen der Formel (I) komplexiert sind.

7. Zusammensetzung nach einem der vorhergehenden Ansprüche, wobei die protonierten Stellen durch mindestens zwei gem-Bisphosphonat-Verbindungen der Formel (I) mit unterschiedlicher Struktur komplexiert sind.

8. Zusammensetzung nach einem der vorhergehenden Ansprüche, wobei die protonierten Stellen durch mindestens.zwei Verbindungen der Formel (I) mit identischer Struktur komplexiert sind.

9. Zusammensetzung nach einem der vorhergehenden Ansprüche, wobei die Magnetpartikel (p) ganz oder teilweise aus Eisenhydroxid, Eisenoxidhydrat, Ferriten, gemischten Eisenoxiden oder einem Gemisch von diesen bestehen.

10. Zusammensetzung nach Anspruch 9, wobei die Magnetpartikel (p) ganz oder teilweise aus einem Ferrit bestehen.

11. Zusammensetzung nach Anspruch 10, wobei es sich bei dem Ferrit um Maghämit, Magnetit oder ein Gemisch von diesen handelt.

12. Zusammensetzung nach einem der vorhergehenden Ansprüche, wobei die Partikel (p) superparamagnetisch sind.

13. Zusammensetzung nach einem der vorhergehenden Ansprüche, wobei zwischen 1 und 70%, vorzugsweise zwischen 1 und 50% der Funktionen X der Verbindungen der Formel (I) an einen Biovektor gekuppelt sind.

14. Zusammensetzung nach einem der vorhergehenden Ansprüche, wobei die Funktionen X der Verbindungen der Formel (I) an mindestens zwei verschiedene Biovektoren gekuppelt sind.

15. Zusammensetzung nach einem der vorhergehenden Ansprüche, wobei der Biovektor ausgewählt ist aus, gegebenenfalls rekombinanten oder mutierten Proteinen, Glykoproteinen, Lektinen, Biotin, Vitaminen, Phospholipiden, Folsäurederivaten, Antikörpern oder Antikörperfragmenten, Peptiden und ihren Derivaten, Mono- oder Polysacchariden, Avidin, Streptavidin, Inhibitoren oder Substraten von Rezeptoren, Steroiden und ihren Analoga, Oligonukleotiden, Ribonukleinsäuresequenzen, Desoxyribonukleinsäuresequenzen, Hormonen oder zu Hormonen analogen Substanzen, Aminosäuren und Derivaten, organischen Molekülen mit pharmakologischer Aktivität, Aminoalkoholen, Mitteln zur Ansteuerung von Integrinen, Mitteln zur Ansteuerung von MMP.

16. Zusammensetzung nach Anspruch 15, wobei der Biovektor ein Peptidderivat, vorzugsweise ein Mittel zur Integrin- oder MMP-Ansteuerung, darstellt.

17. Zusammensetzung nach Anspruch 15, wobei der Biovektor ein Folsäure- oder Anti-Folsäurederivat darstellt.

18. Zusammensetzung nach Anspruch 15, wobei der Biovektor ein Phospholipidderivat, insbesondere ein Phosphatidylserin-, Phosphatidyletholamin-, Phosphatidylcholin-, Phosphatidylinositol-, Sphingomyelinderivat oder Derivate von Ceramiden darstellt.

19. Zusammensetzung nach Anspruch 15, wobei der Biovektor ein, gegebenenfalls rekombinantes oder mutiertes, Protein darstellt.

20. Zusammensetzung nach Anspruch 15, wobei der Biovektor einen Antikörper oder ein Antikörperfragment darstellt, die gegebenenfalls funktionalisiert sind.

21. Zusammensetzung nach Anspruch 15, wobei der Biovektor einen gegebenenfalls funktionalisierten Pharmakophor darstellt.

22. Zusammensetzung nach Anspruch 15, wobei der Biovektor aus den Aminoalkoholen der allgemeinen Formel (II) 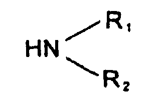 ausgewählt ist, worin R₁ und R₂ gleich oder verschieden sind und eine aliphatische Kohlenwasserstoffkette mit 2 bis 6 Kohlenstoffatomen darstellt, die vorzugsweise mit 6 bis 10 Hydroxylgruppen oder auch mit 4 bis 8 Hydroxylgruppen substituiert ist, wenn R₁ und/oder R₂ durch ein Sauerstoffatom unterbrochen ist.

23. Zusammensetzung nach Anspruch 15, wobei der Biovektor aus Aminopolyethylenglykolen ausgewählt ist.

24. Zusammensetzung nach Anspruch 22, wobei R₁ für eine Gruppe -(CH₂)-(CHOH)₄-CH₂OH oder -(CH₂)-CHOH-CH₂OH und R₂ für eine Gruppe -(CH₂)-(CHOH)₄-CH₂OH steht.

25. Zusammensetzung nach Anspruch 1, wobei der Biovektor die Verbindung N-[(2,3-Dihydroxypropyl)(2,3,4,5,6-pentahydroxyhexyl)]2,4,6-tribrom-5-(glycylamino)isophthalamid oder die Verbindung N-N'-[Bis(2,3,4,5,6-pentahydroxyhexyl)]2,4,6-tribrom-5-(glycylamino)isophthalamid ist.

26. Zusammensetzung nach einem der vorhergehenden Ansprüche, wobei die Funktionen X eine kovalente Bindung mit dem Biovektor des Typs -CONH-, -COO-, -NHCO-, -OCO-, -NH-CS-NH-, -C-S-, -N-NH-CO-, -CO-NH-N-, -CH₂-NH-, -N-CH₂-, -N-CS-N-, -CO-CH₂-S-, -N-CO-CH₂-S-, -N-CO-CH₂-CH₂-S, -CH=NH-NH-, -NH-NH=CH-, -CH-=N-O-, -O-N=CH- sowie der folgenden Formeln 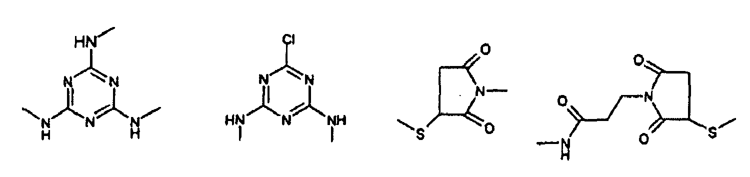 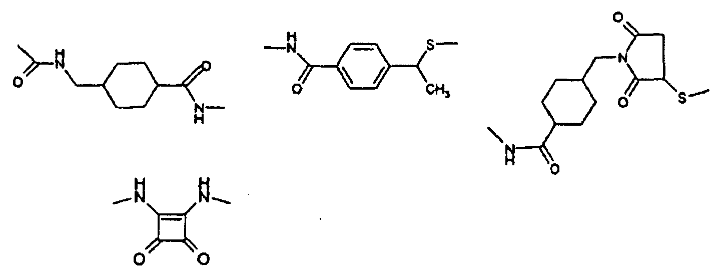 eingehen.

27. Zusammensetzung nach einem der vorhergehenden Ansprüche, wobei die komplexierten oder an den (die) Biovektor(en) gekuppelten Partikel einen globalen hydrodynamischen Durchmesser zwischen 5 nm und 200 nm, vorzugsweise zwischen 5 und 60 nm aufweisen.

28. Zusammensetzung nach einem der vorhergehenden Ansprüche, wobei die Konzentration der Partikel (p) in der Suspension zwischen 2 µmol L⁻¹ und 4 mol L⁻¹ beträgt, ausgedrückt als Gesamtanzahl an Mol Metallionen.

29. Zusammensetzung nach einem der vorhergehenden Ansprüche, die ein oder mehrere pharmazeutisch annehmbare Vehikel und/oder einen oder mehrere pharmazeutisch annehmbare Hilfsstoffe umfasst.

30. Zusammensetzung nach Anspruch 29, **dadurch gekennzeichnet, dass** sie steril ist.

31. Verfahren zur Herstellung einer Zusammensetzung nach den Ansprüchen 1 bis 30, wobei das Verfahren die aufeinander folgenden Schritte umfasst:
(i) Zusammenbringen einer sauren Lösung von sauren Magnetpartikeln (p) auf Basis einer Eisenverbindung mit Verbindungen der Formel (I) nach Anspruch 1 in einer ausreichenden Menge und Gewinnung der erhaltenen komplexierten Partikel;
gegebenenfalls
(ii) Durchführen der Kupplung der gesamten oder eines Teils der Funktionen X der Verbindungen der Formel (I), die an der Oberfläche der magnetischen Partikel (p) komplexiert sind, mit Biovektoren.

32. Verfahren nach Anspruch 31, wobei die im Schritt (i) verwendeten magnetischen Partikel (p) gemäß einem Verfahren erhalten werden, das folgende Schritte umfasst:
a) Herstellen einer kolloidalen Lösung von saurem Ferrofluid;
b) Behandeln der im Schritt a) erhaltenen Lösung mit einer Salpetersäurelösung;
c) Isolieren des im Schritt b) erhaltenen Koagulats und
d) Durchführen einer Peptisierung.

33. Kontrastprodukt für die Bildgebung mittels Magnetresonanz, umfassend magnetische Partikel, die gegebenenfalls an Biovektoren gekuppelt sind, nach den Ansprüchen 1 bis 30, gegebenenfalls zusammen mit einem pharmazeutisch annehmbaren Vehikel.

34. Verwendung von Zusammensetzungen nach den Ansprüchen 1 bis 30 zur Herstellung eines Kontrastmittels, das für die Bildgebung mittels Magnetresonanz verwendbar ist.

35. Verwendung von Zusammensetzungen nach den Ansprüchen 1 bis 30 zur Herstellung eines Kontrastmittels, das für die Nuklearmedizin verwendbar ist.

36. Verwendung von Zusammensetzungen nach den Ansprüchen 2 bis 30, wobei der Biovektor ein spezifischer Ligand eines biologischen Rezeptors ist, zur Herstellung eines Kontrastmittels, das zur Charakterisierung und/oder zur therapeutischen Überwachung, insbesondere bei Kardiovaskulärerkrankungen, Krebserkrankungen oder entzündlichen und degenerativen Erkrankungen, verwendbar ist.

37. Verwendung von Zusammensetzungen nach den Ansprüchen 2 bis 30, wobei der Biovektor ein spezifischer Ligand eines biologischen Rezeptors ist, zur *in-vitro-*Unterscheidung und/oder -Trennung in einem Gemisch von Bestandteilen, die ein Ziel für den Biovektor tragen, und Bestandteilen, die das Ziel nicht tragen.

38. Verwendung von an Biovektoren gekuppelten magnetischen Partikeln nach den Ansprüchen 2 bis 30, wobei der Biovektor der magnetischen Partikel (p) pharmakologische und/oder zytotoxische Eigenschaften besitzt, wobei der Biovektor gegebenenfalls unter der Einwirkung eines Radiofrequenzfelds in der angesteuerten Zone freigesetzt werden kann.

39. Verwendung von Zusammensetzungen nach den Ansprüchen 2 bis 30, wobei der Biovektor ein spezifischer Ligand eines biologischen Rezeptors ist, zur Behandlung pathologischer Gebiete mittels Hyperthermie.

40. Verwendung von Zusammensetzungen nach den Ansprüchen 2 bis 30 zur Zellmarkierung und insbesondere zur Markierung von Stammzellen.
